# FASCICULE DE BREVET EUROPEEN

(11) **EP 2 459 232 B1**
(45) Date de publication et mention de la délivrance du brevet: **08.03.2017**
(21) Numéro de dépôt: 10747922.2
(22) Date de dépôt: 26.07.2010
(51) Int. Cl.: A61K 48/00, C12Q 1/68

(54) **UTILISATION DE MICROARN POUR LE TRAITEMENT DE PATHOLOGIES LIÉES À UN DYSFONCTIONNEMENT DES CILS DES CELLULES ÉPITHÉLIALES MULTICILIÉES**
VERWENDUNG VON MICRO-RNA ZUR BEHANDLUNG VON DURCH DYSFUNKTION DER ZILIEN DER MULTIZILIÄREN EPITHELZELLEN CHARAKTERISIERTEN PATHOLOGIEN
USE OF MIRNA FOR THE TREATMENT OF DISEASES LINKED TO CILIA DYSFUNCTIONING IN MULTICILIATED EPITHELIAL CELLS.

(30) Priorité: 29.07.2009 FR 0903723
(43) Date de publication de la demande: 06.06.2012
(73) Titulaire: Centre National de la Recherche Scientifique, 75016 Paris (FR)
(72) Inventeur: MARCET, Brice, F-06110 Le Cannet (FR); BARBRY, Pascal, F-06000 Nice (FR); WALDMANN, Rainer, F-83600 Les Adrets De L'Esterel (FR); MARI, Bernard, F-06300 Nice (FR); CORAUX, Christelle, F-51100 Reims (FR)
(74) Mandataire: Gevers & Orès
(86) Numéro de dépôt international: PCT/FR2010/000539
(87) Numéro de publication internationale: WO 2011/015720

(56) Documents cités:
- WO-A1-2008/116267
- WO-A1-2009/137912
- WO-A2-2008/073922
- WO-A2-2008/137862
- WO-A2-2009/143379
- QIAN S ET AL: "MicroRNA expression profile of bronchioalveolar stem cells from mouse lung" BIOCHEMICAL AND BIOPHYSICAL RESEARCH COMMUNICATIONS, ACADEMIC PRESS INC. ORLANDO, FL, US, vol. 377, no. 2, 12 décembre 2008 (2008-12-12), pages 668-673, XP025646136 ISSN: 0006-291X [extrait le 2008-10-21]
- PERRY MARK M ET AL: "Rapid changes in microRNA-146a expression negatively regulate the IL-1 beta-induced inflammatory response in hunan lung alveolar epithelial cells" JOURNAL OF IMMUNOLOGY, vol. 180, no. 8, avril 2008 (2008-04), pages 5689-5698, XP7911920 ISSN: 0022-1767
- NANA-SINKAM SERGE P ET AL: "Integrating the MicroRNome into the study of lung disease." AMERICAN JOURNAL OF RESPIRATORY AND CRITICAL CARE MEDICINE 1 JAN 2009, vol. 179, no. 1, 1 janvier 2009 (2009-01-01), pages 4-10, XP7911921 ISSN: 1535-4970
- LU YUN ET AL: "Epithelial progenitor cells of the embryonic lung and the role of microRNAs in their proliferation." PROCEEDINGS OF THE AMERICAN THORACIC SOCIETY 15 APR 2008, vol. 5, no. 3, 15 avril 2008 (2008-04-15), pages 300-304, XP7911922 ISSN: 1546-3222
- DATABASE MEDLINE [en ligne] US NATIONAL LIBRARY OF MEDICINE (NLM), BETHESDA, MD, US 05 Juin 2014 SONG RUI ET AL: 'miR-34/449 miRNAs are required for motile ciliogenesis by repressing cp110.' Database accession no. NLM24899310 & SONG RUI ET AL: 'miR-34/449 miRNAs are required for motile ciliogenesis by repressing cp110.' NATURE vol. 510, no. 7503, 05 Juin 2014, pages 115 - 120 ISSN: 1476-4687
- DATABASE BIOSIS [en ligne] BIOSCIENCES INFORMATION SERVICE, PHILADELPHIA, PA, US Juillet 2014 WU JINGWEN ET AL: 'Two miRNA clusters, miR-34b/c and miR-449, are essential for normal brain development, motile ciliogenesis, and spermatogenesis' Database accession no. PREV201400603968 & PROCEEDINGS OF THE NATIONAL ACADEMY OF SCIENCES OF THE UNITED STATES OF AMERICA, vol. 111, no. 28, July 2014 (2014-07), pages E2851-E2857, ISSN: 0027-8424, DOI: 10.1073/PNAS.1407777111

## Description

La présente invention se rapporte au domaine de la régénération et de la différenciation des épithélia ciliés de vertébrés, notamment de mammifères, et en particulier d'hommes, conduisant à une ciliogénèse fonctionnelle et à l'implication de microARN dans le processus de régénération et de différenciation desdits épithélia ainsi qu'aux gènes modulés par lesdits microARN.

La présente invention se rapporte plus particulièrement à l'utilisation de microARN dans le traitement des maladies liées à un dysfonctionnement des cils des cellules épithéliales multiciliées, en particulier, les désordres résultant d'une ciliogénèse non fonctionnelle, telles que les maladies respiratoires chroniques pour lesquelles la régénération et/ou la différenciation de l'épithélium respiratoire est défectueuse.

Les cellules ciliées qui tapissent la surface apicale des épithélia sont essentielles pour divers processus physiologiques tels que le nettoyage des voies respiratoires, l'implantation de l'embryon ou encore la circulation du fluide céphalorachidien. Une ciliogénèse défectueuse provoque directement ou est associée à une grande variété de pathologies.

Le processus de ciliogénèse comporte une séquence d'évènements qui débute par l'acquisition de l'identité de la cellule ciliée (phase 1). Cette première étape consiste en une inhibition latérale entre deux cellules adjacentes par le système de signalisation Notch via une interaction entre Notch et son ligand tel que delta-like 1 (DLL1). La cellule exprimant le ligand DLL1 devient une cellule progénitrice de cellules ciliées, simultanément, l'activation de Notch dans les cellules voisines empêche la transformation de ces cellules en cellules progénitrices de cellules ciliées. Les inventeurs ont montré que la cellule progénitrice de cellules ciliées exprime les microARN de la famille miR-449 et le facteur de transcription FOXJ1. Au cours d'une seconde phase, les miR-449 inhibent la division cellulaire et induisent la différenciation. La multiplication des centrioles débute dans la cellule progénitrice des cellules ciliées, cette multiplication est suivie l'ancrage des corps basaux aupôle apicale des cellules ; cette étape est suivie par l'assemblage de l'axonème et la synthèse des cils proprement dite.

Les épithéliua assurent une fonction de barrière entre le milieu interne et l'environnement extérieur. Les voies respiratoires sont tapissées par un épithélium pseudo-stratifié hautement différencié constitué de cellules basales, sécrétrices de mucus et ciliées (chaque cellule ciliée disposant de centaines de cils). Le battement coordonné de ces nombreux cils présents à la surface de cet épithélium permet l'élimination des déchets portés par le mucus au cours d'un processus appelé clairance mucociliaire. A ce titre, les cils jouent un rôle important dans les processus de défenses contre les infections de première ligne des voies aériennes (Puchelle et al. Proc Am Thorac Soc (2006) 3, 726-733).

L'exposition permanente de l'épithélium respiratoire à des stress environnementaux causés par des micro-organismes pathogènes, des allergènes, des molécules toxiques, etc... conduit à des lésions tissulaires. A la suite de telles lésions, un processus physiologique de régénération de l'épithélium respiratoire se met en place. Ce processus, lorsqu'il aboutit, répare la lésion et restaure l'intégrité du tissu respiratoire, un tissu différencié et à nouveau fonctionnel se substituant à la lésion.

Cette régénération met en jeu plusieurs étapes :
1) les cellules épithéliales prolifèrent et/ou migrent afin de combler le lit de la blessure ;
2) ces premières étapes sont suivies par la mise en place d'une étape de polarisation cellulaire caractérisée par la formation de jonctions serrées et par un adressage différentiel et spécifique de protéines membranaires (canaux, transporteurs d'ions...) entre le pôle apical et le pôle basolatéral (Puchelle *et al.* 2006 ; Hajj, R. et al. J Pathol (2007) 211, 340-350) ;
3) un stade de différenciation terminale conduisant à la formation de cils à la surface des cellules ciliées (ciliogénèse) et à la présence de cellules sécrétrices responsables de la synthèse et de la sécrétion de mucus.

Un épithélium pseudostratifié mucociliaire se substitue ainsi à la lésion, reconstituant un tissu cilié fonctionnel disposant de propriétés identiques à celles du tissu initial.

L'ensemble de ces phénomènes biologiques est associé à des mécanismes de transduction de signaux et à des profils d'expression géniques particuliers. Parmi certains acteurs connus et impliqués dans la différenciation et la ciliogénèse de l'épithélium respiratoire, le facteur de transcription *Foxj1* est l'un des mieux documentés (Yu, X. et al. (2008) Nat Genet 40, 1445-1453). *Foxj1* agit à une phase tardive de la ciliogénèse, jouant un rôle dans l'ancrage des corps basaux (petits organites d'organisation structurale voisine des centrioles indispensables à la formation de la base des cils) à la membrane apicale durant la formation de l'axonème (Gomperts, B.N. et al. (2004) J Cell Sci 117, 1329-1337 (2004).

Dans certaines maladies respiratoires chroniques telles que les broncho-pneumopathies obstructives chroniques (BPCO), la mucoviscidose, l'asthme ou encore la dyskinésie ciliaire primaire (DCP), des inflammations et infections chroniques aboutissent à une destruction du tissu respiratoire (Marshall, W.F. (2008) J Cell Biol 180, 17-21). Pour des raisons encore mal élucidées, ces maladies sont associées à des défauts de régénération et de différenciation épithéliale. Ces défauts aboutissent à une restructuration anormale du tissu, à une fibrose et à une perte fonctionnelle irréversible (Marshall, W.F., 2008). De nos jours, aucun traitement curatif pour ces différentes maladies n'a été trouvé, et seuls des traitements symptomatiques permettent de lutter avec plus ou moins d'efficacité contre la destruction évolutive du tissu respiratoire. Dans cette perspective, l'élucidation des mécanismes conduisant à la formation de cils fonctionnels (ciliogénèse) représente un enjeu majeur avec des retombées thérapeutiques évidentes.

La différenciation cellulaire implique des régulations temporelles et spaciales fines de la transcription et de la traduction gouvernant l'expression de gènes spécifiques. Ces événements sont contrôlés par divers signaux moléculaires et mécaniques. La compréhension des mécanismes physiologiques sous-jacents qui président la mise en place de la différenciation et de la ciliogénèse est donc un prélude indispensable à l'élaboration d'approches thérapeutiques plus spécifiques et plus efficaces.

Les microARN (miARN), petits ARN non-codants d'environ 22 bases découverts en 1993 et dotés de propriétés régulatrices, jouent un rôle clé dans la régulation des phénomènes cellulaires tels que la survie, l'apoptose, la prolifération, l'homéostasie ou la différenciation (Lu, Y. et al. (2007) Dev Biol 310, 442-453).

Leurs mécanismes d'action impliquent la formation d'un complexe entre plusieurs bases du miARN et la partie 3'-non codante de l'ARNm cible. Cette interaction induirait une déstabilisation de l'ARNm cible et/ou une inhibition de la synthèse protéique. La reconnaissance entre un miARN et sa cible est principalement contrôlée par une séquence d'environ 7 bases, située dans la partie 5' du miARN (ci-après séquence de reconnaissance ou seed). De ce fait, chaque miARN aurait la capacité de réguler la stabilité d'un large spectre d'ARNm distincts.

Plus de 750 miARN ont été caractérisés à ce jour chez l'homme où ils réguleraient plus de 30% des transcrits. La régulation par les miARN apparaît donc comme une régulation majeure de l'expression génique dont l'impact a été jusqu'à présent sous-estimé (Berezikov, E. et al. (2005) Cell 120, 21-24; Xie, X. et al. (2005) Nature 434, 338-345).

Les miARN sont transcrits dans le noyau sous forme de longs précurseurs. Ils subissent une première maturation dans le noyau pour donner un précurseur de miARN (pré-miARN) possédant une structure en épingle à cheveux de plus petite taille. Ce précurseur est exporté du noyau vers le cytoplasme où il va subir une dernière maturation. Une dégradation de sa boucle par l'enzyme Dicer génère deux miARN simple brin (un brin 5p et un brin 3p) ; le brin dit mature est pris en charge par un complexe multi-protéique (le complexe RISC, RNA induced silencing complex) qui interagit avec la partie 3'-non-codante des ARNm cibles, tandis que le brin complémentaire dit « star » va subir une dégradation ; le brin complémentaire d'un miARN miR-xy, miR-xy-z ou let-7x est annoté, respectivement, miR-xy*, miR-xy-z* ou let-7x*.

Des études ont récemment montré l'importance jouée par les microARN chez la souris dans les mécanismes de différenciation et de morphogénèse ; en particulier dans le développement embryonnaire et la prolifération des précurseurs des cellules épithéliales de l'épiderme (Lena, A.M. et al. (2008) Cell Death Differ 15, 1187-1195; Yi, R. et al. (2008) Nature 452, 225-229) ou bien encore le développement pulmonaire (Lu, Y. et al. (2007) Dev Biol 310, 442-453 (2007); Harris, K.S. et al. (2006) Proc Natl Acad Sci U S A 103, 2208-2213; Lu, Y. et al. (2008) Proc Am Thorac Soc 5, 300-304). Plus précisément, Lena *et al.* ont démontré l'implication des miARN du locus miR-17-92 dans la morphogénèse pulmonaire chez la souris. Bien qu'il y ait des preuves de l'implication des miARN au cours de la morphogénèse pulmonaire chez la souris, leur(s) rôle(s) précis et leur(s) mécanismes d'action demeurent encore inexplorés.

Enfin, plusieurs études suggèrent un rôle particulier de certains miARN dans des pathologies telles que les cancers, l'hypertrophie cardiaque, le diabète ou certaines infections virales (Triboulet, R. et al. (2007) Science 315, 1579-1582 ; Calin, G.A. & Croce, C.M. (2006) Nat Rev Cancer 6, 857-866 ; Grassmann, R. & Jeang, K.T. (2008) Biochim Biophys Acta 1779, 706-711 ; Latronico, M.V., (2008) Physiol Genomics 34, 239-242; Poy, M.N. et al. (2004) Nature 432, 226-230).

Aucune étude n'a, à ce jour, montré le rôle ou l'implication des miARN dans la régénération et la différenciation des épithélia ciliés, tel que l'épithélium respiratoire, et le contrôle de la ciliogénèse des vertébrés.

C'est donc pour la première fois que les Inventeurs ont mis en évidence l'implication de certains miARN dans le contrôle de la ciliogénèse de tissus épithéliaux de vertébrés et, en particulier, dans la régénération et la différenciation de cellules épithéliales respiratoires humaines en un épithélium de surface mucociliaire.

Les Inventeurs ont plus particulièrement utilisés différentes approches expérimentales conjuguant le séquençage à haut-débit des miARN, les biopuces miARN ainsi que la RT-PCR quantitative et identifié les miARN intervenant spécifiquement à différents stades de la différenciation de l'épithélium respiratoire humain et des cellules épidermiques ciliées des embryons de grenouille *Xenopus laevis*, à savoir des signatures des miARN spécifiques 1) de l'étape de prolifération, 2) de la polarisation cellulaire et 3) de la différenciation terminale et de la ciliogénèse.

Ils ont également confirmé le rôle des miARN ainsi identifiés dans le contrôle de la ciliogénèse de tissus épithéliaux notamment via la répression du ligand DLL1 de Notch.

Ils ont ainsi montré que le rôle des microARN mis en évidence sur la régénération et la ciliogénèse d'un tissu épithélial respiratoire humain était extrapolable au mécanisme de ciliogénèse de tout tissu épithélial multicilié chez les vertébrés.

Il a ainsi été mis en évidence que 63 miARN sont exprimés ou réprimés et/ou présents en quantité significative dans le tissu épithélial respiratoire sain au cours de la régénération et de la différenciation (voir les Tableaux III, IV, V et VI). La constatation d'une variation de l'expression d'un ou plusieurs miARN chez un individu malade par rapport à un individu sain est un indicateur d'un défaut de régulation de l'expression d'un ou plusieurs gènes ; cette variation pourra donc être compensée en administrant le ou les miARN sous-exprimés et/ou en administrant les antagomirs du ou des miARN sur-exprimés.

Ces résultats trouvent ainsi des applications pour l'évaluation de la capacité de régénération et de la différenciation, et ainsi de conduire une ciliogénèse fonctionnelle, du tissu épithélial cilié d'un sujet ; ils permettent également l'évaluation de la capacité de régénération et/ou de différenciation d'un tissu épithélial respiratoire dudit sujet et trouvent également un intérêt particulier dans le domaine du diagnostic *in vitro* et *in vivo.* En particulier, ils permettent la mise au point d'une méthode d'évaluation de la capacité d'un tissu épithélial cilié de conduire une ciliogénèse fonctionnelle chez un sujet vertébré, notamment mammifère et, de préférence, humain, caractérisée en ce qu'elle comprend les étapes :
(i) de mesure quantitative du niveau d'expression des miARN du tissu épithélial cilié dudit sujet ;
(ii) d'établissement du profil d'expression des miARN du tissu épithélial cilié dudit sujet ;
(iii) de comparaison du profil d'expression des miARN dudit sujet avec le profil d'expression de miARN du tissu épithélial cilié sain d'un ou plusieurs autres sujets, ledit profil comprenant tout ou partie des miARN du Tableau I ci-après ;
(iv) d'identification d'au moins un miARN dont le niveau d'expression par ledit sujet diffère d'au moins un facteur 2 par rapport au niveau d'expression du même miARN par le ou lesdits autres sujets, c'est-à-dire dont l'un quelconque des niveaux d'expression représente au moins deux fois l'autre niveau d'expression ; et
(v) de mise en évidence d'un défaut de la capacité de conduire à une ciliogénèse fonctionnelle d'un tissu épithélial cilié relié à une anomalie d'expression d'au moins un miARN dudit sujet si au moins un miARN dudit profil établi à l'étape (ii)
présente un niveau d'expression qui diffère d'au moins un facteur 2 par rapport au niveau d'expression du même miARN dans le profil d'expression du Tableau I. Lorsque le niveau d'expression est exprimé en log2 (comme dans le Tableau I), cela signifie qu'un défaut de la capacité de régénération et/ou de différenciation, et donc de ciliogénèse, d'un tissu épithélial cilié relié à une anomalie d'expression d'au moins un miARN est observé lorsque le niveau d'expression en log2 du sujet à tester varie d'au moins une unité par rapport au profil d'expression chez un ou plusieurs autres sujets sains.

La mise en oeuvre de cette méthode nécessite l'établissement du profil d'expression des miARN du tissu épithélial cilié d'un sujet pour lequel on souhaite établir un diagnostic relatif à la capacité de régénération et/ou de différenciation d'un tissu épithélial cilié et du profil d'expression des miARN du tissu épithélial cilié d'un ou plusieurs sujets sains, c'est-à-dire choisis pour ne pas présenter de désordre de la différenciation et/ou de la régénération et de la ciliogénèse du tissu épithélial cilié.

Selon une variante, cette méthode permet l'évaluation de la capacité de régénération et/ou de différenciation et de conduire une ciliogénèse fonctionnelle d'un tissu épithélial respiratoire d'un sujet mammifère, de préférence humain, et comprend les étapes :
(i) de mesure quantitative du niveau d'expression des miARN du tissu épithélial respiratoire dudit sujet ;
(ii) d'établissement du profil d'expression des miARN du tissu épithélial respiratoire dudit sujet ;
(iii) de comparaison du profil d'expression des miARN dudit sujet avec le profil d'expression de miARN dudit tissu épithélial respiratoire sain d'un ou plusieurs autres sujets, ledit profil comprenant tout ou partie des miARN du Tableau I ci-après ;
(iv) d'identification d'au moins un miARN dont le niveau d'expression par ledit sujet diffère d'au moins un facteur 2 par rapport au niveau d'expression du même miARN par le ou lesdits autres sujets, c'est-à-dire dont l'un quelconque des niveaux d'expression représente au moins deux fois l'autre niveau d'expression ; et
(v) de mise en évidence d'un défaut de la capacité de régénération et/ou de différenciation d'un tissu épithélial respiratoire relié à une anomalie d'expression d'au moins un miARN dudit sujet si au moins un miARN dudit profil établi à l'étape (ii) présente un niveau d'expression qui diffère d'au moins un facteur 2 par rapport au niveau d'expression du même miARN dans le profil d'expression du Tableau I.

### Définitions

- Par sujet, on entend un individu vertébré, en particulier mammifère et, de préférence, l'homme. De préférence, le sujet pour lequel on souhaite établir un diagnostic relatif à la capacité d'un tissu épithélial cilié à conduire une ciliogénèse fonctionnelle, il pourra plus spécficiquement s'agir de diagnostiquer la capacité de régénération et/ou de différenciation d'un tissu épithélial respiratoire et le ou les sujets sains appartiennent à la même espèce.
- dans le cadre de la présente invention, on utilise indifféremment les termes « cilié » et « multicilié » étant entendu que les tissus épithéliaux considérés sont tels que leurs cellules portent plusieurs cils.
- Par profil d'expression des miARN dans un tissu donné, on entend l'ensemble des miARN ayant un niveau d'expression supérieur ou égal à une valeur choisie.
- Le niveau d'expression d'un miARN dans une cellule ou un tissu est déterminé par mesure des miARN présents dans la cellule ou le tissu.
- La mesure du niveau d'expression d'un miARN peut être réalisée par toute technique connue de l'homme du métier ; on peut notamment citer, après une étape d'extraction des ARN, le séquencage à haut débit des miARN, le séquençage par NASBA (nucleic acid strand based amplification), par extension d'amorce, ou encore, les puces à ADN permettant l'hybridation des miARN.
- Le niveau d'expression des miARN peut être exprimé par différents moyens tels que :
   * le niveau d'intensité d'expression en log2, cette valeur est représentative de la quantité de miARN présent dans une cellule ou un tissu ; pour l'établissement d'un profil d'expression, on retiendra de préférence les miARN qui présentent un niveau d'intensité d'expression en log2 supérieur ou égal à 3 ;
      Ce niveau d'intensité d'expression peut être calculé par différents moyens en fonction de technique utilisée pour mesurer les miARN exprimés.
      Dans le cas d'une mesure de fluorescence sur des puces du type Agilent (voir les exemples ci-après), la mesure du niveau d'expression correspond à une intensité de fluorescence dont est ensuite calculé le log2. Dans le cas d'un séquençage à haut débit, le niveau d'intensité d'expression comptabilise le nombre de fois où la séquence d'un miARN est séquencée, ce nombre est normalisé par rapport au nombre total de séquences puis son log2 est calculé.
   * l'abondance qui représente le pourcentage d'expression d'un miARN dans une cellule ou un tissu par rapport à la quantité totale de miARN exprimés dans la même cellule ou le même tissu, dans ce cas, seront retenus les miARN dont l'abondance est supérieure ou égale à 0,1%, de préférence à 0,5%, de façon encore préférée à 1 %.

De préférence, les valeurs caractérisant le niveau d'expression sont exprimées en log2.

**Le Tableau I ci-dessous décrit le profil d'expression dans lequel le niveau d'expression est exprimé en niveau d'intensité d'expression en log2 des miARN d'un tissu épithélial respiratoire humain sain :**

| Nom du miARN | Numéro de séquence du miARN (SEQ ID) | Niveau d'intensité d'expression en log2 Au stade différencié (WD) mesuré en HTS |
|---|---|---|
| hsa-miR-100 | 1 | 9,00 |
| hsa-miR-106b | 2 | 5,39 |
| hsa-miR-125a-5p | 3 | 14,08 |
| hsa-miR-130a | 4 | 11,98 |
| hsa-miR-140-3p | 5 | 13,12 |
| hsa-miR-141 | 6 | 9,75 |
| hsa-miR-148a | 7 | 8,97 |
| hsa-miR-151-5p | 8 | 10,22 |
| hsa-miR-15a | 9 | 7,68 |
| hsa-miR-16 | 10 | 5,35 |
| hsa-miR-17 | 11 | 11,61 |
| hsa-miR-181a | 12 | 0,01 |
| hsa-miR-191 | 13 | 16,82 |
| hsa-miR-193b | 14 | 11,67 |
| Hsa-miR-1975 | 15 | 11,22 |
| hsa-miR-200a | 16 | 10,96 |
| hsa-miR-200b | 17 | 10,64 |
| hsa-miR-200c | 18 | 14,57 |
| hsa-miR-203 | 19 | 14,55 |
| hsa-miR-205 | 20 | 15,24 |
| hsa-miR-21 | 21 | 8,09 |
| Hsa-miR-21* | 73 | 10,49 |
| hsa-miR-210 | 22 | 9,29 |
| hsa-miR-22 | 23 | 11,22 |
| hsa-miR-224 | 24 | 14,10 |
| hsa-miR-23a | 25 | 15,35 |
| hsa-miR-23b | 26 | 9,35 |
| hsa-miR-25 | 27 | 3,52 |
| hsa-miR-26a | 28 | 7,75 |
| hsa-miR-26b | 29 | 6,81 |
| hsa-miR-27b | 30 | 14,81 |
| hsa-miR-29a | 31 | 14,53 |
| hsa-miR-29c | 32 | 9,74 |
| hsa-miR-30b | 33 | 0,01 |
| hsa-miR-30c | 34 | 10,87 |
| hsa-miR-30d | 35 | 8,76 |
| hsa-miR-30e | 36 | 12,74 |
| hsa-miR-31 | 37 | 11,83 |
| hsa-miR-31* | 89 | 11,18 |
| hsa-miR-34a | 38 | 13,75 |
| hsa-miR-34b | 39 | 10,65 |
| Hsa-miR-34b* | 91 | 14,29 |
| hsa-miR-34c-5p | 40 | 0,01 |
| hsa-miR-365 | 41 | 2,78 |
| hsa-miR-374a | 42 | 11,87 |
| hsa-miR-378 | 43 | 11,70 |
| hsa-miR-425 | 44 | 9,53 |
| hsa-miR-429 | 45 | 15,11 |
| hsa-miR-449a | 46 | 12,24 |
| hsa-miR-449b | 47 | 14,56 |
| hsa-miR-449c | 201 | 10,98 |
| hsa-miR-574-3p | 48 | 13,42 |
| hsa-miR-92b | 49 | 0,01 |
| hsa-miR-939 | 50 | 5,43 |
| hsa-miR-96 | 51 | 12,65 |
| hsa-miR-99a | 52 | 9,00 |
| hsa-let-7a | 157 | 4,31 |
| hsa-let-7b | 158 | 11,28 |
| hsa-let-7c | 159 | 7,86 |
| hsa-let-7e | 160 | 7,60 |
| hsa-let-7f | 161 | 5,35 |
| hsa-let-7g | 162 | 12,40 |

La méthode peut présenter une étape additionnelle consistant à comparer le profil d'expression de microARN du tissu épithélial cilié, notamment respiratoire, du sujet pour lequel on souhaite établir un diagnostic relatif à la capacité de conduire une ciliogénèse fonctionnelle dudit tissu, notamment de la capacité de régénération et/ou de différenciation, avec un profil d'expression de miARN du tissu épithélial cilié sain d'un ou plusieurs sujets établi à un stade de différenciation particulier, un tel profil peut être établi avec le Tableau IV.

La mise en oeuvre cette méthode a permis d'identifierles miARN suivants hsa-miR-100, hsa-miR-106b, hsa-miR-125a-5p, hsa-miR-130a, hsa-miR-140-3p, hsa-miR-141, hsa-miR-151-5p, hsa-miR-15a, hsa-miR-16, hsa-miR-17, hsa-miR-181a, hsa-miR-191, hsa-miR-193b, hsa-miR-1975, hsa-miR-200a, hsa-miR-200b, hsa-miR-200c, hsa-miR-203, hsa-miR-205, hsa-miR-21, hsa-miR-210, hsa-miR-22, hsa-miR-224, hsa-miR-23a, hsa-miR-23b, hsa-miR-25, hsa-miR-26a, hsa-miR-26b, hsa-miR-27b, hsa-miR-29a, hsa-miR-29c, hsa-miR-30b, hsa-miR-30c, hsa-miR-30d, hsa-miR-30e, hsa-miR-31, hsa-miR-34a, hsa-miR-34b, hsa-miR-34c-5p, hsa-miR-365, hsa-miR-374a, hsa-miR-378, hsa-miR-425, hsa-miR-429, hsa-miR-449a, hsa-miR-449b, hsa-miR-449c, hsa-miR-574-3p, hsa-miR-92b, hsa-miR-939, hsa-miR-96, hsa-miR-99a, hsa-let-7a, hsa-let-7b, hsa-let-7c, hsa-let-7e, hsa-let-7f et hsa-let-7g de séquence SEQ ID N°1 à 6, 8 à 52, 157 à 162 et 201 (voir le tableau I ci-dessous), leur brin complémentaire « star » de séquence SEQ ID N°53 à 58, 60 à 104, 163 à 168 et 202, ou le brin de séquence complémentaire éventuellement modifié chimiquement, et leur précurseur de séquence SEQ ID N°105 à 110, 112 à 156, 169 à 174, 193 à 200 et 203 pour leur utilisation pour la prévention et/ou le traitement de désordres liés à un dysfonctionnement des cils de tissu épithélial cilié.

La présente invention se rapporte à un miARN sélectionné dans le groupe constitué par hsa-miR-92b (SEQ ID N°49), hsa-miR-1975 (SEQ ID N°15), hsa-miR-99a (SEQ ID N°52), hsa-miR-191 (SEQ ID N°13), hsa-miR-378 (SEQ ID N°43), hsa-miR-125a-5p (SEQ ID N°3), hsa-miR-203 (SEQ ID N°19), et leur précurseurs de séquence SEQ ID N°107, 117, 119, 123, 147, 153 et 156 et/ou au moins un brin complémentaire des miARN éventuellement modifié chimiquement choisis parmi hsa-miR-205, hsa-miR-17, hsa-miR-29a, hsa-miR-193b, hsa-miR-210 hsa-miR-130a : hsa-miR-205* (SEQ ID N°72), hsa-miR-31 et hsa-miR-31* (SEQ ID N°37 et 89), hsa-miR-17* (SEQ ID N°63), hsa-miR-29a* (SEQ ID N°83), hsa-miR-193b* (SEQ ID N°66), hsa-miR-210* (SEQ ID N°74), hsa-miR-130a* (SEQ ID N°56) et leur précurseurs de séquence SEQ ID N°108, 115, 118, 124 à 126, 135 et 141 pour son utilisation pour la prévention et/ou le traitement de désordres liés à un dysfonctionnement des cils de tissu épithélial cilié.

On entend par tissu épithélial cilié un tissu dont les cellules portent sur leur surface apicale des cils ; chez les mamifères, il s'agit notamment de l'épithélium respiratoire ou encore de l'épithélium des trompes utérines et l'endomètre de l'utérus, du plexus choroïdien et cellules épendymaires dans le cerveau, et des spermatozoïdes, rete testis et les canaux déférents chez le mâle.

Par brin de séquence complémentaire d'un premier fragment d'acide nucléique simple brin donné, on entend le fragment d'acide nucléique simple brin dont la séquence est le complément de la séquence dudit premier fragment et capable de s'apparier avec ledit premier fragment.

Dans le cadre de l'invention, il peut être utilisé des brins de séquence complémentaires modifiés chimiquement, c'est-à-dire que leur séquence comprend une ou plusieurs bases modifiées chimiquement, afin d'améliorer leur stabilité intracellulaire et extracellulaire et les rendre moins sensibles à l'hydrolyse dans des conditions acides ou basiques ainsi que sous l'action de nucléases ; les modifications envisageables sont notamment celles listées pour les ARN interférents (siRNA) dans la revue de T. M. Rana (Nature Reviews, 2007, vol 8: 23-36) ou telles que décrites dans la Demande WO 20071021896 ; en particulier, les brins de séquence complémentaires peuvent comprendre un nucléotide modifié chimiquement choisi parmi les nucléotides modifié en 2' tels qu'une base 2'-désoxy, 2'-désoxy-2'-fluoro, 2'-O-méthyl, 2'-O-méthoxyéthyl (2'-O-MOE), 2'-O-aminopropyl (2'-OAP), 2'-O-diméthylaminoéthyl (2'-O-DMAOE), 2'-O-diméthylaminopropyl (2'-O-DMAP), 2'-O-diméthylaminoéthyloxyéthyl (2'-O-DMAEOE) ou 2'-O-N-méthylacétamido (2'-O-NMA).

**Tableau II - miARN et référence de leur séquence nucléotidique, de la séquence nucléotidique de leur brin complémentaire et de celle de leur précurseur**

| **Nom et Séquence (numéro de SEQ ID) des miARN** | | **Nom et séquence (numéro de SEQ ID) du brin complémentaire** | | **Séquence du précurseur (numéro de SEQ ID)** |
|---|---|---|---|---|
| hsa-miR-100 | 1 | hsa-miR-100* | 53 | 105 |
| hsa-miR-106b | 2 | hsa-miR-106b* | 54 | 106 |
| hsa-miR-125a-5p | 3 | hsa-miR-125a-3p | 55 | 107 |
| hsa-miR-130a | 4 | hsa-miR-130a* | 56 | 108 |
| hsa-miR-140-3p | 5 | hsa-miR-140-5p | 57 | 109 |
| hsa-miR-141 | 6 | hsa-miR-141* | 58 | 110 |
| hsa-miR-148a | 7 | hsa-miR-148a* | 59 | 111 |
| hsa-miR-151-5p | 8 | hsa-miR-151-3p | 60 | 112 |
| hsa-miR-15a | 9 | hsa-miR-15a* | 61 | 113 |
| hsa-miR-16 | 10 | hsa-miR-16* | 62 | 114, 193 |
| hsa-miR-17 | 11 | hsa-miR-17* | 63 | 115 |
| hsa-miR-181a | 12 | hsa-miR-181a* | 64 | 116, 194 |
| hsa-miR-191 | 13 | hsa-miR-191* | 65 | 117 |
| hsa-miR-193b | 14 | hsa-miR-193b* | 66 | 118 |
| Hsa-miR-1975 | 15 | Hsa-miR-1975* | 67 | 119 |
| hsa-miR-200a | 16 | hsa-miR-200a* | 68 | 120 |
| hsa-miR-200b | 17 | hsa-miR-200b* | 69 | 121 |
| hsa-miR-200c | 18 | hsa-miR-200c* | 70 | 122 |
| hsa-miR-203 | 19 | hsa-miR-203* | 71 | 123 |
| hsa-miR-205 | 20 | hsa-miR-205* | 72 | 124 |
| hsa-miR-21 | 21 | hsa-miR-21* | 73 | 125 |
| hsa-miR-210 | 22 | hsa-miR-210* | 74 | 126 |
| hsa-miR-22 | 23 | hsa-miR-22* | 75 | 127 |
| hsa-miR-224 | 24 | hsa-miR-224* | 76 | 128 |
| hsa-miR-23a | 25 | hsa-miR-23a* | 77 | 129 |
| hsa-miR-23b | 26 | hsa-miR-23b* | 78 | 130 |
| hsa-miR-25 | 27 | hsa-miR-25* | 79 | 131 |
| hsa-miR-26a | 28 | hsa-miR-26a* | 80 | 132, 195 |
| hsa-miR-26b | 29 | hsa-miR-26b* | 81 | 133 |
| hsa-miR-27b | 30 | hsa-miR-27b* | 82 | 134 |
| hsa-miR-29a | 31 | hsa-miR-29a* | 83 | 135 |
| hsa-miR-29c | 32 | hsa-miR-29c* | 84 | 136 |
| hsa-miR-30b | 33 | hsa-miR-30b* | 85 | 137 |
| hsa-miR-30c | 34 | hsa-miR-30c* | 86 | 138, 196 |
| hsa-miR-30d | 35 | hsa-miR-30d* | 87 | 139 |
| hsa-miR-30e | 36 | hsa-miR-30e* | 88 | 140 |
| hsa-miR-31 | 37 | hsa-miR-31* | 89 | 141 |
| hsa-miR-34a | 38 | hsa-miR-34a* | 90 | 142 |
| hsa-miR-34b | 39 | hsa-miR-34b* | 91 | 143 |
| hsa-miR-34c-5p | 40 | hsa-miR-34c-3p | 92 | 144 |
| hsa-miR-365 | 41 | hsa-miR-365* | 93 | 145, 197 |
| hsa-miR-374a | 42 | hsa-miR-374a* | 94 | 146 |
| hsa-miR-378 | 43 | hsa-miR-378* | 95 | 147 |
| hsa-miR-425 | 44 | hsa-miR-425* | 96 | 148 |
| hsa-miR-429 | 45 | hsa-miR-429* | 97 | 149 |
| hsa-miR-449a | 46 | hsa-miR-449a* | 98 | 150 |
| hsa-miR-449b | 47 | hsa-miR-449b* | 99 | 151 |
| hsa-miR-449c | 201 | hsa-miR-449c* | 202 | 203 |
| hsa-miR-574-3p | 48 | hsa-miR-574-5p | 100 | 152 |
| hsa-miR-92b | 49 | hsa-miR-92b* | 101 | 153 |
| hsa-miR-939 | 50 | hsa-miR-939* | 102 | 154 |
| hsa-miR-96 | 51 | hsa-miR-96* | 103 | 155 |
| hsa-miR-99a | 52 | hsa-miR-99a* | 104 | 156 |
| hsa-let-7a | 157 | hsa-let-7a* | 163 | 169, 198, 199 |
| hsa-let-7b | 158 | hsa-let-7b* | 164 | 170 |
| hsa-let-7c | 159 | hsa-let-7c* | 165 | 171 |
| hsa-let-7e | 160 | hsa-let-7e* | 166 | 172 |
| hsa-let-7f | 161 | hsa-let-7f* | 167 | 173,200 |
| hsa-let-7g | 162 | hsa-let-7g* | 168 | 174 |

Les miARN selon la présente invention trouvent ainsi un intérêt particulier pour la prévention et/ou le traitement de ciliopathies dites primaires, c'est-à-dire qui sont directement associées à un dysfonctionnement des cils, ces ciliopathies primaires sont notamment :
- la dyskinésie ciliaire primaire ou syndrome de Kartagener ;
- *situs invertus* ;
- l'infertilité masculine (mobilité des spermatozoïdes) et féminine (e.g. grossesses extra-utérines) ;
- le syndrome d'Alstrom ;
- le syndrome de Bardet-Biedl ;
- le syndrome de Meckel-Gruber ;
- la polykystique des reins ;
- la dégénération rétinienne ;
- le syndrome de Senior-loken.

Les miARN selon la présente invention trouvent également un intérêt pour la prévention et/ou le traitement des ciliopathologies dites secondaires, c'est-à-dire qui sont associées à un défaut de fonctionnement des cils telles que :
- la mucoviscidose ;
- les broncho-pneumopathies obstructives chroniques (BCPO) ;
- l'asthme ;
- la bronchiolite ;
- les infections respiratoires d'origine virale.

L'utilisation selon la présente invention trouve donc plus particulièrement des applications dans la prévention et/ou le traitement des pathologies impliquant un désordre de la régénération et/ou de la différenciation de l'épithélium respiratoire ; ces pathologies sont notamment des maladies respiratoires chroniques et/ou héréditaires telles que les broncho-pneumopathies obstructives chroniques (BCPO), la mucoviscidose, l'asthme, la dyskinésie ciliaire primaire, les inflammations et infections chroniques des voies respiratoires et les insuffisances respiratoires.

Les pathologies respiratoires chroniques et/ou héréditaires (e.g. BPCO, mucoviscidose, asthme, DCP, rhinite allergique...) représentent un problème majeur de santé publique, quatrième cause de mortalité dans les pays industrialisés, et dont la prévalence est en augmentation rapide, d'où la nécessité importante de mieux appréhender les causes et les mécanismes de ces maladies afin de mieux cibler leurs traitements.

L'asthme est la plus commune des maladies respiratoires chroniques chez l'enfant (5 à 20%) et représente une des causes majeures d'hospitalisation. La recrudescence d'allergies respiratoires et l'augmentation de la pollution industrielle participent à l'augmentation importante des cas d'asthme dans la population (O van Schayck, et al. IPAG DIAGNOSIS & MANAGEMENT HANDBOOK. Chronic Airways Diseases. A Guide for Primary Care Physicians. 1-34, 2005).

Les BPCO sont un groupe de maladies respiratoires associées à une forte morbidité et à une forte mortalité. Les BPCO sont causées par des particules toxiques inhalées, dont le tabac est la plus représentative, qui vont conduire à une inflammation et à des infections chroniques fragilisant le tissu respiratoire. Environ 15% des fumeurs sont susceptibles de développer une BPCO dans leur vie. L'organisation mondiale de la santé estime qu'il existe près de 1,1 milliard de fumeurs dans le monde, avec une prévalence de BPCO de 0,8 à 6% de la population mondiale. En 2020, des études prédisent que les BPCO représenteront la troisième cause de mortalité dans le monde. Bien que les causes et les mécanismes de ces maladies demeurent largement inconnus, il est probable que d'autres facteurs (géniques, épigénétiques) soient susceptibles d'agir sur le développement de BPCO (O van Schayck, *et al.* 2005).

La mucoviscidose est la maladie génétique héréditaire la plus fréquente dans la population caucasienne (1 naissance sur 2500), affectant les glandes exocrines et donc l'ensemble du tissu épithélial sécréteur des différents organes (poumons, pancréas, foie, glandes sudoripares, intestins). Des défauts de transport d'ions à travers les épithéliums conduisent à un épaississement du mucus responsable notamment d'inflammation et d'infections respiratoires chroniques, aboutissant à une destruction tissulaire irréversible et à une insuffisance respiratoire sévère. Il n'existe aucun traitement curatif de cette maladie, mais l'avancée des connaissances scientifiques et médicales a permis d'instaurer des traitements symptomatiques qui ont fait progresser l'espérance de vie des patients de 3 ans dans les années 60 à environ 45 ans aujourd'hui.

La dyskinésie ciliaire primaire (DCP) est une maladie autosomique récessive rare (1 naissance sur 15000) causant un défaut de mobilité des cils de l'épithélium respiratoire conduisant à une clairance mucociliaire défectueuse, des inflammations et infections chroniques et une destruction tissulaire irréversible.

L'intérêt de ces miARN en vue du traitement de pathologies impliquant un désordre de la régénération et/ou de la différenciation de l'épithélium respiratoire est confirmé par la régulation par ces miARN de gènes connus pour être impliqués dans la régénération et/ou de la différenciation de l'épithélium respiratoire (voir l'exemple III ci-après).

On préfère utiliser des miARN qui sont présents en quantité significative au sein du tissu épithélial cilié au cours du processus de ciliogénèse (de préférence, dont l'abondance est d'au moins 1% des miARN totaux, voir le Tableau III) tels qu'un miARN choisi parmi hsa-miR-34a (SEQ ID N°38), hsa-miR-34b (SEQ ID N°39), hsa-miR-34c-5p (SEQ ID N°40), hsa-miR-449a (SEQ ID N°46), hsa-miR-449b (SEQ ID N°47), hsa-miR-449b* (SEQ ID N°99), hsa-miR-449c (SEQ ID N°201), hsa-miR-92b (SEQ ID N°49), hsa-miR-1975 (SEQ ID N°15), hsa-miR-99a (SEQ ID N°52), hsa-miR-191 (SEQ ID N°13), hsa-miR-378 (SEQ ID N°43), hsa-miR-23b (SEQ ID N°26), hsa-miR-125a-5p (SEQ ID N°3), hsa-miR-203 (SEQ ID N°19), hsa-miR-574-3p (SEQ ID N°48), hsa-miR-29c (SEQ ID N°32), hsa-miR-140-3p (SEQ ID N°5), hsa-miR-205 (SEQ ID N°20), hsa-miR-23a (SEQ ID N°25), hsa-miR-31 (SEQ ID N°37), hsa-miR-31* (SEQ ID N°89), hsa-miR-21 (SEQ ID N°21), hsa-miR-17 (SEQ ID N°11), hsa-miR-29a (SEQ ID N°31), hsa-miR-193b (SEQ ID N°14), hsa-miR-210 (SEQ ID N°22) et hsa-miR-130a (SEQ ID N°4), leur brin complémentaire « star » de séquence SEQ ID N°55 à 57, 63, 65 à 67, 71 à 74, 77, 78, 83, 84, 90 à 92, 95, 98, 100, 101, 104 et 202, leur brin de séquence complémentaire éventuellement modifié chimiquement, et leurs précurseurs de SEQ ID N°107 à 109, 115, 117 à 119, 123 à 126, 129, 130, 135, 136, 141 à 144, 147, 151 à 153, 156 et 203.

Parmi ces miARN, on peut distinguer ceux dont l'expression est induite de ceux dont l'expression est réprimée (Tableau IV) ; afin de reproduire un profil d'expression de miARN comparable à celui d'un individu sain, il apparait avantageux d'utiliser un ou plusieurs miARN dont l'expression est induite et/ou un ou plusieurs antagomirs de miARN dont l'expression est réprimée au cours du processus de ciliogénèse des tissus épithéliaux ciliés et, notamment, de régénération et de différenciation du tissu épithélial respiratoire ; un tel miARN peut être choisi parmi hsa-miR-34a (SEQ ID N°38), hsa-miR-34b (SEQ ID N°39), hsa-miR-34c-5p (SEQ ID N°40), hsa-miR-449a (SEQ ID N°46), hsa-miR-449b (SEQ ID N°47), hsa-miR-449b* (SEQ ID N°99), hsa-miR-449c (SEQ ID N°201), hsa-miR-92b (SEQ ID N°49), hsa-miR-1975 (SEQ ID N°15), hsa-miR-99a (SEQ ID N°52), hsa-miR-191 (SEQ ID N°13), hsa-miR-378 (SEQ ID N°43), hsa-miR-23b (SEQ ID N°26), hsa-miR-125a-5p (SEQ ID N°3), hsa-miR-203 (SEQ ID N°19), et leur précurseurs de séquence SEQ ID N°107, 117, 119, 123, 130, 142 à 144, 147, 150, 151, 153, 156 et 203 et/ou au moins un brin complémentaire « star » des miARN choisis parmi hsa-miR-205, hsa-miR-31, hsa-miR-21, hsa-miR-17, hsa-miR-29a, hsa-miR-193b, hsa-miR-31*, hsa-miR-210 hsa-miR-130a ; ces brins complémentaires « star » sont hsa-miR-205* (SEQ ID N°72), hsa-miR-31 et hsa-miR-31* (SEQ ID N°37 et 89), hsa-miR-21* (SEQ ID N°73), hsa-miR-17* (SEQ ID N°63), hsa-miR-29a* (SEQ ID N°83), hsa-miR-193b* (SEQ ID N°66), hsa-miR-210* (SEQ ID N°74), hsa-miR-130a* (SEQ ID N°56) et leurs précurseurs de séquence SEQ ID N°108, 115, 118, 124 à 126, 135 et 141.

Par exemple" lesdits miARN dont l'expression est modulée au cours du processus de ciliogénèse des tissus épithéliaux ciliés et, notamment, de régénération et/ou de différenciation du tissu épithélial respiratoire dont l'expression est induite sont : hsa-miR-34a (SEQ ID N°38), hsa-miR-34b (SEQ ID N°39), hsa-miR-34c-5p (SEQ ID N°40), hsa-miR-449a (SEQ ID N°46), hsa-miR-449b (SEQ ID N°47), hsa-miR-449c (SEQ ID N°201), hsa-miR-449b* (SEQ ID N°99), hsa-miR-92b (SEQ ID N°49), hsa-miR-1975 (SEQ ID N°15), hsa-miR-99a (SEQ ID N°52), hsa-miR-191 (SEQ ID N°13), hsa-miR-378 (SEQ ID N°43), hsa-miR-23b (SEQ ID N°26), hsa-miR-125a-5p (SEQ ID N°3), hsa-miR-203 (SEQ ID N°19), et leur précurseurs de séquence SEQ ID N°107, 117, 119, 123, 130, 142 à 144, 147, 150, 151, 153, 156 et 203.

L'expression des miARN identifiés peut se faire grace à des vecteurs d'expression exprimant au moins un miARN dont l'expression est induite au cours du processus de ciliogénèse d'un tissu épithélial ou au cours du processus de régénération et/ou de différenciation du tissu épithélial respiratoire. Tout vecteur d'expression capable d'exprimer des ARN dans une cellule eucaryote et dans lequel est clonée une cassette d'expression d'un miARN pourra être utilisé.

L'invention se rapporte également à l'utilisation de miARN comportant la séquence de reconnaissance GGCAGUG (SEQ ID N°175) positionnée sur la région 2-8 du miARN, c'est-à-dire du 2^{ème} au 8^{ème} nucléotide du miARN, ou sur la région 3-9 du miARN, c'est-à-dire du 3^{ème} au 9^{ème} nucléotide du miARN ; il s'agit notamment des miARN hsa-miR-34a (SEQ ID N°38), hsa-miR-34c-5p (SEQ ID N°40), hsa-miR-449a (SEQ ID N°46),hsa-miR-449b (SEQ ID N°47) et hsa-miR-449c (SEQ ID N°201).

L'invention se rapporte aussi à l'utilisation de miARN comportant la séquence de reconnaissance AAUCACU (SEQ ID N°176) positionnée sur la région 2-8 ou sur la région 3-9 du miARN ; il s'agit notamment du miARN hsa-miR-34b (SEQ ID N°39).

L'invention se rapporte encore à l'utilisation de miARN comportant la séquence de reconnaissance AUCACUA (SEQ ID N°177) positionnée sur la région 2-8 ou sur la région 3-9 du miARN ; il s'agit notamment du miARN hsa-miR-34b (SEQ ID N°39).

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :

### FIGURES

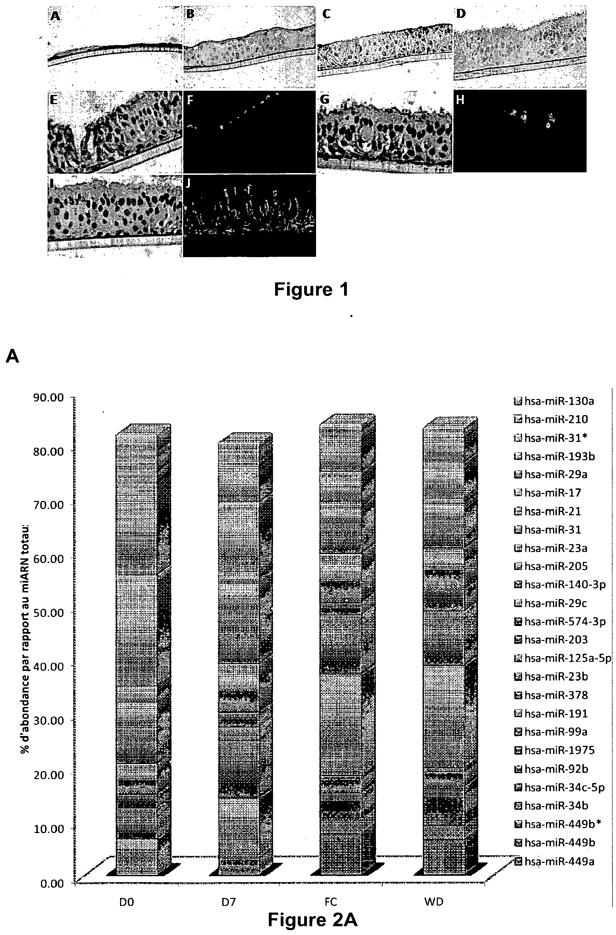

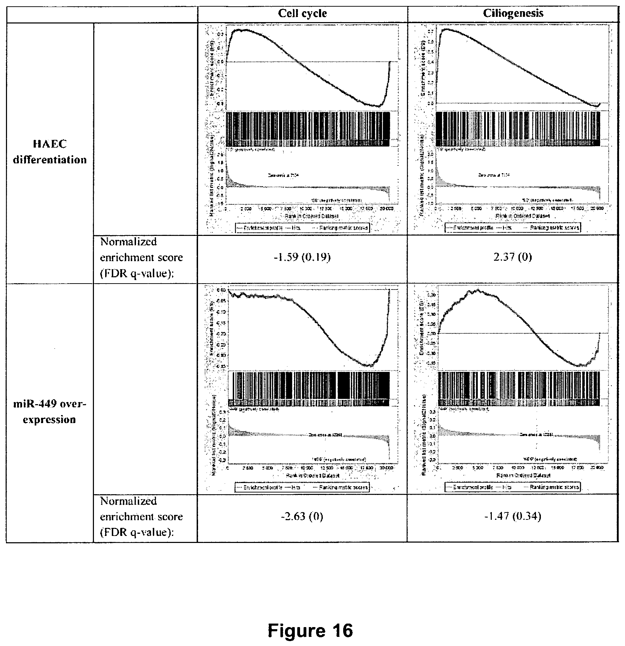

La **Figure 1** représente des clichés de cultures primaires de cellules épithéliales respiratoires humaines observées par microscopie confocale et électronique permettant d'observer la morphologie des cellules marquées à l'hématoxyline et à l'éosine. Les cellules ont également été immunomarquées afin d'examiner l'expression de marqueurs épithéliaux de différenciation (tubuline-β4 pour les cellules ciliées (**Figure 1E-F**), mucine MUC5AC pour les cellules sécrétrices à mucus (**Figure 1G-H**), et la cytokératine 13 pour les cellules basales (**Figure 1I-J**)). Les états de différenciation ont été évalués.

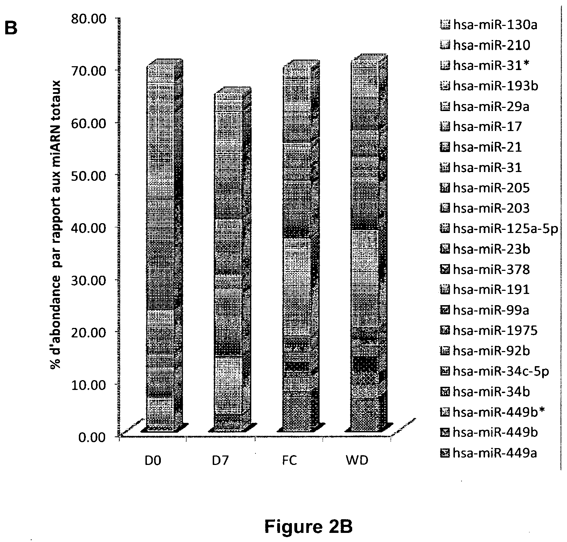

La **Figure 2A** représente les histogrammes cumulatifs montrant les 26 miARN ayant la plus forte expression dans l'un au moins des différents stades de différenciation de l'épithélium respiratoire humain chez 3 patients distincts : ALI-D0 (D0, prolifération), ALI-D7 (D7, prolifération et polarisation), ALI-FC (FC, début de ciliogénèse), ALI-WD (WD, épithélium pleinement différencié). La **Figure 2B** représente les histogrammes cumulatifs montrant les 22 miARN ayant la plus forte expression et étant significativement modulés dans l'un au moins des différents stades de différenciation de l'épithélium respiratoire humain chez 3 patients distincts : ALI-D0 (D0, prolifération), ALI-D7 (D7, prolifération et polarisation), ALI-FC (FC, début de ciliogénèse), ALI-WD (WD, épithélium pleinement différencié).

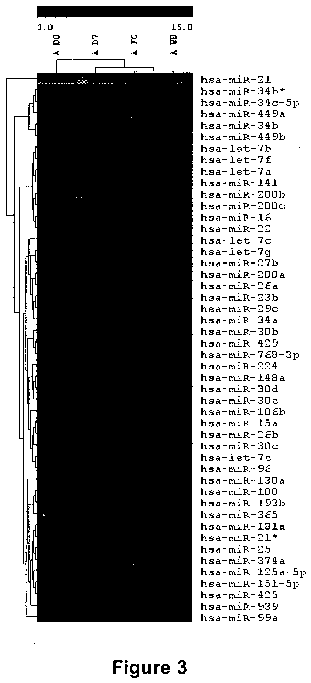

La **Figure 3** représente un cluster hiérarchique obtenu par puces miARN Agilent® montrant les variations d'intensité de l'expression des miARN statistiquement exprimés (A>8, P<0.05) et modulés dans au moins une condition de culture cellulaire (1<M<-1) (plus les cases sont foncées, moins les miARN sont exprimés) en fonction des états de différenciation des cultures cellulaires.

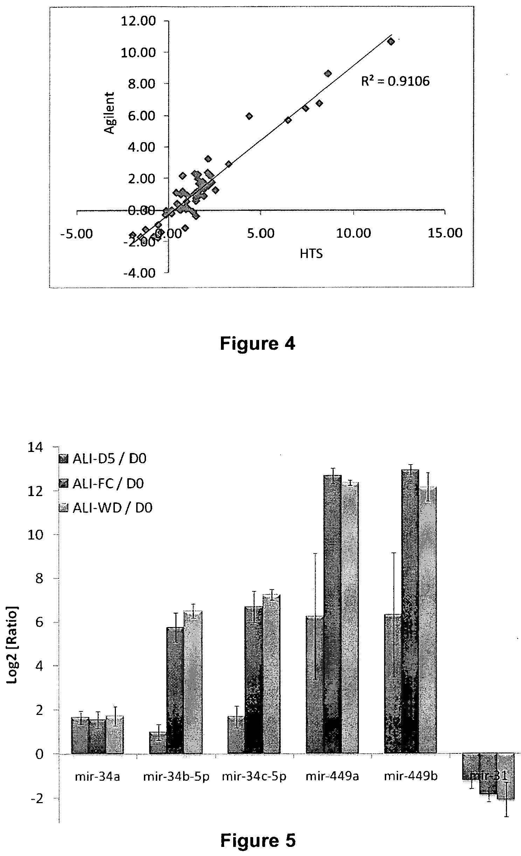

La **Figure 4** est un graphe montrant la corrélation entre les résultats obtenus en séquençage haut-débit (HTS) et en puces miARN (Agilent®).
La **Figure 5** represente un histogramme des variations observées par PCR quantitative des brins 5p de chacun des miARN testés à chaque stade de différenciation comme indiqué sur le graphique. Les résultats représentent la moyenne de 3 donneurs distincts.

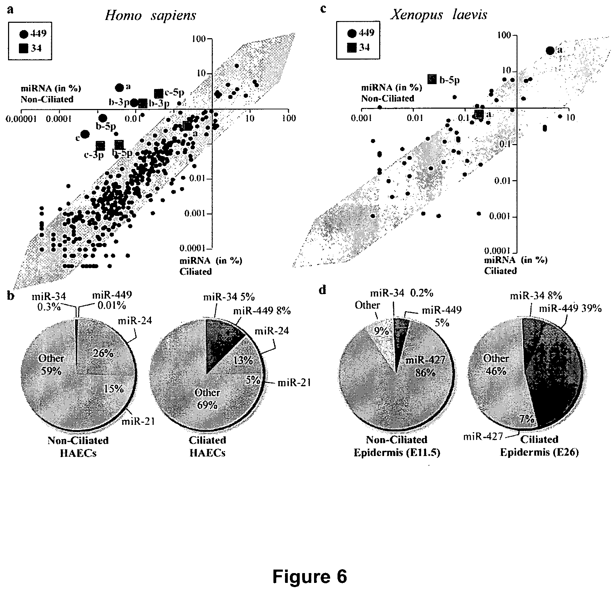

La **Figure 6** regroupe deux graphes représentant les microARN régulés au cours de la ciliogénèse. Les diagrammes a et c représentent l'abondance des microARN en pourcentage par rapport aux microARN totaux dans des cellules HAEC non différenciées (non ciliées, stade ALI-D0) (diagramme a) et dans un explant épidermique d'embryon de Xénope avant le stade embryonnaire E11.5 (gastrula) et après le stade E26 (bourgeon caudal) (diagramme c). Les diagrammes b et d reprennent ces mêmes données et illustrent l'abondance quantitative des miRNA.

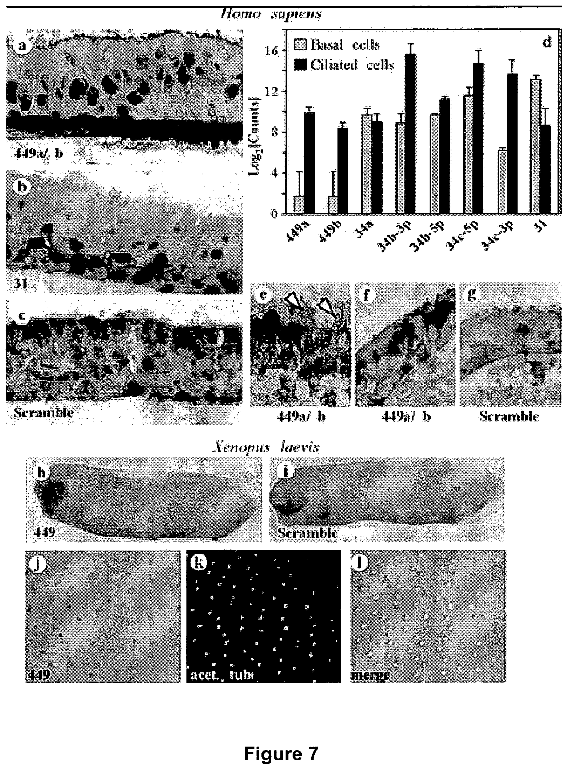

La **Figure 7** présente des clichés permettant de visualiser la localisation spécifique de miR-449 dans les cellules ciliées. Ces essais ont été réalisés par hybridation *in situ* sur des coupes congelées de culture de cellules HAEC à 21 jours (stade ALI-D21) (clichés a, b, c), de tissus bronchiques humains (clichés e, f, g) ou à partir d'embryons de Xenope au stade bourgeon caudal (clichés h, i, j, k, l) en utilisant des sondes LNA marquées à la digoxigénine et dirigées contre miR-449 (clichés a, e, f, h, i, j, l) ou contre miR-31 (cliché b) et des sondes aléatoires (« scramble ») comme contrôle négatif (clichés c, g, i). Les cellules ciliées d'embryon de Xenope ont été identifiées par un immunocytomarquage avec un anticorps primaire dirigé contre la tubuline acétylée. Les sondes LNA dirigées contre miR-449 marquent les cellules cylindriques ciliées (clichés a, e, h). Inversement, les sondes dirigées contre miR-31 ont marqué préférentiellement les cellules basales (cliché b). Les cellules muco-sécrétrices ont été marquées à l'aide d'anticorps anti-mucine MUC5AC (indiquées par des flèches dans le panneau e). Le diagramme d illustre les niveaux d'expresion des miR-449, miR-31 et miR-34 dans les cellules basales du tissu épithélial des voies respiratoires humaines et dans les cellules cylindriques ciliées.

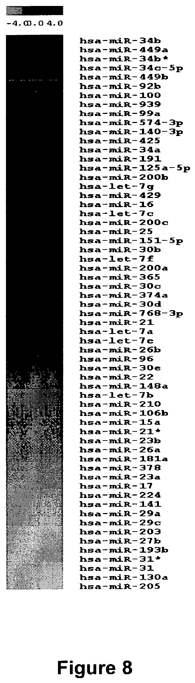

La **Figure 8** représente une cartographie (Heat-map) des microARN significativement régulés entre des cellules pyramidales (ciliées+sécrétrices) versus des cellules basales. Les 5 microARN positionnés en haut de la colonne sont spécifiques des cellules pyramidales (ciliées+sécrétrices) ; en dessous, plus les miARN apparaissent clairs, plus ils sont spécifiques des cellules basales.

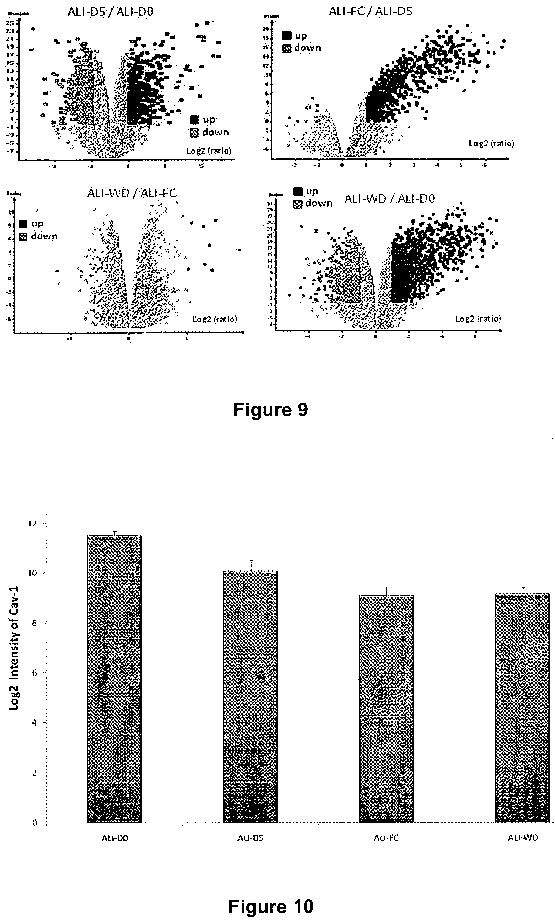

La **Figure 9** est un graphe montrant les relations existant entre le log₂ du ratio à deux stades de différenciation (abscisse) et la valeur de significativité statistique (ordonnées) pour chacune des comparaisons 2 à 2 indiquées sur le graphique. Les gènes significativement surexprimés sont indiqués en gris foncé (partie droite du graphe) et les gènes significativement réprimés sont indiqués en gris moyen (partie gauche du graphe).
La **Figure 10** est un histogramme montrant le log₂ de l'intensité d'expression de l'ARNm de la cavéoline-1 dans chacune des conditions de cultures étudiées, comme indiqué sur le graphique.

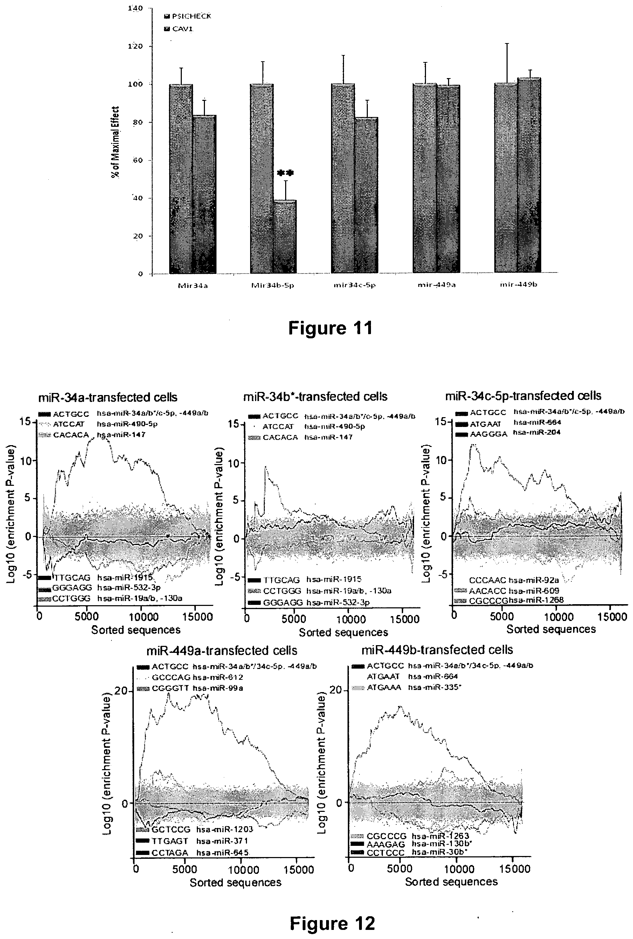

La **Figure 11** est un histogramme montrant l'effet de chacun des miARN sélectionnés sur l'activité de la luciférase (exprimée par rapport au % de l'effet maximal obtenu avec le vecteur contrôle PSICHECK) quand le gène rapporteur de la luciférase est fusionné à la partie 3' non-codante de l'ARNm de la Cav-1 versus le contrôle (PSICHECK). Les données ont été normalisées par rapport aux résultats obtenus avec un miARN contrôle négatif.
La **Figure 12** regroupe des graphiques illustrant l'enrichissement de gènes contenant la séquence de la séquence de reconnaissance (« seed ») commun aux mir-449a, mir-449b, mir-34a, mir-34b* et mir-34c. En abscisse est représenté le nombre de gènes analysés (environ 25000) ; ces gènes sont positionnés en fonction de leur niveau de répression ou d'expression : de la gauche vers la droite, figurent les gènes du plus réprimé au plus induit. L'ordonnée représente la valeur statistique en log10 du niveau d'enrichissement (le score d'enrichissement reporté sur l'axe des ordonnées correspond à la valeur absolue du log10 de la valeur P, calculée suivant un modèle cumulatif hypergéométrique et binomial décrit par van Dongen S et al. Nat Methods. 2008 Dec;5(12):1023-5). Sur chaque graphe est indiqué le miARN qui a été transfecté dans les cellules HAECs.

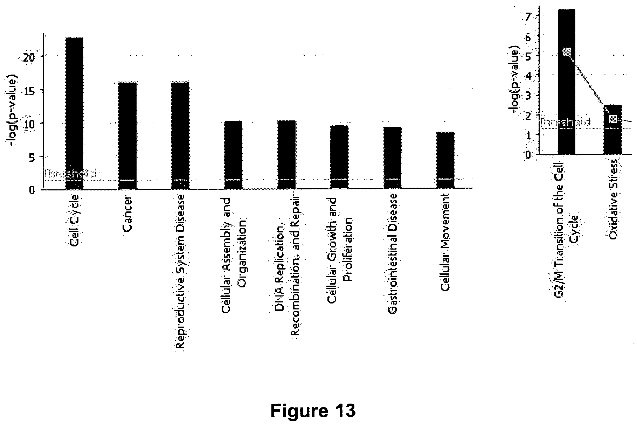

La **Figure 13** représente le résultat de l'identification à l'aide du software IPA de fonctions biologiques et de maladies en relation avec les gènes trouvés modulés par l'expression des miARN selon l'invention.

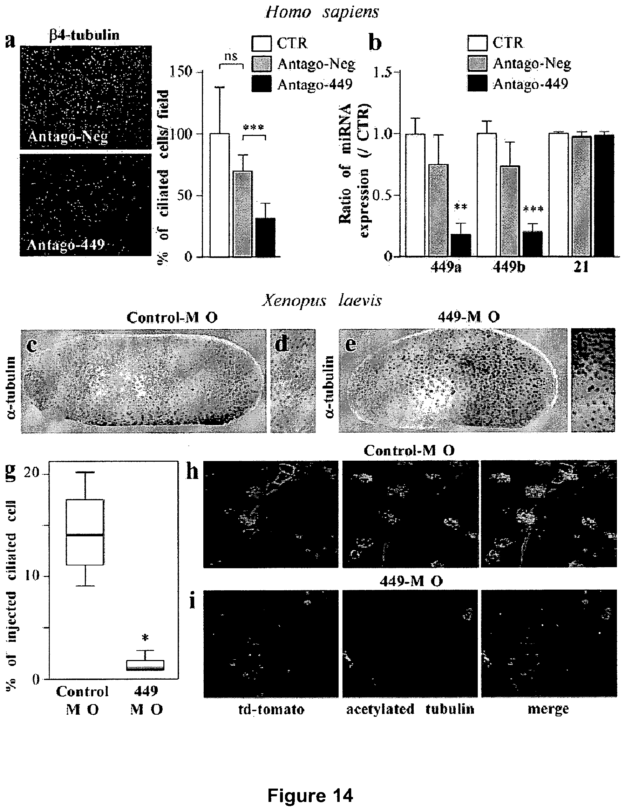

La **Figure 14** illustre l'inhibition de la ciliogénèse par la suppression de l'expression de miR-449.
   (a,b) les cellules HEACs sont traitées à plusieurs reprises avec des antagomirs anti-miR-449 ou un antagomir de contrôle (20 nM) au cours de leur régénération (généralement 21 jours). Le pourcentage de cellules ciliées a été défini comme le ratio du nombre de cellules positives à la -tubuline-β4 par rapport au nombre de noyaux (20 champs/filtre et 3 filtres/donneur). Le cliché (a) représente un immunomarquage typique de cellules HAEC traitées avec un antagomir de contrôle (Antago-Neg) ou un antagomir anti-miR-449 (Antago-449). L'histogramme indique le pourcentage de cellules ciliées par champ pour chaque condition expérimentale (n = 6, ***, P <0,001, Student-test). Le cliché (b) montre que l'antagomir anti-miR-449 supprime l'expression des miR449a et miR-449b, en revanche, il n'a pas d'effet dur l'expression de miR-21 (n = 6, ns = non significatif, **, P<0,01, ***, P<0,001, Student-test).
   Essais sur Xenope : un mélange comprenant 30 ng d'oligonucléotides morpholino (MO) de contrôle (clichés c, d, h) ou de MO miR-449a/b/c (10 ng chacun) et 2,5 ng de lysine-dextran fluorescent (FLDx, colorés en orange/marron, clichés c et f) ou 500 pg de l'ARNm de TD-tomato-CAAX pour tracer les cellules injectées (clichés g, i) est injecté dans l'épiderme d'embryons de Xenope au stade de la segmentation.
   Les clichés e et f permettent la détection de progéniteurs de cellules ciliées par un marquage à la tubuline-α avec la technique d'hybridation *in situ.*
   Cliché f : la région déplétée en miR-449 (haut) montre un excés de cellules positives à la tubuline-α par rapport à la région non injectée (bas). Les clichés h et i montrent la détection de cils chez les embryons au stade de têtard avec un anticorps anti-tubuline acétylée. L'image est prise dans la région de la nageoire caudale. Aucun cil n'est détecté dans les cellules co-injectées avec le MO miR-449 et l'ARNm TD-tomato-CAAX. Le graphe g représente le pourcentage de cellules injectées (positive pour la fluorescence rouge TD-tomato) qui développent des cils dans l'explant de contrôle (225 cellules, 8 queues) et dans celui modifié avec le MO miR-449 (242 cellules, 8 queues, p=0,036, test de Kruskall-Wallis).

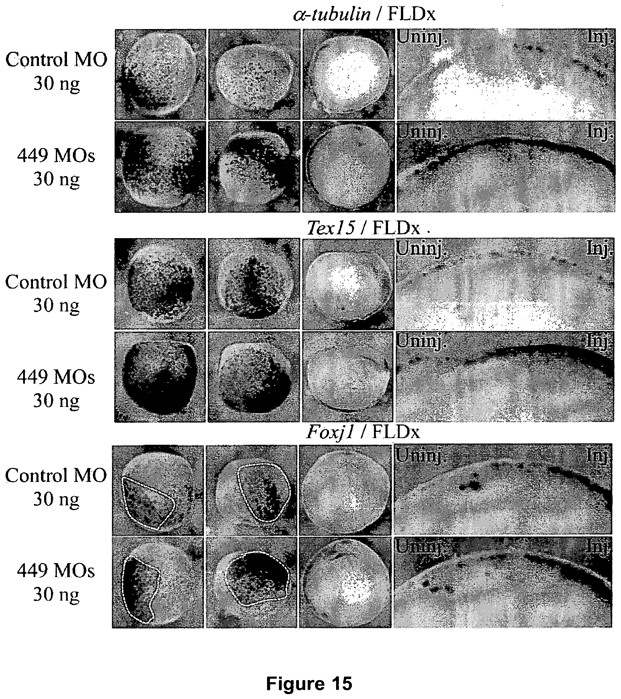

La **Figure 15** illustre l'induction de la spécialisation des cellules ciliées par la suppression de miR-449. Un mélange de MO anti-miR-449 ou de MO contrôle et de FLDx est injecté dans l'épiderme d'embryons au stade de la segmentation. Les embryons sont fixés au stade neurula précoce puis soumis à une hybridation *in situ* avec des ribosondes de tubuline-α, Tex15 et Foxj1, ils sont ensuite immunomarqués pour révéler la présence de FLDx (lysine-dextran fluorescente). Les embryons ont ensuite été sectionnés pour mieux visualiser la densité de progéniteurs des cellules ciliées dans les domaines injectés. Pour les trois marqueurs, la suppression de l'expresion des miR-449 a conduit à une augmentation des progéniteurs des cellules ciliées.
Les quatre sections de la **Figure 16** illustrent les résultats d'enrichissement des gènes comme expliqué sur le site http://www.broadinstitute.org/gsea/doc/GSEAUserGuideFrame.html. Le GSEA calcule un score d'enrichissement (ES) qui reflète le degré auquel un gene est surexprimé au sein d'une liste de gènes donnés. Un ES positif indique l'enrichissement d'un gène à gauche de l'ensemble des gènes (correspondant à un gène sur-régulé), un ES négatif indique l'enrichissement d'un gène à droite dudit ensemble de gènes (correspondant à un gène sous-régulé). Pour chaque section, le panneau du haut est une représentation graphique du score d'enrichissement d'un ensemble de genes comme indiqué sur la gauche. La partie du milieu du panneau montre où les membres de l'ensemble de gènes apparaissent dans la liste (« cycle cellulaire » ou « ciliogénèse »). La partie basse du panneau montre la valeur d'ordonnancement métrique qui mesure la coorélation entre l'expression d'un gène et le phénotype cellulaire ; une valeur positive indique une corrélation avec le premier phénotype (différenciation ou surexpression des miR-449) et une valeur négative indique une corrélation avec le second phénotype (non différencié ou contrôle). Toutes ces données expérimentales sont stockées dans la Base Gene Expression Omnibus sous le numéro d'accession GSE22147.

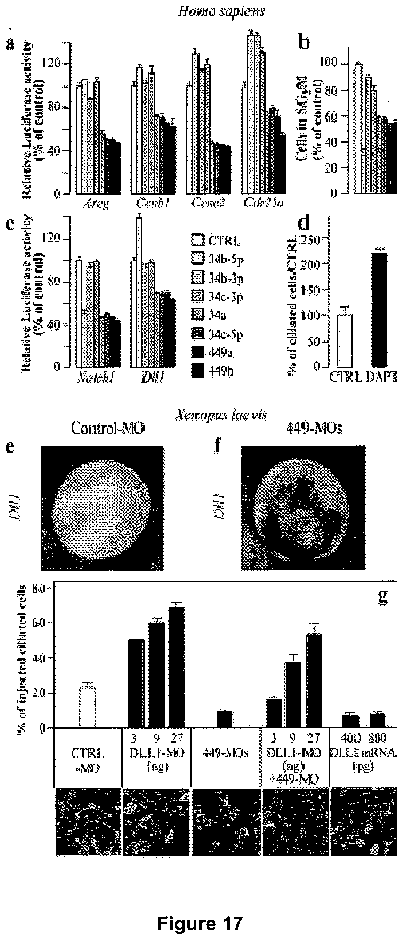

La **Figure 17** présente les résultats montrant que miR-449 cible le cycle cellulaire et la voie Notch.
   Cycle cellulaire : le graphe (a) représente la modulation de l'activité du gène rapporteur de la luciférase fusionné à la partie 3' non codantes de l'ARNm de *Areg*, *Ccnb1, Ccne2, Cdc25a* par miR-449a/b et miR-34a/c5-p.
   Voie de signalisaiton Notch : Le graphe (c) représente la modulation de l'activité du gène rapporteur de la luciférase fusionné à la partie 3' non codantes de l'ARNm de DLL1 et Notch1 par miR-449a/b et miR-34a/c5-p.
   Toutes les expériences ont été effectuées au moins deux fois en trois exemplaires. Les valeurs ont été normalisées au contrôle interne de luciférase de Renilla. Les barres d'erreur indiquent l'écart-type.
   Le graphe (b) représente le blocage du cycle cellulaire en phase G1 par miR-449 et miR-34.
   Le graphe (d) représente l'impact de l'inhibition de la voie Notch par DAPT (10 µM) sur la ciliogénèse des cellules HAECs.
   Les clichés (e) et (f) représentent des embryons au stade de segmentation dont l'épiderme a reçu une injection d'un mélange de MO anti-miR-449 et de lysine-dextran fluorescent (FLDx).
   Une coloration par hybridation *in situ* révèle l'expression soutenue de DLL1 dans l'épiderme déficient en miR-449.
   Le panneau (g) montre des embryons au stade de la segmentation dont l'épiderme a reçu une injection de MO de contrôle, de MO anti-miR-449 et de MO Dll1 ou d'ARNm DLL1. Les cils ont été colorés avec un anticorps anti-tubuline acétylée (en rouge). Pour chaque condition, au moins 200 cellules positives au FLDx (en vert), sur 6 à 8 queues, présentent des cils (P <0,03). Il convient de noter que l'injection d'ARNm de Dll1 supprime la ciliogénèse, bien qu'elle augmente le nombre de progéniteurs des cellules ciliées. L'extinction de l'expression de Dll1 induit la ciliogénèse.

### EXEMPLES

### I- Préparation du modèle cellulaire biologique

### I-A. cellules épithéliales respiratoires humaines

Les essais ont été réalisés avec des cultures primaires de cellules épithéliales respiratoires humaines saines isolées à partir de cornets ou de polypes nasaux collectés chez des patients devant subir une septoplastie, une turbinectomie ou une polypectomie. Les cellules ont ensuite été cultivées sur support poreux recouvert de collagène de type IV (Transwell® clear, polyester, 0.4 µm, Costar) en interface air-liquide (ALI) dans le but d'induire la différenciation (Puchelle, E *et al*., 2006). Les caractéristiques morphologiques et physiologiques de l'épithélium respiratoire ont été par ailleurs bien décrites (Puchelle, E *et al*., 2006).

### I-A.1. Matériels et méthodes

### Patients et échantillons tissulaires

Des cornets inférieurs ou des polypes nasaux ont été collectés à partir de 3 patients devant subir une turbinectomies ou des polypectomies pour obstruction nasale ou septoplastie (Dr. Castillo, Département ORL, Hôpital Pasteur, Nice, France). Les patients atteints d'asthme, de mucoviscidose ou d'allergies ont été exclus de l'étude. Toutes les procédures ont été approuvées par le comité d'éthique de l'Université de Nice-Sophia-Antipolis.

### Isolement et culture de cellules épithéliales respiratoires humaines saines (HAECs)

Les cultures primaires de HAECs saines dérivées de muqueuse nasale ont été réalisées à l'aide d'une méthode adaptée d'études précédentes (Wu *et al*., 1985; Marcet *et al*., 2007; LeSimple *et al*., 2007). Après excision, le tissu est immédiatement immergé dans une solution équilibrée en sels (HBSS (sans Ca²⁺/Mg²⁺) Invitrogen) contenant de l'HEPES (25 mM, Invitrogen), 100 U/ml pénicilline (Gibco, Invitrogen), 100 mg/ml streptomycine (Gibco, Invitrogen), 100 mg/ml de sulfate de gentamicine (Gibco, Invitrogen), et 2,5 mg/ml d'amphotéricine B (Gibco, Invitrogen). Après 3 rinçages avec du milieu HBSS-HEPES-antibiotiques, le tissu est digéré avec 0,1% de pronase (Sigma) à 4°C, toute la nuit. Le tissu est ensuite délicatement retiré du milieu de digestion, et les cellules épithéliales respiratoires à la surface de la muqueuse nasale sont détachées du stroma par de légères agitations avec du milieu HBSS-HEPES-antibiotiques contenant 10% de sérum de veau foetal (SVF). La suspension cellulaire est centrifugée à 150g, 10 min à 4°C et le culot cellulaire est resuspendu dans 10% SVF-HBSS-HEPES-antibiotiques et centrifugé à nouveau. Le second culot cellulaire est alors resuspendu dans un milieu 10% SVF-antibiotiques-Dulbecco's modified Eagle's (DMEM, Invitrogen) à l'aide d'une aiguille (0,8 mm) et d'une seringue de 10 ml pour dissocier les agrégats cellulaires. Les cellules sont ensuite ensemencées (10⁴/cm²) sur des supports poreux perméables (Transwell®, Costar, Sigma) recouverts de collagène de type IV (Sigma) et incubées dans une atmosphère humidifiée à 37°C, 5% CO₂. Le lendemain, le milieu de culture est remplacé par du milieu de prolifération (BEGM) reconstitué à l'aide du milieu BEBM (Lonza) contenant les suppléments hormonaux et autres facteurs de croissances suivants : insuline, apo-transferrine, facteur de croissance épidermique (EGF), épinéphrine, hydroxycortisone, 3,30,5-triiodothyronine, supplément de croissance pour cellules endothéliales (endothelial cell growth supplement), acide rétinoïque (à faible concentration, environ 10 nM), amphotéricine B (2,5 mg/ml), streptomycine (100 mg/ml), pénicilline (100 U/ml), sulfate de gentamicine (50 mg/ml) et L-glutamine (2 mM).

La confluence de la monocouche de cellules épithéliales respiratoires est typiquement achevée après 7 jours de culture. Le milieu de surface apicale est alors retiré afin de mettre les cellules en interface air-liquide et le milieu basolatéral est changé par du milieu de différenciation : BEBM/DMEM ratio (1 : 1) et contenant les mêmes suppléments que cités ci-dessus mis à part l'acide rétinoïque qui est cette fois ajouté à forte concentration, environ 300 nM, pour induire la différenciation mucociliaire de l'épithélium respiratoire. Les expériences sont ensuite réalisées sur des cultures différenciées ayant une résistance transépithéliale supérieure à 500 ohm/cm².

### Marquage immunocytochimique et microscopie confocale et électronique

Les coupes de membranes de cultures de cellules respiratoires cultivées en interface air-liquide sont fixées dans l'acétone ou dans le méthanol (10 min, -20°C). Après rinçage dans du PBS, les sites non-spécifiques sont bloqués dans du PBS-BSA 3%, puis l'anticorps primaire est incubé dans du PBS-BSA-1% de 1h à température ambiante à 16h à 4°C selon l'anticorps utilisé. Après rinçage, l'anticorps secondaire couplé et mis en solution dans du PBS-BSA 1% est incubé 1 h à température ambiante. Après rinçage, les noyaux sont marqués au DAPI et contre colorés à l'hématoxyline de Harris. Après rinçage, les lames sont montées et observées au microscope confocal. Dans le cas de doubles marquages séquentiels impliquant deux anticorps de souris, une étape de blocage des sites libres des anticorps primaires est réalisée à l'aide d'anticorps anti-souris anti-Fab (H+L) 30 minutes à température ambiante.

Pour la microscopie électronique, les cellules sont fixées dans du tampon phosphate monosodique 0.1M contenant 1,6% de glutaraldéhyde.

### Tri des cellules épithéliales respiratoires

Après dissociation du stroma de polypes nasaux, les cellules épithéliales (environ 20.10⁶) sont incubées en PBS-BSA-EDTA pendant 30 min puis incubées avec l'anticorps marqué CD151-PE et l'anti-FT (facteur tissulaire)-FITC pendant 20 min, 4°C (voir Hajj, R. et al., Stem Cells 25, 139-148 (2007)). Après deux lavages, les cellules sont reprises dans du PBS-EDTA et incubées avec du DAPI pour marquer les cellules dont l'intégrité est altérée.

Le tri cellulaire a été effectué sur le trieur FACSAria (BD Biosciences) équipé de 3 lasers, bleu, rouge et violet. Les cellules mortes, les agrégats et les doublets cellulaires ont été exclus. La population doublement positive de cellules basales (CD151+/FT+) et la population négative de cellules cylindriques (contenant les cellules ciliées et les cellules sécrétrices de mucus) (CD151-/FT-) ont été sélectionnées et triées à la vitesse de 5000 événements/s (30 MHz de fréquence). La pureté et l'identité des cellules triées ont ensuite été vérifiées par cytomètre et immunocytochimie.

### I-A.2. Observation de la morphologie des cellules marquées à l'hématoxyline et à l'éosine

Des immunomarquages ont été réalisés afin d'examiner l'expression de marqueurs épithéliaux de différenciation (tubuline-β4 pour les cellules ciliées (**Figure1E-F**), mucine MUC5AC pour les cellules sécrétrices à mucus (**Figure 1G-H**), et la cytokératine 13 pour les cellules basales (**Figure 1I-J**)).

Les états de différenciation ont été évalués par microscopie confocale et électronique. Il a été choisi d'étudier quatre temps clés de la différenciation :
(1) durant les premiers jours de l'établissement de l'interface air-liquide (ALI-D0), les cellules prolifèrent et forment un épithélium squameux stratifié;
(2) après 5-7 jours d'interface air-liquide (ALI-D7), la polarisation cellulaire débute;
(3) après environ 14 jours d'interface air-liquide, les cellules forment un épithélium pseudostratifié où les premiers cils apparaissent à la surface des cellules (ALI-FC), ainsi qu'un allongement cellulaire, indiquant le début de la ciliogénèse (**Figure 1C**);
(4) après environ 21 jours d'interface air-liquide (ALI-WD), l'épithélium respiratoire devient pseudostratifié, stade complètement différencié où la plupart des cellules sont cylindriques et ciliées, avec une couche sous-jacente de cellules basales, ainsi que des cellules sécrétrices de mucus (**Figure 1D**).

Après 3-4 semaines de culture, les critères morphologiques et les marqueurs spécifiques de différenciation des 3 types cellulaires de l'épithélium respiratoire de surface (i.e. basales (**Figure 1I-J**), sécrétrices (**Figure 1G-H**), et ciliées (**Figure 1E-F**) peuvent être observés.

### II- Mesure de l'expression des miARN dans les cellules sélectionnées au cours de la ciliogénèse

Les techniques de séquençage à haut-débit permettent d'établir l'abondance de certains miARN au sein d'un mélange complexe.

Sur la base du nombre de séquences spécifiques d'un miARN au sein d'une condition expérimentale, il est possible d'évaluer l'abondance dudit miARN au sein de l'ensemble des miARN.

### II-1. Matériels et méthodes

### Cellules épidermiques ciliées d'embryons de Xenopus laevis

L'épiderme cilié des embryons de *Xenopus laevis* est utilisé comme un modèle d'épithélium mucociliaire comme décrit par Hayes et al. (Dev Biol 312 (1), 115 (2007)).

### Extraction d'ARN totaux et contrôles qualité

Les ARN totaux ont été extraits à partir des HAECs cultivées en interface air-liquide à quatre stades de différenciation : ALI-D0 (0 jour), ALI-D7 (7 jours), ALI-FC (lors de l'apparition des premiers cils à environ 14 jours) et ALI-WD (pleinement différenciées à environ 21 jours).Les cellules sont lysées dans du réactif au Trizol® (Invitrogen). Les ARN totaux contenant les petits ARN et les microARN sont purifiés sur colonnes Qiagen RNEasy (Qiagen) selon les instructions du fournisseur.

La pureté et la concentration des échantillons d'ARN totaux sont d'abord évalués en utilisant le spectrophotomètre Nanodrop. Les ratios 260/280 (ratio des valeurs d'absorbance à 260 et 280 nm d'un échantillon mesurées à l'aide d'un spectrophotomètre) et 260/230 (ratio des valeurs d'absorbance à 260 et 230 nm d'un échantillon mesurées à l'aide d'un spectrophotomètre) ; ces ratios reflètent la pureté en ARN lorsqu'ils sont compris entre 1,5 et 2 ; ils sont vérifiés et doivent avoir une valeur proche de 2.

Les ARN totaux de cellules embryonnaires de *Xenope* ont été purifies avec un kit "Qiagen RNeasy kit » (Qiagen).

Les ARN sont ensuite chargés sur une puce « RNAnano chip » (Agilent Technologie, France) et leur qualité (intégrité et niveau de dégradation des ARN) est analysée à l'aide du Bioanalyzer System (Agilent Technologies, France).

### Séquençage haut-débit de microARN

Les ARN totaux contenant les petits ARN et les microARN sont isolés comme précédemment. La procédure est basée sur la technologie Applied Biosystems Ligase-Enhanced Genome Detection (LEGenD™) ; le kit SOLiD™ Small RNA Expression (Applied Biosystems, France) a été utilisé. Cette méthode permet de convertir les petits ARN d'un échantillon dans une librairie d'ADN double brin ; elle a été développée par Applied Biosystems SOLiD™ System pour faire du séquençage haut-débit de nouvelle génération.

Le séquençage haut-débit des microARN a été réalisé selon les recommandations du fournisseur. Brièvement, les ARN totaux contenant les petits ARN sont hybridés (65°C, 10 min, puis à 16°C, 5 min) et ligués (16°C, 2-16 h dans un thermocycleur) à l'Adaptor Mix A pour produire une matrice pour le séquençage de l'extrémité 5' des petits ARN, ou à l'Adaptor Mix B pour séquencer l'extrémité 3'. Les échantillons sont ensuite reverse-transcrits (42°C, 30 min) pour synthétiser l'ADN complémentaire (ADNc). L'amplification de la bibliothèque des petits ARN est effectuée par PCR et après migration sur gel de polyacrylamide les petits ARN amplifiés sont découpés et extraits du gel suivant leur taille (environ 105-150 bases de longueur, selon les instructions du fournisseur), élués et resuspendus dans de l'eau exempte de nucléases. La concentration d'acides nucléiques est ensuite mesurée et normalisée avant de procéder à la préparation des échantillons pour le séquençage.

### Analyses statistiques des données de séquençage haut-débit

Les analyses statistiques sont réalisées à l'aide du logiciel R de Bioconductor® (Peter Dalgaard, statistics and computing, Introductory statistics with R. Springer, 2002 ; R. Gentleman, V.J. Carey, W. Huber, R.A. Irizarry, S. Dudoit. Statistics for biology and health. Bioinformatics and computational biology solutions using R and bioconductor. Springer, 2005).

Pour chaque microARN séquencé, le nombre de séquences du brin 5p et du brin 3p du microARN a été normalisé à 10⁶ séquences et converti en pourcentage d'abondance d'expression pour chacun des microARN. Les données ont ensuite été normalisées suivant un modèle linéaire et une méthode Bayésienne empirique en utilisant le logiciel R. Pour les analyses ultérieures, n'ont été retenus que les microARN dont le pourcentage d'expression (ou abondance) est supérieure à 1% du total des microARN dans au moins une condition de culture, avec un |Log₂ Ratio| inférieur à 0,5 et une P-Value ajustée inférieure à 0,05.

### Analyse du microARNome par puces microARN (Agilent Technologies)

En parallèle du séquençage haut-débit des microARN, le répertoire d'expression des microARN (microARNome) est étudié à l'aide de la technologie de puces microARN Agilent®. Pour cela, les échantillons ARN des mêmes patients que précédemment ont été utilisés, marqués et hybridés sur puces miARN Agilent® (Human miRNA Microarray v2, contenant 866 miARN humains et 89 miARN viraux humains, *i.e.* l'ensemble des miARN humains contenus et référencés dans Sanger miRBase v.12.0, Agilent Technologies, France) à l'aide du kit « miRNA labelling and hybridization » suivant les instructions du fournisseur (Agilent Technologies).

### Analyses du transcriptome par puces ADN (Affymetrix®)

Pour les analyses du transcriptome, les ARN totaux sont purifiés et leur qualité est vérifiée comme précédemment.

L'analyse est ensuite réalisée sur des puces ADN GeneChip® Human Gene 1.0 ST Array (Affymetrix®). Chacun des 28869 gènes est représenté sur la puce par environ 26 sondes recouvrant la longueur entière du gène. Les ARN totaux sont marqués et hybridés à l'aide de "whole Transcript (WT) Sense Target Labeling and Control Reagents, fluidics and scanning instrumentation and basic analysis software".

### Analyses des données de transcriptome obtenues par puces ADN (Affymetrix®)

Les analyses de données sont réalisées à l'aide du logiciel R Bioconductor développé par le consortium statistique R. Puis, les données sont visualisées à l'aide de l'interface Mediante, un système d'information développé pour l'analyse et le stockage d'informations à large échelle obtenues lors d'analyses de puces (Le Brigand and Barbry, 2007).

Pour les puces Affymetrix®, les données sont analysées en utilisant l'algorithme RMA (Robust Multi-Chip Average), qui réalise une correction du bruit de fond, une étape de normalisation, et un compte-rendu des niveaux des sondes. Cette méthode montre une précision élevée, particulièrement pour des valeurs d'expression faibles, et montre une spécificité et une sensibilité plus élevée que de nombreuses autres méthodes connues (Irizarry *et al*., 2003). Les données sont normalisées suivant un modèle linéaire et une méthode Bayesienne empirique à l'aide du logiciel R Bioconductor. Des graphiques en forme de Volcano plots (Figure 7) sont utilisées pour montrer le niveau de régulation quantitatif des gènes exprimé en log2 en fonction de leur significativité statistique par rapport à des réplicas expérimentaux.

### Hybridation in situ

Après fixation dans du paraformaldéhyde à 4% (Electron Microscopy Sciences), des coupes congelées de cultures cellulaires au stade ALI-D21 ou de tissus des voies respiratoires de l'homme ont été acétylées, incubées une nuit à 55°C avec 0,3 ng/µl de sondes LNA marquées avec la digoxigénine ciblant des microARN (Exiquon, Woburn, MA) dans du formamide désionisée 50%, 0,3 M NaCl, 20 mMTris-HCl pH 8,0, 5 mM EDTA, 10 mM NaPO4 pH 8,0, 10% sulfate de dextran, 1X de solution de Denhardt, et de 0,5 mg/ml d'ARN de levure.

Les séquences des sondes sont :
pour miR-449 : ccagctaacaatacactgcc (SEQ. ID. N°204)
pour miR-31: agctatgccagcatcttgcct (SEQ. ID. N°205)
pour le microARN de contrôle négatif (« scramble ») : gtgtaacacgtctatacgccca (SEQ. ID. N°206).

Les sondes ont été révélées par incubations séquentielles à la peroxydase conuguée avec des anticorps anti-digoxigénine (Roche) avec le kit d'amplification de signal « Tyramide Signal Aplificatin Plus DNP AP System » (Perkin Elmer) sur le substrat BCIP/NBT (DakoCytomation).

Certaines lames ont ensuite été exposées à des anticorps de souris anti-MUC5AC et révélées avec le kit « LSAB2 System-HRP » (Dako). Les coupes ont été contremarquées avec le colorant éosine/safran « Nuclear Fast Red », et montées à l'aide milieu de montage Eukitt (Electron Microscopy Sciences).

Chez le Xenope, des oeufs obtenus de femelles NASCO sont fécondées *in vitro,* cultivées et injectées tel que décrit par Marchal, L et al. (Proc Natl Acad Sci U S A 106 (41), 17437 (2009)). Les ARNc de DII1 et de la centrin-2-GFP sont préparées avec le kit « Ambion mMessage Machine ». le vecteur Td-tomato-CAAX lié à la membrane (don de Chenbei Chang) est linéarisé avec l'Asel et l'ARNc est synthétisé avec la Sp6 polymerase. La lysine-dextran fluorescente (FLDx, 2.5 ng/cellule) est co-injectée avec les oligonucléotides morpholino (MO) pour marquer les embryons vivants et une immunodétection anti-fluorescéine est réalisée pour suivre la distribution des MO dans les embryons. Toutes les injections sont dupliquées.
Une sonde LNA anti-miR-449a marquée à la mono-digoxygénine (Exiqon) a été utilisée pour l'hybridation *in situ* qui a été réalisée comme décrit par Kloosterman et al. (Nat Methods 3 (1), 27 (2006)). Des sondes ribosomiques antisens de la tubulin-α, DIl1, Tex15 et Foxj1 ont été préparées comme décrit par : Deblandre et al. (Development 126 (21), 4715 (1999)) pour la tubulin-α et DII1 ; Hayes et al. (Dev Biol 312 (1), 115 (2007)) pour Tex15 et Pohl et al. (Dev Genes Evol 214 (4), 200 (2004)) pour Foxj1.

### II-2. Expression des miRNA dans les cellules sélectionnées

### II-2.A. cellules HAECs

Sur l'ensemble des miARN humains connus aujourd'hui (environ 750), les Inventeurs ont ainsi trouvé dans un premier temps à l'aide de la technique de séquençage haut-débit (HTS) de miARN que 115 miARN se retrouvaient exprimés au cours de la différenciation avec une valeur d'intensité supérieure à 8 (correspondant au Log2 du nombre normalisé de miARN séquencés).

A l'aide de la technique de séquençage, il est possible de rendre compte de l'abondance en pourcentage de chacun des miARN séquencés. Les Inventeurs ont ainsi pu établir de manière surprenante que 26 miARN étaient présents au sein des différents stades de différenciation de l'épithélium respiratoire humain avec une abondance supérieure à 1% (indiqués dans la **Figure 2** et le **Tableau IIIA** ci-dessous).

Les 26 miARN identifiés couvrent à eux-seuls de l'ordre de 80% de tous les miARN exprimés dans l'épithélium respiratoire humain ; cette quantité montre leur implication dans le processus de régénération et/ou de diffrenciation du tissu épithélial respiratoire.

Une analyse plus fine de ces 26 miARN montre que 22 sont significativement régulés dans au moins un des quatre stades de différenciation de l'épithélium respiratoire humain et représentent environ 70 % du total des miARN : 13 miARN sont surexprimés tandis que 9 miARN sont réprimés (voir la Figure 2B et le **Tableau IV** ci-après).

La valeur de modulation (aussi désignée ratio du niveau d'intensité d'expression en log2 à deux stades de différenciation) est calculée par la différence entre les niveaux d'intensité d'expression en log2 aux deux stades de différenciation indiqués en haut de chaque colonne.

**Tableau IIIA. miARN significativement exprimés dans l'épithélium respiratoire humain et avec une abondance supérieur à 1% à au moins un stade de différenciation, identifiés par séquençage de miARN haut-débit (HTS).**

| | | | **Niveau d'intensité d'expression en log2** | | | | **Abondance (%)** | | | | **Modulation (ratio du niveau d'intensité d'expression en log2 à deux stades de différenciation)** | | | |
|---|---|---|---|---|---|---|---|---|---|---|---|---|---|---|
| **Nom** | **Pre-mir** | **brins** | **D0** | **D7** | **FC** | **WD** | **D0** | **D7** | **FC** | **WD** | **D7-D0** | **FC-D7** | **WD-FC** | **WD-D0** |
| hsa-miR-449a | hsa-mir-449a | 5p | 4,48 | 9,69 | 14,90 | 15,11 | 0,00 | 0,18 | 6,49 | 5,70 | 5,21 | 5,21 | 0,21 | 10,63 |
| hsa-miR-449b | hsa-mir-449b | 5p | 3,64 | 6,96 | 12,36 | 12,24 | 0,00 | 0,03 | 1,12 | 0,78 | 3,32 | 5,40 | -0,12 | 8,60 |
| hsa-miR-449b* | hsa-mir-449b | 3p | 6,40 | 10,23 | 13,59 | 13,84 | 0,02 | 0,26 | 2,62 | 2,37 | 3,83 | 3,36 | 0,26 | 7,45 |
| hsa-miR-449c | hsa-mir-449c | | 1,54 | 4,13 | 11,57 | 10,98 | 0,00 | 0,00 | 0,32 | 0,20 | 2,59 | 7,44 | -0,59 | 9,44 |
| hsa-miR-34b | hsa-mir-34b | 3p | 7,01 | 8,26 | 12,43 | 13,75 | 0,03 | 0,07 | 1,17 | 2,22 | 1,25 | 4,17 | 1,32 | 6,74 |
| hsa-miR-34c-5p | hsa-mir-34c | 5p | 7,88 | 9,21 | 13,23 | 14,29 | 0,05 | 0,13 | 2,05 | 3,23 | 1,33 | 4,02 | 1,06 | 6,41 |
| hsa-miR-92b | hsa-mir-92b | 3p | 7,49 | 8,70 | 12,82 | 13,42 | 0,04 | 0,09 | 1,54 | 1,77 | 1,21 | 4,12 | 0,60 | 5,93 |
| hsa-miR-1975 | hsa-mir-1975 | 3p | 11,08 | 13,22 | 13,55 | 14,01 | 0,48 | 2,09 | 2,56 | 2,67 | 2,13 | 0,34 | 0,46 | 2,93 |
| hsa-miR-99a | hsa-mir-99a | 5p | 9,77 | 11,19 | 11,96 | 12,65 | 0,19 | 0,52 | 0,85 | 1,03 | 1,43 | 0,77 | 0,69 | 2,88 |
| hsa-miR-191 | hsa-mir-191 | 5p | 14,67 | 15,57 | 16,40 | 16,82 | 5,71 | 10,68 | 18,46 | 18,69 | 0,90 | 0,84 | 0,42 | 2,15 |
| hsa-miR-378 | hsa-mir-378 | 3p | 10,37 | 12,53 | 12,26 | 11,87 | 0,29 | 1,30 | 1,04 | 0,61 | 2,15 | -0,26 | -0,39 | 1,50 |
| hsa-miR-23b | hsa-mir-23b | 3p | 14,06 | 15,35 | 15,18 | 15,35 | 3,73 | 9,19 | 7,92 | 6,72 | 1,30 | -0,17 | 0,16 | 1,29 |
| hsa-miR-125a-5p | hsa-mir-125a | 5p | 12,92 | 13,55 | 13,29 | 14,08 | 1,69 | 2,64 | 2,13 | 2,79 | 0,64 | -0,27 | 0,79 | 1,16 |
| hsa-miR-203 | hsa-mir-203 | 3p | 13,49 | 13,60 | 13,39 | 14,57 | 2,53 | 2,73 | 2,29 | 3,92 | 0,11 | -0,21 | 1,18 | 1,08 |
| hsa-miR-574-3p | hsa-mir-574 | 3p | 13,64 | 14,12 | 14,24 | 14,56 | 2,80 | 3,92 | 4,12 | 3,90 | 0,48 | 0,12 | 0,32 | 0,92 |
| hsa-miR-29c | hsa-mir-29c | 3p | 13,66 | 14,48 | 14,50 | 14,53 | 2,84 | 5,03 | 4,95 | 3,82 | 0,82 | 0,02 | 0,03 | 0,87 |
| hsa-miR-140-3p | hsa-mir-140 | 3p | 12,39 | 12,72 | 13,08 | 13,12 | 1,17 | 1,48 | 1,85 | 1,44 | 0,33 | 0,37 | 0,04 | 0,73 |
| hsa-miR-205 | hsa-mir-205 | 5p | 14,61 | 15,23 | 14,11 | 14,55 | 5,48 | 8,43 | 3,76 | 3,87 | 0,62 | -1,12 | 0,44 | -0,06 |
| hsa-miR-23a | hsa-mir-23a | 3p | 14,42 | 14,47 | 13,67 | 14,10 | 4,79 | 4,99 | 2,77 | 2,83 | 0,06 | -0,80 | 0,43 | -0,31 |
| hsa-miR-31 | hsa-mir-31 | 5p | 13,69 | 13,31 | 12,40 | 12,74 | 2,89 | 2,24 | 1,15 | 1,10 | -0,37 | -0,91 | 0,34 | -0,95 |
| hsa-miR-21 | hsa-mir-21 | 5p | 16,40 | 15,66 | 14,50 | 15,24 | 19,01 | 11,41 | 4,94 | 6,26 | -0,74 | -1,16 | 0,74 | -1,16 |
| hsa-miR-17 | hsa-mir-17 | 5p | 12,85 | 12,68 | 11,81 | 11,61 | 1,61 | 1,44 | 0,76 | 0,50 | -0,17 | -0,87 | -0,20 | -1,24 |
| hsa-miR-29a | hsa-mir-29a | 3p | 16,24 | 14,90 | 14,79 | 14,81 | 17,01 | 6,72 | 6,02 | 4,64 | -1,34 | -0,11 | 0,02 | -1,43 |
| hsa-miR-193b | hsa-mir-193b | 3p | 13,26 | 12,09 | 11,19 | 11,67 | 2,16 | 0,96 | 0,50 | 0,52 | -1,17 | -0,90 | 0,48 | -1,60 |
| hsa-miR-31* | hsa-mir-31 | 3p | 13,56 | 12,91 | 11,60 | 11,83 | 2,64 | 1,69 | 0,66 | 0,59 | -0,65 | -1,31 | 0,24 | -1,73 |
| hsa-miR-210 | hsa-mir-210 | 3p | 12,29 | 11,33 | 10,60 | 10,49 | 1,10 | 0,57 | 0,33 | 0,23 | -0,96 | -0,74 | -0,10 | -1,80 |
| hsa-miR-130a | hsa-mir-130a | 3p | 13,91 | 12,51 | 12,71 | 11,98 | 3,38 | 1,28 | 1,42 | 0,65 | -1,40 | 0,19 | -0,73 | -1,94 |

**Tableau IV. miARN significativement exprimés et modulés au cours de la différenciation de l'épithélium respiratoire, avec une abondance supérieur à 1% identifiés par séquençage de miARN haut-débit (HTS).**

| | | | **Niveau d'intensité d'expression en log2** | | | | **Abondance (%)** | | | | **Modulation (ratio du niveau d'intensité d'expression en log2 à deux stades de différenciation)** | | | |
|---|---|---|---|---|---|---|---|---|---|---|---|---|---|---|
| **Nom** | **Pre-mir** | **brins** | **D0** | **D7** | **FC** | **WD** | **D0** | **D7** | **FC** | **WD** | **D7-D0** | **FC-D7** | **WD-FC** | **WD-D0** |
| hsa-miR-449a | hsa-mir-449a | 5p | 4,48 | 9,69 | 14,90 | 15,11 | 0,00 | 0,18 | 6,49 | 5,70 | 5,21 | 5,21 | 0,21 | 10,63 |
| hsa-miR-449b | hsa-mir-449b | 5p | 3,64 | 6,96 | 12,36 | 12,24 | 0,00 | 0,03 | 1,12 | 0,78 | 3,32 | 5,40 | -0,12 | 8,60 |
| hsa-miR-449b* | hsa-mir-449b | 3p | 6,40 | 10,23 | 13,59 | 13,84 | 0,02 | 0,26 | 2,62 | 2,37 | 3,83 | 3,36 | 0,26 | 7,45 |
| hsa-miR-449c | hsa-mir-449c | | 1,54 | 4,13 | 11,57 | 10,98 | 0,00 | 0,00 | 0,32 | 0,20 | 2,59 | 7,44 | -0,59 | 9,44 |
| hsa-miR-34b | hsa-mir-34b | 3p | 7,01 | 8,26 | 12,43 | 13,75 | 0,03 | 0,07 | 1,17 | 2,22 | 1,25 | 4,17 | 1,32 | 6,74 |
| hsa-miR-34c-5p | hsa-mir-34c | 5p | 7,88 | 9,21 | 13,23 | 14,29 | 0,05 | 0,13 | 2,05 | 3,23 | 1,33 | 4,02 | 1,06 | 6,41 |
| hsa-miR-92b | hsa-mir-92b | 3p | 7,49 | 8,70 | 12,82 | 13,42 | 0,04 | 0,09 | 1,54 | 1,77 | 1,21 | 4,12 | 0,60 | 5,93 |
| hsa-miR-1975 | hsa-mir-1975 | 3p | 11,08 | 13,22 | 13,55 | 14,01 | 0,48 | 2,09 | 2,56 | 2,67 | 2,13 | 0,34 | 0,46 | 2,93 |
| hsa-miR-99a | hsa-mir-99a | 5p | 9,77 | 11,19 | 11,96 | 12,65 | 0,19 | 0,52 | 0,85 | 1,03 | 1,43 | 0,77 | 0,69 | 2,88 |
| hsa-miR-191 | hsa-mir-191 | 5p | 14,67 | 15,57 | 16,40 | 16,82 | 5,71 | 10,68 | 18,46 | 18,69 | 0,90 | 0,84 | 0,42 | 2,15 |
| hsa-miR-378 | hsa-mir-378 | 3p | 10,37 | 12,53 | 12,26 | 11,87 | 0,29 | 1,30 | 1,04 | 0,61 | 2,15 | -0,26 | -0,39 | 1,50 |
| hsa-miR-23b | hsa-mir-23b | 3p | 14,06 | 15,35 | 15,18 | 15,35 | 3,73 | 9,19 | 7,92 | 6,72 | 1,30 | -0,17 | 0,16 | 1,29 |
| hsa-miR-125a-5p | hsa-mir-125a | 5p | 12,92 | 13,55 | 13,29 | 14,08 | 1,69 | 2,64 | 2,13 | 2,79 | 0,64 | -0,27 | 0,79 | 1,16 |
| hsa-miR-203 | hsa-mir-203 | 3p | 13,49 | 13,60 | 13,39 | 14,57 | 2,53 | 2,73 | 2,29 | 3,92 | 0,11 | -0,21 | 1,18 | 1,08 |
| hsa-miR-205 | hsa-mir-205 | 5p | 14,61 | 15,23 | 14,11 | 14,55 | 5,48 | 8,43 | 3,76 | 3,87 | 0,62 | -1,12 | 0,44 | -0,06 |
| hsa-miR-31 | hsa-mir-31 | 5p | 13,69 | 13,31 | 12,40 | 12,74 | 2,89 | 2,24 | 1,15 | 1,10 | -0,37 | -0,91 | 0,34 | -0,95 |
| hsa-miR-21 | hsa-mir-21 | 5p | 16,40 | 15,66 | 14,50 | 15,24 | 19,01 | 11,41 | 4,94 | 6,26 | -0,74 | -1,16 | 0,74 | -1,16 |
| hsa-miR-17 | hsa-mir-17 | 5p | 12,85 | 12,68 | 11,81 | 11,61 | 1,61 | 1,44 | 0,76 | 0,50 | -0,17 | -0,87 | -0,20 | -1,24 |
| hsa-miR-29a | hsa-mir-29a | 3p | 16,24 | 14,90 | 14,79 | 14,81 | 17,01 | 6,72 | 6,02 | 4,64 | -1,34 | -0,11 | 0,02 | -1,43 |
| hsa-miR-193b | hsa-mir-193b | 3p | 13,26 | 12,09 | 11,19 | 11,67 | 2,16 | 0,96 | 0,50 | 0,52 | -1,17 | -0,90 | 0,48 | -1,60 |
| hsa-miR-31* | hsa-mir-31 | 3p | 13,56 | 12,91 | 11,60 | 11,83 | 2,64 | 1,69 | 0,66 | 0,59 | -0,65 | -1,31 | 0,24 | -1,73 |
| hsa-miR-210 | hsa-mir-210 | 3p | 12,29 | 11,33 | 10,60 | 10,49 | 1,10 | 0,57 | 0,33 | 0,23 | -0,96 | -0,74 | -0,10 | -1,80 |
| hsa-miR-130a | hsa-mir-130a | 3p | 13,91 | 12,51 | 12,71 | 11,98 | 3,38 | 1,28 | 1,42 | 0,65 | -1,40 | 0,19 | -0,73 | -1,94 |

Les Inventeurs ont ensuite utilisé les puces miARN Agilent pour étudier le répertoire d'expression des miARN de l'épithélium respiratoire humain et comparer les résultats obtenus en séquençage haut-débit avec ceux obtenus avec des puces commerciales miARN Agilent®. A l'aide de cette technique, 48 miARN sont retrouvés significativement exprimés et modulés (Log2 du niveau d'intensité de l'expression > 8 ; 1 < log₂(ratio) < -1 et une valeur P ajustée < 0,05) dans au moins un des quatre stades de différenciation (voir la **Figure 3** et le **Tableau V**).

**Tableau V. miARN significativement exprimés et modulés au cours de la différenciation de l'épithélium respiratoire identifiés par puces miARN Agilent®**

| | **Niveau d'intensité d'expression en log2** | | | | **Modulation (ratio du niveau d'intensité d'expression en log2 à deux stades de différenciation)** | | | |
|---|---|---|---|---|---|---|---|---|
| **Nom** | **D0** | **D7** | **FC** | **WD** | **D7-D0** | **FC-D7** | **WD-FC** | **WD-D0** |
| hsa-miR-449a | 1,47 | 8,00 | 13,70 | 13,54 | 6,53 | 5,70 | -0,15 | 12,08 |
| hsa-miR-449b | 0,47 | 3,08 | 9,33 | 9,11 | 2,61 | 6,25 | -0,22 | 8,64 |
| hsa-miR-34b | 0,77 | 3,65 | 8,13 | 8,94 | 2,88 | 4,48 | 0,81 | 8,17 |
| hsa-miR-34c-5p | 3,94 | 6,29 | 10,56 | 11,39 | 2,35 | 4,27 | 0,82 | 7,44 |
| hsa-miR-34b* | 5,96 | 7,98 | 11,35 | 12,44 | 2,02 | 3,36 | 1,10 | 6,48 |
| hsa-miR-99a | 5,02 | 7,35 | 8,12 | 8,31 | 2,33 | 0,77 | 0,19 | 3,28 |
| hsa-miR-30b | 7,62 | 9,98 | 10,03 | 10,17 | 2,36 | 0,05 | 0,15 | 2,55 |
| hsa-miR-768-3p | 7,43 | 9,51 | 9,81 | 9,96 | 2,08 | 0,30 | 0,14 | 2,53 |
| hsa-miR-30e | 6,73 | 9,50 | 9,25 | 9,12 | 2,76 | -0,25 | -0,13 | 2,38 |
| hsa-miR-200a | 8,64 | 10,58 | 10,79 | 10,84 | 1,94 | 0,20 | 0,06 | 2,20 |
| hsa-miR-26a | 8,50 | 10,70 | 10,59 | 10,65 | 2,20 | -0,11 | 0,06 | 2,15 |
| hsa-miR-200b | 9,93 | 11,94 | 11,87 | 12,04 | 2,01 | -0,07 | 0,17 | 2,11 |
| hsa-miR-429 | 7,97 | 9,68 | 9,82 | 9,93 | 1,71 | 0,14 | 0,10 | 1,95 |
| hsa-miR-34a | 8,58 | 10,89 | 10,35 | 10,49 | 2,31 | -0,54 | 0,13 | 1,91 |
| hsa-miR-141 | 11,15 | 13,31 | 13,03 | 13,03 | 2,16 | -0,28 | 0,00 | 1,88 |
| hsa-miR-15a | 7,74 | 10,10 | 9,71 | 9,61 | 2,36 | -0,39 | -0,10 | 1,87 |
| hsa-miR-30d | 7,06 | 8,94 | 8,88 | 8,92 | 1,88 | -0,06 | 0,05 | 1,86 |
| hsa-miR-23b | 8,92 | 10,88 | 10,61 | 10,64 | 1,96 | -0,27 | 0,04 | 1,72 |
| hsa-miR-224 | 6,84 | 9,40 | 8,52 | 8,52 | 2,55 | -0,87 | 0,00 | 1,68 |
| hsa-miR-26b | 7,97 | 9,91 | 9,54 | 9,62 | 1,94 | -0,37 | 0,08 | 1,65 |
| hsa-miR-148a | 7,35 | 9,41 | 8,91 | 8,95 | 2,06 | -0,50 | 0,04 | 1,60 |
| hsa-miR-16 | 9,47 | 11,54 | 11,18 | 11,06 | 2,07 | -0,36 | -0,11 | 1,59 |
| hsa-miR-29c | 9,04 | 10,66 | 10,38 | 10,62 | 1,61 | -0,28 | 0,24 | 1,57 |
| hsa-miR-425 | 6,61 | 8,21 | 8,39 | 8,18 | 1,59 | 0,19 | -0,21 | 1,57 |
| hsa-miR-30c | 7,80 | 9,77 | 9,43 | 9,31 | 1,97 | -0,34 | -0,12 | 1,51 |
| hsa-let-7g | 9,30 | 11,12 | 10,59 | 10,80 | 1,83 | -0,54 | 0,21 | 1,50 |
| hsa-miR-151-5p | 6,71 | 8,54 | 8,17 | 8,17 | 1,83 | -0,37 | 0,00 | 1,46 |
| hsa-miR-200c | 10,20 | 12,01 | 11,54 | 11,64 | 1,82 | -0,47 | 0,09 | 1,44 |
| hsa-miR-27b | 9,26 | 11,03 | 10,57 | 10,62 | 1,77 | -0,47 | 0,05 | 1,36 |
| hsa-let-7f | 11,11 | 12,75 | 12,01 | 12,19 | 1,63 | -0,73 | 0,17 | 1,08 |
| hsa-let-7c | 9,10 | 10,71 | 10,00 | 10,15 | 1,61 | -0,71 | 0,14 | 1,05 |
| hsa-let-7a | 11,54 | 13,16 | 12,47 | 12,55 | 1,62 | -0,69 | 0,08 | 1,01 |
| hsa-let-7b | 11,00 | 12,66 | 11,89 | 11,96 | 1,67 | -0,77 | 0,07 | 0,97 |
| hsa-let-7e | 8,53 | 9,91 | 9,34 | 9,48 | 1,38 | -0,57 | 0,15 | 0,95 |
| hsa-miR-21 | 14,27 | 15,83 | 15,15 | 15,20 | 1,56 | -0,69 | 0,06 | 0,93 |
| hsa-miR-374a | 7,04 | 8,45 | 7,91 | 7,83 | 1,41 | -0,54 | -0,08 | 0,79 |
| hsa-miR-125a-5p | 7,37 | 8,66 | 8,20 | 8,15 | 1,29 | -0,46 | -0,04 | 0,79 |
| hsa-miR-939 | 6,64 | 8,04 | 7,41 | 7,34 | 1,40 | -0,63 | -0,07 | 0,70 |
| hsa-miR-106b | 8,08 | 9,47 | 8,94 | 8,77 | 1,39 | -0,53 | -0,18 | 0,69 |
| hsa-miR-25 | 7,15 | 8,50 | 7,85 | 7,80 | 1,35 | -0,65 | -0,05 | 0,66 |
| hsa-miR-96 | 8,52 | 9,71 | 9,21 | 9,16 | 1,19 | -0,50 | -0,04 | 0,64 |
| hsa-miR-22 | 10,67 | 11,75 | 11,10 | 11,15 | 1,09 | -0,66 | 0,05 | 0,48 |
| hsa-miR-181a | 7,05 | 8,47 | 7,62 | 7,25 | 1,41 | -0,84 | -0,37 | 0,20 |
| hsa-miR-21* | 7,28 | 8,49 | 7,75 | 7,46 | 1,21 | -0,74 | -0,29 | 0,18 |
| hsa-miR-365 | 8,44 | 8,41 | 7,21 | 7,25 | -0,02 | -1,21 | 0,05 | -1,18 |
| hsa-miR-130a | 8,83 | 9,48 | 7,95 | 7,52 | 0,65 | -1,53 | -0,43 | -1,31 |
| hsa-miR-100 | 8,00 | 7,55 | 6,29 | 6,52 | -0,46 | -1,25 | 0,22 | -1,49 |
| hsa-miR-193b | 8,62 | 7,95 | 6,76 | 6,69 | -0,67 | -1,19 | -0,07 | -1,93 |

En comparant les miARN que l'on retrouve significativement modulés et exprimés dans au moins une condition de culture cellulaire en séquençage haut-débit de miARN (HTS) ou en puces miARN Agilent®, il est obtenu une forte corrélation entre les résultats obtenus avec les deux techniques, avec un coefficient de corrélation r=0,9106 (voir la **Figure 4**).

En résumé, 26 miARN ont été détectés et sélectionnés en HTS dans au moins une des conditions de culture (avec une abondance d'expression supérieure à 1%) et 48 miARN sur puces miARN Agilent® (avec un niveau d'intensité d'expression en log2 supérieure à 8). Ce qui fait en comptant les miARN communs obtenus par les deux techniques, 61 miARN distincts significativement exprimés dans au moins une des conditions de différenciation de l'épithélium (60 figurent dans le **Tableau VI** auxquels il faut ajouter hsa-miR-1975, voir le **Tableau III**).

Les 22 miARN identifiés comme significativement régulés et suffisamment abondants en HTS, sont retrouvés aussi modulés sur puces miARN Agilent® avec plus ou moins de significativité et avec des intensités plus ou moins fortes.

Comme décrit plus haut, les miARN sont synthétisés sous forme de précurseurs ayant une structure en épingle à cheveux puis subissent une dernière maturation par l'enzyme Dicer pour donner deux petits ARN simple brin (5p et 3p) dont un des deux brins dit mature va interagir avec le complexe RISC pour exercer sa fonction modulatrice tandis que l'autre brin complémentaire dit star (annoté mir-xy*) va être dégradé. De ce fait, les Inventeurs ont mesuré systématiquement les deux brins de chacun des miARN à l'aide des différentes techniques utilisées (puces miARN, séquençage, PCR). Les Inventeurs ont pu observer que pour certains des miARN sélectionnés les deux brins (5p et 3p) sont modulés, mais un seul des deux brins pour un miARN sélectionné donné est retrouvé plus abondamment exprimé.

**Tableau VI. Comparaison des miARN modulés et exprimés entre puces miARN Agilent® et HTS**

| | **Modulation (ratio du niveau d'intensité d'expression en log2 à deux stades de différenciation) mesurée avec les puces Agilent** | | | | **Modulation (ratio du niveau d'intensité d'expression en log2 à deux stades de différenciation) mesurée par séquençage à haut débit** | | | | **Niveau d'intensité d'expression en log2 mesuré avec les puces Agilent** | | | | **Niveau d'intensité d'expression en log2 mesuré par séquençage à haut débit** | | | |
|---|---|---|---|---|---|---|---|---|---|---|---|---|---|---|---|---|
| **Nom** | **D7-D0** | **FC-D7** | **WD-FC** | **WD-D0** | **D7-D0** | **FC-D7** | **WD-FC** | **WD-D0** | **D0** | **D7** | **FC** | **WD** | **D0** | **D7** | **FC** | **WD** |
| hsa-let-7a | 1,62 | -0,69 | 0,08 | 1,01 | -0,64 | 0,28 | 0,88 | 0,51 | 11,54 | 13,16 | 12,47 | 12,55 | 3,80 | 3,16 | 3,43 | 4,31 |
| hsa-let-7b | 1,67 | -0,77 | 0,07 | 0,97 | -0,57 | -0,03 | 0,66 | 0,05 | 11,00 | 12,66 | 11,89 | 11,96 | 11,23 | 10,65 | 10,62 | 11,28 |
| hsa-let-7c | 1,61 | -0,71 | 0,14 | 1,05 | 0,14 | 0,21 | 0,55 | 0,90 | 9,10 | 10,71 | 10,00 | 10,15 | 6,96 | 7,10 | 7,31 | 7,86 |
| hsa-let-7e | 1,38 | -0,57 | 0,15 | 0,95 | 0,38 | -0,27 | 0,07 | 0,18 | 8,53 | 9,91 | 9,34 | 9,48 | 7,42 | 7,80 | 7,53 | 7,60 |
| hsa-let-7f | 1,63 | -0,73 | 0,17 | 1,08 | 0,22 | -0,56 | 0,45 | 0,11 | 11,11 | 12,75 | 12,01 | 12,19 | 5,24 | 5,46 | 4,90 | 5,35 |
| hsa-let-7g | 1,83 | -0,54 | 0,21 | 1,50 | -0,24 | -0,03 | 0,83 | 0,56 | 9,30 | 11,12 | 10,59 | 10,80 | 11,84 | 11,60 | 11,57 | 12,40 |
| hsa-miR-100 | -0,46 | -1,25 | 0,22 | -1,49 | -1,05 | -2,36 | 1,69 | -1,72 | 8,00 | 7,55 | 6,29 | 6,52 | 10,72 | 9,67 | 7,32 | 9,00 |
| hsa-miR-106b | 1,39 | -0,53 | -0,18 | 0,69 | 1,33 | -0,28 | -0,09 | 0,96 | 8,08 | 9,47 | 8,94 | 8,77 | 4,43 | 5,76 | 5,48 | 5,39 |
| hsa-miR-125a-5p | 1,29 | -0,46 | -0,04 | 0,79 | 0,64 | -0,27 | 0,79 | 1,16 | 7,37 | 8,66 | 8,20 | 8,15 | 12,92 | 13,55 | 13,29 | 14,08 |
| hsa-miR-130a | 0,65 | -1,53 | -0,43 | -1,31 | -1,40 | 0,19 | -0,73 | -1,94 | 8,83 | 9,48 | 7,95 | 7,52 | 13,91 | 12,51 | 12,71 | 11,98 |
| hsa-miR-140-3p | 1,54 | -0,08 | 0,06 | 1,52 | 0,33 | 0,37 | 0,04 | 0,73 | 3,55 | 5,09 | 5,01 | 5,07 | 12,39 | 12,72 | 13,08 | 13,12 |
| hsa-miR-141 | 2,16 | -0,28 | 0,00 | 1,88 | 1,17 | 0,37 | 0,17 | 1,70 | 11,15 | 13,31 | 13,03 | 13,03 | 8,05 | 9,22 | 9,58 | 9,75 |
| hsa-miR-148a | 2,06 | -0,50 | 0,04 | 1,60 | 1,31 | -0,28 | 0,91 | 1,93 | 7,35 | 9,41 | 8,91 | 8,95 | 7,04 | 8,34 | 8,06 | 8,97 |
| hsa-miR-151-5p | 1,83 | -0,37 | 0,00 | 1,46 | 1,13 | -0,02 | -0,09 | 1,02 | 6,71 | 8,54 | 8,17 | 8,17 | 9,20 | 10,33 | 10,31 | 10,22 |
| hsa-miR-15a | 2,36 | -0,39 | -0,10 | 1,87 | 1,41 | -0,19 | 0,35 | 1,57 | 7,74 | 10,10 | 9,71 | 9,61 | 6,11 | 7,52 | 7,33 | 7,68 |
| hsa-miR-16 | 2,07 | -0,36 | -0,11 | 1,59 | 2,01 | -1,17 | 1,38 | 2,22 | 9,47 | 11,54 | 11,18 | 11,06 | 3,13 | 5,14 | 3,97 | 5,35 |
| hsa-miR-17 | 0,31 | -1,14 | -0,39 | -1,22 | -0,17 | -0,87 | -0,20 | -1,24 | 9,03 | 9,34 | 8,19 | 7,81 | 12,85 | 12,68 | 11,81 | 11,61 |
| hsa-miR-181a | 1,41 | -0,84 | -0,37 | 0,20 | 0,00 | 0,00 | 0,00 | 0,00 | 7,05 | 8,47 | 7,62 | 7,25 | 0,01 | 0,01 | 0,01 | 0,01 |
| hsa-miR-191 | 2,32 | 0,29 | -0,30 | 2,31 | 0,90 | 0,84 | 0,42 | 2,15 | 0,64 | 2,97 | 3,25 | 2,95 | 14,67 | 15,57 | 16,40 | 16,82 |
| hsa-miR-193b | -0,67 | -1,19 | -0,07 | -1,93 | -1,17 | -0,90 | 0,48 | -1,60 | 8,62 | 7,95 | 6,76 | 6,69 | 13,26 | 12,09 | 11,19 | 11,67 |
| hsa-miR-200a | 1,94 | 0,20 | 0,06 | 2,20 | 1,00 | 0,67 | 0,54 | 2,20 | 8,64 | 10,58 | 10,79 | 10,84 | 9,02 | 10,01 | 10,68 | 11,22 |
| hsa-miR-200b | 2,01 | -0,07 | 0,17 | 2,11 | 0,76 | 0,56 | 1,03 | 2,36 | 9,93 | 11,94 | 11,87 | 12,04 | 8,60 | 9,37 | 9,93 | 10,96 |
| hsa-miR-200c | 1,82 | -0,47 | 0,09 | 1,44 | 0,78 | 0,46 | 1,05 | 2,29 | 10,20 | 12,01 | 11,54 | 11,64 | 8,35 | 9,13 | 9,59 | 10,64 |
| hsa-miR-203 | 0,97 | -0,67 | 0,13 | 0,44 | 0,11 | -0,21 | 1,18 | 1,08 | 10,64 | 11,61 | 10,95 | 11,08 | 13,50 | 13,60 | 13,39 | 14,57 |
| hsa-miR-205 | 1,00 | -0,72 | -0,39 | -0,11 | 0,62 | -1,12 | 0,44 | -0,06 | 13,18 | 14,18 | 13,46 | 13,07 | 14,61 | 15,23 | 14,11 | 14,55 |
| hsa-miR-21 | 1,56 | -0,69 | 0,06 | 0,93 | -0,74 | -1,16 | 0,74 | -1,16 | 14,27 | 15,83 | 15,15 | 15,20 | 16,40 | 15,66 | 14,50 | 15,24 |
| hsa-miR-21* | 1,21 | -0,74 | -0,29 | 0,18 | 0,44 | -0,62 | -0,07 | -0,25 | 7,28 | 8,49 | 7,75 | 7,46 | 8,34 | 8,78 | 8,16 | 8,09 |
| hsa-miR-210 | 0,18 | -0,63 | -0,11 | -0,56 | -0,96 | -0,74 | -0,10 | -1,80 | 9,08 | 9,25 | 8,63 | 8,52 | 12,29 | 11,33 | 10,60 | 10,49 |
| hsa-miR-22 | 1,09 | -0,66 | 0,05 | 0,48 | 0,80 | -1,14 | 0,73 | 0,40 | 10,67 | 11,75 | 11,10 | 11,15 | 8,89 | 9,69 | 8,55 | 9,29 |
| hsa-miR-224 | 2,55 | -0,87 | 0,00 | 1,68 | 1,52 | -0,72 | 0,83 | 1,63 | 6,84 | 9,40 | 8,52 | 8,52 | 9,59 | 11,12 | 10,39 | 11,22 |
| hsa-miR-23a | 0,78 | -0,94 | 0,02 | -0,14 | 0,06 | -0,80 | 0,43 | -0,31 | 11,72 | 12,50 | 11,56 | 11,58 | 14,42 | 14,47 | 13,67 | 14,10 |
| hsa-miR-23b | 1,96 | -0,27 | 0,04 | 1,72 | 1,30 | -0,17 | 0,16 | 1,29 | 8,92 | 10,88 | 10,61 | 10,64 | 14,06 | 15,35 | 15,18 | 15,35 |
| hsa-miR-25 | 1,35 | -0,65 | -0,05 | 0,66 | 0,66 | -0,03 | -0,39 | 0,24 | 7,15 | 8,50 | 7,85 | 7,80 | 9,11 | 9,77 | 9,74 | 9,35 |
| hsa-miR-26a | 2,20 | -0,11 | 0,06 | 2,15 | 1,31 | -0,98 | 2,90 | 3,22 | 8,50 | 10,70 | 10,59 | 10,65 | 0,30 | 1,61 | 0,62 | 3,52 |
| hsa-miR-26b | 1,94 | -0,37 | 0,08 | 1,65 | 0,09 | 0,05 | 0,78 | 0,92 | 7,97 | 9,91 | 9,54 | 9,62 | 6,84 | 6,93 | 6,97 | 7,75 |
| hsa-miR-27b | 1,77 | -0,47 | 0,05 | 1,36 | 0,64 | -0,69 | -0,06 | -0,11 | 9,26 | 11,03 | 10,57 | 10,62 | 6,92 | 7,57 | 6,88 | 6,81 |
| hsa-miR-29a | 0,17 | -0,71 | 0,16 | -0,38 | -1,34 | -0,11 | 0,02 | -1,43 | 11,23 | 11,40 | 10,69 | 10,85 | 16,24 | 14,90 | 14,79 | 14,81 |
| hsa-miR-29c | 1,61 | -0,28 | 0,24 | 1,57 | 0,82 | 0,02 | 0,03 | 0,87 | 9,04 | 10,66 | 10,38 | 10,62 | 13,66 | 14,48 | 14,50 | 14,53 |
| hsa-miR-30b | 2,36 | 0,05 | 0,15 | 2,55 | 0,83 | -0,27 | 0,68 | 1,24 | 7,62 | 9,98 | 10,03 | 10,17 | 8,50 | 9,33 | 9,06 | 9,74 |
| hsa-miR-30c | 1,97 | -0,34 | -0,12 | 1,51 | 0,32 | -0,33 | -0,39 | -0,40 | 7,80 | 9,77 | 9,43 | 9,31 | 0,42 | 0,73 | 0,41 | 0,01 |
| hsa-miR-30d | 1,88 | -0,06 | 0,05 | 1,86 | 0,64 | -0,09 | 1,20 | 1,75 | 7,06 | 8,94 | 8,88 | 8,92 | 9,12 | 9,76 | 9,67 | 10,87 |
| hsa-miR-30e | 2,76 | -0,25 | -0,13 | 2,38 | 1,27 | -0,02 | 0,46 | 1,71 | 6,73 | 9,50 | 9,25 | 9,12 | 7,04 | 8,31 | 8,30 | 8,76 |
| hsa-miR-31 | 0,77 | -1,16 | -0,15 | -0,54 | -0,37 | -0,91 | 0,34 | -0,95 | 10,13 | 10,90 | 9,74 | 9,59 | 13,69 | 13,31 | 12,40 | 12,74 |
| hsa-miR-31* | 0,43 | -1,10 | -0,10 | -0,77 | -0,65 | -1,31 | 0,24 | -1,73 | 9,00 | 9,42 | 8,33 | 8,22 | 13,56 | 12,91 | 11,60 | 11,83 |
| hsa-miR-34a | 2,31 | -0,54 | 0,13 | 1,91 | 1,46 | -1,04 | 0,45 | 0,88 | 8,58 | 10,89 | 10,35 | 10,49 | 10,31 | 11,77 | 10,73 | 11,18 |
| hsa-miR-34b | 2,88 | 4,48 | 0,81 | 8,17 | 1,25 | 4,17 | 1,32 | 6,74 | 0,77 | 3,65 | 8,13 | 8,94 | 7,01 | 8,26 | 12,43 | 13,75 |
| hsa-miR-34b* | 2,02 | 3,36 | 1,10 | 6,48 | 1,22 | 3,50 | 0,95 | 5,67 | 5,96 | 7,98 | 11,35 | 12,44 | 4,99 | 6,20 | 9,70 | 10,65 |
| hsa-miR-34c-5p | 2,35 | 4,27 | 0,82 | 7,44 | 1,33 | 4,02 | 1,06 | 6,41 | 3,94 | 6,29 | 10,56 | 11,39 | 7,88 | 9,21 | 13,23 | 14,29 |
| hsa-miR-365 | -0,02 | -1,21 | 0,05 | -1,18 | -0,39 | 0,00 | 0,00 | -0,39 | 8,44 | 8,41 | 7,21 | 7,25 | 0,41 | 0,01 | 0,01 | 0,01 |
| hsa-miR-374a | 1,41 | -0,54 | -0,08 | 0,79 | -0,33 | 1,74 | 0,74 | 2,16 | 7,04 | 8,45 | 7,91 | 7,83 | 0,62 | 0,30 | 2,04 | 2,78 |
| hsa-miR-378 | 3,23 | -0,81 | -0,26 | 2,16 | 2,15 | -0,26 | -0,39 | 1,50 | 4,38 | 7,61 | 6,80 | 6,54 | 10,37 | 12,53 | 12,26 | 11,87 |
| hsa-miR-425 | 1,59 | 0,19 | -0,21 | 1,57 | 0,42 | 0,74 | -0,04 | 1,12 | 6,61 | 8,21 | 8,39 | 8,18 | 10,58 | 11,01 | 11,75 | 11,70 |
| hsa-miR-429 | 1,71 | 0,14 | 0,10 | 1,95 | 0,42 | 0,14 | 0,74 | 1,30 | 7,97 | 9,68 | 9,82 | 9,93 | 8,23 | 8,65 | 8,79 | 9,53 |
| hsa-miR-449a | 6,53 | 5,70 | -0,15 | 12,08 | 5,21 | 5,21 | 0,21 | 10,63 | 1,47 | 8,00 | 13,70 | 13,54 | 4,48 | 9,69 | 14,90 | 15,11 |
| hsa-miR-449b | 2,61 | 6,25 | -0,22 | 8,64 | 3,32 | 5,40 | -0,12 | 8,60 | 0,47 | 3,08 | 9,33 | 9,11 | 3,64 | 6,96 | 12,36 | 12,24 |
| hsa-miR-574-3p | 1,69 | -0,46 | -0,18 | 1,04 | 0,48 | 0,12 | 0,32 | 0,92 | 4,73 | 6,42 | 5,96 | 5,77 | 13,64 | 14,12 | 14,24 | 14,56 |
| hsa-miR-92b | 0,48 | 4,21 | -0,29 | 4,39 | 1,21 | 4,12 | 0,60 | 5,93 | 0,10 | 0,57 | 4,78 | 4,49 | 7,49 | 8,70 | 12,82 | 13,42 |
| hsa-miR-939 | 1,40 | -0,63 | -0,07 | 0,70 | 0,13 | -0,13 | 0,00 | 0,00 | 6,64 | 8,04 | 7,41 | 7,34 | 0,01 | 0,15 | 0,01 | 0,01 |
| hsa-miR-96 | 1,19 | -0,50 | -0,04 | 0,64 | -0,08 | -0,71 | 0,81 | 0,02 | 8,52 | 9,71 | 9,21 | 9,16 | 5,41 | 5,33 | 4,62 | 5,43 |
| hsa-miR-99a | 2,33 | 0,77 | 0,19 | 3,28 | 1,43 | 0,77 | 0,69 | 2,88 | 5,02 | 7,35 | 8,12 | 8,31 | 9,77 | 11,19 | 11,96 | 12,65 |

. On peut noter que mir-449a, mir-449b, mir-449b*, mir-34a, mir-34b (3p), mir-34b* (5p), mir-34c (5p) sont retrouvés significativement modulés au cours de la différenciation et donc impliqués dans l'initiation et le maintien de l'état de différenciation de l'épithélium respiratoire.

Ces cinq miARN : mir-449a, mir-449b, mir-34a, mir-34b, mir-34c sont retrouvés fortement induits au cours de la différenciation, plus particulièrement au début de la ciliogénèse. Une fois induite, leur expression demeure élevée et stable tout au long de la différenciation y compris dans l'épithélium pleinement différencié. Le répertoire des miARN de l'épithélium complètement différencié se caractérise par une abondance remarquable de ces 5 miARN qui représentent près de 20% du nombre total de tous les miARN exprimés dans ce tissu. Concernant mir-34a, son induction est néanmoins moins forte que les quatre autres miARN et est retrouvée de manière plus précoce au stade ALI-D7.

De manière intéressante, les brins 5p de chacun des miARN mir-449a, mir-449b, mir-34a, mir-34b et mir-34c partagent une homologie de séquence au niveau de leur séquence de reconnaissance suggérant qu'ils ciblent en commun certains transcrits.

C'est pourquoi les Inventeurs ont ensuite concentré leurs efforts sur ces 5 miARN. Les niveaux de régulations de ces miARN ainsi que de mir-31 (qui est retrouvé réprimé au cours de la différenciation de l'épithélium respiratoire) ont été validés par une troisième approche par PCR quantitative (voir la **Figure 5**).

Ces 5 miARN surexprimés au cours de la différenciation de l'épithélium respiratoire appartiennent à deux familles de miARN distinctes : mir-449 et mir-34. Par ailleurs, la localisation génomique de mir-449a et de mir-449b est identique : tous deux sont localisés au niveau du deuxième intron du gène cdc20b, situé sur le chromosome 5 humain (5q11.2, chr5:54456388-54504760). La famille mir-34 est composée de mir-34a qui est localisé dans une région intergénique du chromosome 1 alors que les miARN mir-34b et mir-34c appartiennent au même cluster et sont localisés tous deux dans une région intergénique du chromosome 11. La famille mir-34 a été fonctionnellement reliée à la voie de signalisation p53.

### II-2.B. Cellules épidermiques ciliées d'embryons de Xenope

Les réusltats obtenus pour ce modèle sont présentés dans le Tableau IIIB ci-après et à la **Figure 6** qui présente également des résultats pour les cellules HAEC.

Ces essais montrent que, là encore, la famille de microARN miR-449, et plus particulièrement le miR-449a, constituent de loin les microARN les plus fortement induits au cours ciliogenèse.

**Tableau IIIB - miARN significativement exprimés dans les cellules ciliées de l'épiderme d'embryon de xénope, comparaison avec des cellules non ciliées**

| xenopus miRNA | LogRatio d'expression des miR entre cilié-non cilié | % de miR cellules non ciliées | % de miR cellules ciliées | sd non-cilié | sd cilié |
|---|---|---|---|---|---|
| miR-449a | 3,166507029 | 4,241910975 | 38,99145137 | 0,66831542 | 0,5580561 |
| miR-17-5p | 1,843961214 | 1,739241798 | 6,439037664 | 0,03088444 | 0,44692614 |
| miR-427 | -3,577711451 | 84,3912305 | 7,287645762 | 3,81200367 | 0,43536764 |
| miR-18a | 1,963297831 | 1,445998485 | 5,770842336 | 0,25353698 | 0,40662557 |
| miR-203 | 2,899525765 | 0,802824596 | 6,122022692 | 0,14427702 | 0,16748381 |
| miR-429 | 3,489311848 | 0,5611861 | 6,308452174 | 0,18811487 | 0,19190601 |
| miR-34b | 8,639233391 | 0,023309799 | 6,678155706 | 0,02362849 | 0,24212636 |
| miR-130c | 3,303007088 | 0,319262952 | 3,090857721 | 0,13938414 | 0,20761583 |
| miR-20b | 0,920427989 | 1,297427834 | 1,839130412 | 1,29177087 | 0,55721948 |
| miR-200a | 2,92597595 | 0,309242272 | 2,418052837 | 0,03713634 | 0,23727106 |
| miR-130b | 5,041603605 | 0,061086987 | 2,057820515 | 0,01136075 | 0,16380899 |
| miR-106 | 2,067406718 | 0,395322902 | 1,683867839 | 0,09703348 | 0,14099092 |
| miR-93b | 0,31478571 | 0,797852579 | 0,902730776 | 0,63514139 | 0,4494722 |
| miR-93a | 0,317106219 | 0,795629375 | 0,898232543 | 0,63828547 | 0,44663817 |
| miR-26 | 2,754387045 | 0,166267205 | 1,158759618 | 0,02096265 | 0,18818027 |
| miR-20a | 2,428912948 | 0,212684269 | 1,096366145 | 0,11342212 | 0,12723715 |
| miR-218 | 12,28452357 | 0,002273204 | 1,086641349 | 0,00307337 | 0,11041905 |
| miR-34a | 1,883909316 | 0,183851237 | 0,690835543 | 0,04597169 | 0,08895895 |

### II-2.C. Discussions

Alors que les miR-449 représentent moins de 0,01% de toutes les séquences de microARN dans les cellules HAECs au cours de leur multiplication, ces miR-449 représentent plus de 8% des microARN exprimés dans les cellules HAECs différenciées (voir la **Figure 6** graphes a et b). De même, le miR-449a dans les cellules de Xenope augmente significativement au cours de la différenciation, représentant jusqu'à 39% de toutes les séquences de microARN dans ciliées d'explants épidermique (voir la **Figure 6****,** graphes c-d).

Comme indiqué plus haut, les miR-449 et les miR-34 appartiennent à une même superfamille de microARN. De façon intéressante, on note que l'expression des membres de la famille miR-34 a été également été induite au cours de la ciliogenèse dans les deux modèles (voir la **Figure 6****,** graphes a et b), même si elle l'est dans une moindre mesure que celle des miR-449.

La famille des miR-449 semble être conservée chez les vertébrés : l'étude des loci synténiques de Cdc20b (où est localisé le cluster miR-449 chez l'homme et chez la grenouille) a révélé l'existence de miR-449 dans tous les génomes de vertébrés totalement ou partiellement séquencés.

Les cellules HAECs différenciées et les cellules ciliées de l'épiderme de *Xenopus laevis* sont constitués d'un mélange de différents types cellulaires, incluant des cellules basales, des cellules muco-sécrétrices et des cellules multiciliées.

L'hybridation *in situ* sur les cultures primaires HAEC (voir les clichés a et c de la **Figure 7**) et sur le tissu bronchique de l'homme (voir les clichés e et g de la **Figure 7**) a révélé que les miR-449 ont été exprimées dans les cellules cylindriques multiciliées mais pas dans les cellules basales (voir le cliché a de la **Figure 7**) ou dans les cellules sécrétrices muc5AC-positives (voir le cliché e de la **Figure 7**).

Ces résultats ont été confirmés par les essais de séquençage haut débit sur des fractions enrichies en cellules cylindriques (principalement composées de cellules multiciliées et de quelques cellules muco-sécrétrices) et en cellules basales, dérivées de l'épithélium des voies respiratoires de l'homme.

Le graphe d de la **Figure 7** montre un enrichissement des miR-449 dans la fraction de cellules ciliées, tandis que l'expression des miR-34 observée est plus uniforme et a été observée dans les deux types de cellules.

Enfin, l'hybridation *in situ* sur les cellules d'embryons de *Xenope* a révélé que l'expression des miR-449 a été restreinte aux cellules ciliées, cellules qui sont positives au marquage à la tubuline acétylée (voir les clichés h et l de la **Figure 7**).

L'ensemble de ces résultats montrent que les miR-449 sont les microARN les plus abondants des cellules multiciliées de vertébrés.

### III- Détermination des cibles spécifiques des miARN sélectionnés

### III-1. Identification du type cellulaire spécifique de chaque miARN

Comme l'épithélium respiratoire natif, le modèle de différenciation *in vitro* utilisé dans ces essais est constitué de cellules basales, de cellules ciliées et de cellules sécrétrices de mucus. En vue d'identifier les répertoires des miARN spécifiques à chacun des types cellulaires, les Inventeurs ont utilisé le tri cellulaire par cytométrie de flux à l'aide de marqueurs spécifiques (Hajj, R. *et al.* 2007). A l'aide de cette technique, des cellules respiratoires adultes progénitrices (correspondant *a priori* à des cellules basales) ont été isolées, sélectionnées et triées (de telles cellules apparaissent doublement positives pour la tétraspanine CD151 et pour le facteur tissulaire) des cellules pyramidales (i.e. les cellules ciliées et les cellules sécrétrices de mucus) qui apparaissent négatives pour ces mêmes marqueurs. Ces purifications ont été réalisées à partir de polypes ou de cornets nasaux issus de 3 donneurs distincts.

Après avoir extrait les ARN totaux, les profils d'expression des miARN ont été établis à l'aide de puces commerciales Agilent® ou par séquençage à haut-débit (HTS). Les deux approches expérimentales corroborent pour plusieurs miARN sélectionnés dont mir-449a, mir-449b, mir-34a, mir-34b, mir-34b* (mir-34b-5p), mir-34c et mir-34c* (mir-34c-3p) leur expression spécifique dans les cellules épithéliales ciliées. De plus, cette expérience de tri cellulaire permet de montrer que les miARN réprimés dans les cellules en culture (e.g. mir-31, mir-31*, mir-205, mir-130a, mir-193b) sont plus spécifiques des cellules basales (voir la **Figure 8** et le **Tableau VII).** Pour la première fois, les Inventeurs attribuent ainsi de manière spécifique un répertoire d'expression des miARN à des types cellulaires distincts de l'épithélium respiratoire au cours de la différenciation.

**Tableau VII. miARN spécifiques des cellules basales ou des cellules pyramidales (ciliées+sécrétrices) identifiés par HTS et puces Agilent®.**

| Nom | Modulation de l'expression des miARN dans les cellules pyramidales (323(-)) par rapport aux cellules basales 323(+) | Intensité du niveau d'expression en log2 dans descellules basales (323(+)) | Intensité du niveau d'expression en log2 dans des cellules pyramydales (323(-)) |
|---|---|---|---|
| hsa-let-7a | -1,29 | 11,93 | 10,65 |
| hsa-let-7b | -1,96 | 12,20 | 10,24 |
| hsa-let-7c | -0,48 | 9,43 | 8,95 |
| hsa-let-7e | -1,42 | 8,24 | 6,82 |
| hsa-let-7f | -0,94 | 10,82 | 9,88 |
| hsa-let-7q | -0,15 | 8,37 | 8,22 |
| hsa-miR-100 | 1,62 | 3,28 | 4,90 |
| hsa-miR-106b | -2,05 | 7,22 | 5,16 |
| hsa-miR-125a-5p | -0,05 | 6,63 | 6,58 |
| hsa-miR-130a | -6,33 | 9,76 | 3,43 |
| hsa-miR-140-3p | 0,91 | 2,75 | 3,66 |
| hsa-miR-141 | -2,88 | 11,12 | 8,24 |
| hsa-miR-148a | -1,76 | 7,14 | 5,39 |
| hsa-miR-151-5p | -0,73 | 7,24 | 6,51 |
| hsa-miR-15a | -2,10 | 7,86 | 5,75 |
| hsa-miR-16 | -0,48 | 9,10 | 8,62 |
| hsa-miR-17 | -2,60 | 6,64 | 4,04 |
| hsa-miR-181a | -2,29 | 6,88 | 4,59 |
| hsa-miR-191 | 0,00 | 0,14 | 0,14 |
| hsa-miR-193b | -3,57 | 3,90 | 0,33 |
| hsa-miR-200a | -0,95 | 8,80 | 7,85 |
| hsa-miR-200b | -0,15 | 9,99 | 9,84 |
| hsa-miR-200c | -0,67 | 10,24 | 9,57 |
| hsa-miR-203 | -3,50 | 3,64 | 0,14 |
| hsa-miR-205 | -7,00 | 12,44 | 5,44 |
| hsa-miR-21 | -1,26 | 11,34 | 10,08 |
| hsa-miR-21* | -2,20 | 5,03 | 2,83 |
| hsa-miR-210 | -2,03 | 6,60 | 4,57 |
| hsa-miR-22 | -1,73 | 8,81 | 7,07 |
| hsa-miR-224 | -2,84 | 3,65 | 0,82 |
| hsa-miR-23a | -2,45 | 10,25 | 7,80 |
| hsa-miR-23b | -2,23 | 9,41 | 7,18 |
| hsa-miR-25 | -0,70 | 5,40 | 4,70 |
| hsa-miR-26a | -2,24 | 9,92 | 7,68 |
| hsa-miR-26b | -1,47 | 8,75 | 7,28 |
| hsa-miR-27b | -3,56 | 9,77 | 6,21 |
| hsa-miR-29a | -2,95 | 11,09 | 8,14 |
| hsa-miR-29c | -3,06 | 11,06 | 8,00 |
| hsa-miR-30b | -0,80 | 8,76 | 7,96 |
| hsa-miR-30c | -1,01 | 6,92 | 5,91 |
| hsa-miR-30d | -1,11 | 8,16 | 7,05 |
| hsa-miR-30e | -1,60 | 6,94 | 5,34 |
| hsa-miR-31 | -4,31 | 6,36 | 2,05 |
| hsa-miR-31* | -3,70 | 3,84 | 0,14 |
| hsa-miR-34a | 0,64 | 6,72 | 7,36 |
| hsa-miR-34b | 8,24 | 0,88 | 9,12 |
| hsa-miR-34b* | 6,05 | 5,48 | 11,52 |
| hsa-miR-34c-5p | 5,74 | 4,75 | 10,49 |
| hsa-miR-365 | -0,99 | 4,83 | 3,84 |
| hsa-miR-374a | -1,04 | 4,26 | 3,22 |
| hsa-miR-378 | -2,43 | 4,11 | 1,68 |
| hsa-miR-425 | 0,70 | 4,52 | 5,22 |
| hsa-miR-429 | -0,31 | 6,92 | 6,61 |
| hsa-miR-449a | 6,07 | 2,81 | 8,88 |
| hsa-miR-449b | 4,05 | 0,14 | 4,18 |
| hsa-miR-574-3p | 0,91 | 3,53 | 4,44 |
| hsa-miR-768-3p | -1,20 | 10,15 | 8,95 |
| hsa-miR-92b | 1,67 | 0,40 | 2,07 |
| hsa-miR-939 | 1,28 | 4,81 | 6,08 |
| hsa-miR-96 | -1,51 | 6,81 | 5,30 |
| hsa-miR-99a | 0,99 | 5,42 | 6,40 |

### III-2. Identification des ARNm cibles des miRNA sélectionnés

Un enjeu majeur dans la biologie des miARN est de pouvoir identifier et caractériser expérimentalement les ARNm cibles qu'ils régulent. Dans cette perspective, ont été combinées des approches *in silico* (logiciels bioinformatiques prédictifs de cibles), des approches expérimentales (puces transcriptomes, expression ectopique de miARN ainsi que de vecteurs rapporteur contenant la partie 3'-UTR du gène d'intérêt fusionné à la luciférase). Des expériences complémentaires ont utilisé des approches d'immunocytochimie, de vidéo-microscopie et de biochimie.

Plusieurs algorithmes de prédiction de cibles ont été proposés. Ils sont basés généralement sur : i) la complémentarité entre le miARN et le 3'UTR de l'ARNm cible au niveau de la région 5' du miARN (séquence de reconnaissance) ; ii) la conservation phylogénétique de cette séquence dans le 3'UTR de l'ARNm cible.

Afin de déterminer les gènes cibles potentiellement régulés par ces miARN d'intérêt, les Inventeurs ont procédé à l'établissement d'un profilage des ARNm par puce d'expression (Affymetrix, human HuGene 1.0 ST microarrays). Les échantillons analysés sont les mêmes que ceux qui ont servi pour la mesure des miARN au cours de la différenciation. Une approche complémentaire a consisté à évaluer le transcriptome de cellules épithéliales respiratoires après manipulation du niveau d'expression des miARN sélectionnés par transfection. Une sélection de gènes-cibles en relation avec la régénération et la différenciation de l'épithélium a été réalisée à l'aide de supports bioinformatiques (Mediante, Ingenuity Pathway™). Les Inventeurs ont ainsi pu établir un lien fonctionnel entre les miARN précédemment identifiés et un certain nombre de transcrits impliqués dans la régénération et la différenciation de l'épithélium respiratoire humain. Les résultats obtenus montrent que 500-1000 transcrits sont associés à la différentiation (voir la **Figure 9**).

Seule l'expression de quelques gènes varie entre ALI-FC and ALI-WD, indiquant une stabilité de l'expression des ARN une fois la ciliogénèse enclenchée.

### III-3. Identification in silico des gènes cibles putatifs communs des brins 5p des miARN mir-449a, mir-449b, mir-34a, mir-34b et mir-34c

Les gènes cibles prédits ont été récupérés pour chacun des brins 5p des 5 miARN sélectionnés mir-449a, mir-449b et mir-34a, mir-34b-5p (mir-34b*) et mir-34c-5p qui partagent une homologie de séquence au niveau de leur séquence de reconnaissance, à l'aide d'outils bioinformatiques prédictifs (*i.e*. TargetScan, mirbase target, picTar and Microcible 2to8) accessibles à travers l'interface réseau Mediante (www.microarray.fr). Les Inventeurs ont tout d'abord sélectionné les gènes cibles prédits communs aux 5 miARN d'intérêts, qui ont été validés à l'aide de la méthodologie détaillée ci-dessus. Sur près de 3500 cibles prédites pour chacun des miARN pris séparément, 1229 cibles sont communes aux 5 miARN sélectionnés. Les 1229 cibles prédites ont ensuite été comparées avec celles significativement réprimées (environ 1000, P<0.05) au cours de la différenciation plus particulièrement entre la condition pleinement différenciée et la première étape de prolifération non-différenciée (ALI-WD versus ALI-D0 (n=3 donneurs)). Ainsi, les résultats montrent 62 gènes communs aux 5 miARN sélectionnés susceptibles de jouer un rôle clé dans la régénération de l'épithélium respiratoire (voir le **Tableau VIII).** Parmi ces gènes, est retrouvée notamment la cavéoline-1. Les résultats montrent que la cavéoline-1 est fortement inhibée tout au long de la différenciation (voir la **Figure 8**).

**Tableau VIII. Listes des gènes cibles putatifs des microARN sélectionnés retrouvés inhibés au cours de la différenciation de l'épithélium respiratoire.**

| Gène | Modulation de l'expression des gène entre les stades de différenciation WD et D0-ALI | Amean (sur 3 donneurs) | Adjust.P.Value | Numéro d'accession du gène |
|---|---|---|---|---|
| ABLIM3 | -1.4481743 | 7.524965 | 3.82E-04 | NM_014945 |
| ADAM 19 | -1.1163435 | 6.14820233 | 5.45E-07 | NM_033274 |
| ADAMTSL4 | -1.1186815 | 7.2720022 | 4.62E-04 | NM_019032 |
| ADCY7 | -1.382631 | 7.6534838 | 2.38E-07 | NM_001114 |
| AMOTL1 | -1.7460034 | 8.6474232 | 2.94E-06 | NM_130847 |
| ARPP-19 | -1.0941527 | 8.8116738 | 1.22E-05 | NM_006628 |
| BMP1 | -0,9680967 | 8,6574928 | 1.38E-05 | NM_001199 |
| BTBD11 | -1.8490199 | 7.6618604 | 1.14E-05 | NM_001018072 |
| C12orf29 | -1.2553972 | 8.48381407 | 1.68E-04 | NM_001009894 |
| CAV1 | -2.368023 | 9.93025267 | 4.53E-06 | NM_001753 |
| CDA | -2.481947 | 6.80947653 | 2.94E-07 | NM_001785 |
| CDC25A | -1.3080745 | 6.28751913 | 4.79E-06 | NM_001789 |
| CDCA5 | -1.0540303 | 6.48987507 | 1.09E-04 | NM_080668 |
| COL7A1 | -1.6867157 | 8.59302327 | 1.04E-06 | NM_000094 |
| CPM | -1.4438189 | 8.6954668 | 9.13E-03 | NM_001005502 |
| CRABP2 | -1.4293157 | 9.39830873 | 6.87E-05 | NM_001878 |
| CTNNBIP1 | -1.0108869 | 9.35766273 | 2.86E-08 | NM_020248 |
| DKK1 | -2.5935028 | 7.03329227 | 4.98E-04 | NM_012242 |
| DLL1 | 0,2733639 | 7,4865392 | | NM_005618 |
| DSC3 | -1.3986191 | 9.60600093 | 5.07E-03 | NM_024423 |
| EFNA3 | -1.0526506 | 7.54088253 | 1.52E-03 | NM_004952 |
| EFNB1 | -1.6142037 | 8.63543853 | 1.49E-05 | NM_004429 |
| EMP1 | -3.521182 | 8.67082233 | 5.65E-04 | NM_001423 |
| FSTL3 | -1.617425 | 8.44148553 | 2.91 E-06 | NM_005860 |
| FUT11 | -1.0545791 | 8.3877732 | 3.81 E-04 | NM_173540 |
| GLTP | -1.197381 | 10.2894117 | 5.63E-04 | NM_016433 |
| GPX3 | -1.483986 | 8.54605007 | 1.59E-04 | NM_002084 |
| IL1RN | -2.7062559 | 9.60439793 | 2.04E-05 | NM_173841 |
| IQGAP3 | -1.1769401 | 6.3339546 | 1.56E-03 | NM_178229 |
| IRAK2 | -1.1324037 | 7.05078867 | 1.95E-04 | NM_001570 |
| ITPR2 | -1.1585075 | 6.7519412 | 9.93E-05 | NM_002223 |
| JAG1 | -0,8238893 | 10,024193 | | NM_00214 |
| KDELR3 | -1.604097 | 6.37016313 | 1.74E-07 | NM_006855 |
| LYPD3 | -1.9136232 | 9.53049673 | 1.19E-03 | NM_014400 |
| MAP4K4 | -1.3458818 | 9.26761513 | 5.77E-07 | NM_145686 |
| MYO5A | -1.5881695 | 7.809912 | 3.04E-10 | NM_000259 |
| NDRG1 | -1.3955203 | 11.7800207 | 4.34E-03 | NM_006096 |
| NEDD4 | -1.8082366 | 7.2788706 | 7.67E-04 | NM_006154 |
| NOTCH1 | -0,5783195 | 8,7229346 | 0,0256397 | NM_017617 |
| PDLIM7 | -1.3596731 | 8.12085653 | 4.91 E-06 | NM_005451 |
| PEA15 | -1.1301513 | 9.77535027 | 1.27E-03 | NM_003768 |
| PGAM4 | -1.1640039 | 10.9063747 | 6.86E-08 | NM_001029891 |
| PNPLA3 | -1.3599861 | 6.92883227 | 4.50E-06 | NM_025225 |
| PPL | -1.1742775 | 10.6121583 | 1.74E-03 | NM_002705 |
| PRELID2 | -1.2843971 | 6.360054 | 2.69E-08 | NM_182960 |
| PRKCA | -1.1283394 | 6.67201333 | 2.48E-04 | NM_002737 |
| RAET1 L | -2.9534389 | 8.77895953 | 1.67E-06 | NM_130900 |
| RIN1 | -1.23138 | 7.72833807 | 2.35E-06 | NM_004292 |
| SC5DL | -1.1485737 | 9.615791 | 3.44E-06 | NM_006918 |
| SEMA4B | -1.1518643 | 9.4275898 | 3.23E-05 | NM_198925 |
| SERPINB2 | -1.398391 | 11.0805848 | 8.01 E-05 | NM_002575 |
| SERPINE1 | -2.945564 | 9.083679 | 1.16E-02 | NM_000602 |
| SLC37A2 | -1.5591718 | 7.97890727 | 2.68E-06 | NM_198277 |
| SLC4A7 | -1.5922747 | 8.1975794 | 8.73E-10 | NM_003615 |
| SPARC | -2.1890128 | 6.9962164 | 3.28E-06 | NM_003118 |
| SYNC1 | -1.1653204 | 6.9710348 | 5.37E-05 | NM_030786 |
| TGFA | -2.1820264 | 8.82130713 | 1.10E-07 | NM_003236 |
| TGFBI | -1.3975298 | 9.96521733 | 1.62E-03 | NM_000358 |
| TGM2 | -2.4139445 | 8.10195833 | 7.22E-08 | NM_004613 |
| TMBIM1 | -1.2519402 | 11.9735373 | 2.56E-04 | NM_022152 |
| TMCC3 | -1.117217 | 7.91308893 | 1.99E-06 | NM_020698 |
| TMEFF1 | -1.3004997 | 6.23670373 | 3.30E-04 | NM_003692 |
| TNS4 | -1.1104828 | 9.96975587 | 3.06E-06 | NM_032865 |
| UHRF2 | -1.1846124 | 8.1264516 | 1.95E-05 | NM_152896 |
| VAT1 | -1.2608379 | 9.68055807 | 6.22E-07 | NM_006373 |
| VSIG1 | -1.3975763 | 4.822476 | 6.93E-10 | NM_182607 |
| WNT4A | 0,9209719 | 8,8476432 | 0,0017319 | NM_030761 |
| WNT7A | -2,0382706 | 8,0121046 | 1,19E-08 | NM_004625 |

A cette liste de gènes cibles d'intérêt, peuvent être ajoutés les gènes Rfx2, Rfx3, FoxJ1 et STATH qui sont les cibles prédites par bioinformatique de miR-31 et/ou miR-130a.

### IV- Validation de la Cavéoline-1 comme cible des miARN sélectionnés

La cavéoline-1 (Cav-1) est une protéine membranaire de 22-kDa essentielle à la formation de petites invaginations de la membrane plasmique appelées caveolae. Le gène Cav-1 est exprimé dans les cellules adhérentes (endothéliales, épithéliales, fibroblastes, cellules musculaires lisses).

Plus particulièrement, il a été montré que les cavéolines sont présentes à la surface membranaire des cellules épithéliales basales et ciliées, indiquant un rôle crucial dans ces types cellulaires (Krasteva, G. et al. (2006) Respir Res 7, 108). Une perte d'expression de cavéoline-1 peut conduire à une prolifération et une différenciation épithéliales défectueuses (Yang, G. et al. (2008) Exp Mol Pathol 84, 131-140).

En outre, les cavéolines ont été incriminées dans diverses susceptibilités aux pathologies respiratoires. Par exemple, les *caveolae* contiennent une variété de récepteurs, et la Cav-1 a été impliquée dans la réduction du nombre de récepteurs du transforming growth factor (TGF)-beta présents à la surface cellulaire. Des travaux antérieurs ont souligné la contribution probable d'un défaut de cavéoline-1 dans plusieurs pathologies respiratoires en conséquence d'une perturbation de la voie du TGF-beta Le Saux, C.J. et al. (2008) J Biol Chem 283, 5760-5768).

Par ailleurs, il a été montré que plusieurs micro-organismes utilisaient sélectivement les *caveolae* pour infecter les cellules (Norkin, L.C. et al. (2001) Exp Cell Res 266, 229-238). Comme ces *caveolae* sont localisés sur la surface basolatérale des cellules ciliées de l'épithélium respiratoire, ils pourraient notamment être impliqués dans l'endocytose d'agents infectieux lors d'une lésion épithéliale (Krasteva, G. *et al.,* 2006). En effet, les adénovirus nécessitent une perte d'intégrité de l'épithélium ou des jonctions serrées pour accéder à la membrane basolatérale des cellules ciliées pour exercer leur pouvoir pathogène (Walters, R.W. et al. (1999) J Biol Chem 274, 10219-10226). Enfin, les cellules basales seraient plus vulnérables aux infections (Pickles, R.J. et al. (1996) Hum Gene Ther 7, 921-931 (1996). En accord avec cela, il a été montré que le nombre des *caveolae* est plus important au niveau des cellules basales que des cellules ciliées (Krasteva, G. *et al.,* 2006).

### IV-1. Principe des essais

L'algorithme MicroCible a été utilisé ; il identifie 7 sites de fixation différents pour miR-34b-5p dans le transcrit de Cav-1, contre 3 sites de fixation différents pour mir-34a/34c-5p, mir-449a/b. Les Inventeurs ont construit un vecteur d'expression d'un gène rapporteur dans lequel la partie 3'-UTR entière de la Cav-1 a été insérée en aval de la séquence codante de la luciférase. Ensuite, les cellules HEK293T ont été co-transfectées avec ce vecteur et chacun des 5 miARN sélectionnés (mir34a/b-5p/c-5p/449a/b), indépendamment en comparaison avec un miARN contrôle négatif.

### IV-2. Matériels et méthodes

### Vecteurs d'expression 3'-UTR et mesure d'activité luciférase.

La séquence complète de la partie non codante (3'-UTR) de la cavéoline (SEQ ID N°178) est amplifiée par PCR puis clonée au niveau des sites Xhol and NotI du vecteur psiCheck2 (Promega).

Les microARN synthétiques d'intérêts (miR-34a, miR-34b*, miR-34c-5p et le miR contrôle négatif (miR-Neg1)) ont été synthétisés par la société Ambion (Applied Biosystems). Une transfection inverse a été réalisée sur les cellules HEK293T (20000 cellules par puits) dans une plaque 96-puits blanche avec 100ng de vecteur plasmidique psiCheck2 et 5 nmoles de miARN synthétique en utilisant comme agent de transfection la Lipofectamine 2000 (Invitrogen). 48 heures après la transfection, les activités des luciférases renilla et firefly ont été évaluées avec le kit Dual Glo Luciferase Assay System (Promega) et mesurées à l'aide d'un luminomètre (Luminoskan Ascent, Thermolab system).

### Identification de voies de signalisation

Le software Ingenuity Pathway Analysis (IPA) (Ingenuity Systems, Mountain View, USA) a été utilisé pour identifier des réseaux d'interaction entre gènes d'intérêts et d'autres groupes fonctionnels. Les gènes ayant un ratio supérieur à 1 ont été sélectionnés. Ainsi, il est possible d'associer des fonctions biologiques et des maladies à nos résultats expérimentaux.

### IV-3. Résultats

Les Inventeurs ont montré que mir-34b-5p inhibait significativement (P<0.01) l'expression du gène de la luciférase lorsque celle-ci est fusionnée à la partie 3' non-codante de la cavéoline-1. Ces résultats additionnés au fait que les inventeurs retrouvent la cavéoline-1 fortement inhibée au cours de la différenciation indiquent que la Cav-1 est une cible spécifique de mir-34b* (mir-34b-5p) impliquée dans le processus de différenciation de l'épithélium respiratoire (**Figure 11**).

Il apparait vraisemblable que les miARN, mir-449a, mir-449b, mir-34a et mir-34b*, ne réprimant pas la cavéoline-1, agissent sur la régulation d'un ou plusieurs autres gènes.

Selon le même principe, l'essai réalisé avec la cavéoline a été reproduit avec d'autres gènes cibles d'intérêt ; la séquence 3'-UTR des gènes suivants a été clonée AREG (SEQ ID N°179), AURKA (SEQ ID N°180), CAPN13 (SEQ ID N°181), CCNB1 (SEQ ID N°182), CCNE2 (SEQ ID N°183), CDC6 (SEQ ID N°184), CDC25A (SEQ ID N°185), CENPK (SEQ ID N°186), CEP55 (SEQ ID N°187), CDC20B (SEQ ID N°188), E2F7 (SEQ ID N°189), FOXM1 (SEQ ID N°190), STATH (SEQ ID N°191) et TOP2A (SEQ ID N°192).

**Tableau IX - liste des gènes cibles validé par au moins un des miARN sélectionné**

| Cibles de miR-449a/b ou miR-34a,b,b*,c-3p,c-5p testées expérimentalement | Description du gène | Numéro d'accession du gène | cibles validées pour au moins un des miARN |
|---|---|---|---|
| AREG (SEQ ID N°179) | Homo sapiens amphiregulin (AREG), mRNA. | NM_001657 | miR-449a/b et miR-34a/c-5p |
| AURKA (SEQ ID N°180) | Homo sapiens aurora kinase A (AURKA), transcript variant 1, mRNA. | NM_198433 | miR-34b |
| CCNB1 (SEQ ID N°182) | Homo sapiens cyclin B1 (CCNB1), mRNA. | NM_031966 | miR-449a/b et miR-34a/c-5p |
| CCNE2 (SEQ ID N°183) | Homo sapiens cyclin E2 (CCNE2), mRNA. | NM_057749 | miR-449a/b et miR-34a/c-5p |
| CDC25A (SEQ ID N°185) | Homo sapiens cell division cycle 25 homolog A (S. pombe) (CDC25A), transcript variant 1, mRNA. | NM_001789 | miR-449a/b et miR-34a/c-5p |
| CEP55 (SEQ ID N°187) | Homo sapiens centrosomal protein 55kDa (CEP55), transcript variant 1, mRNA. | NM_018131 | miR-34b* |

Une cible est validée dès lors que son expression est inhibée par un miARN.

### V- Identification par puces ADN (Affymetrix®) des cibles des miARN d'intérêts : mir-449a, mir-449b, mir-34a, mir-34b*, mir-34c-5p

Afin de déterminer les gènes modulés spécifiquement par l'expression d'un miARN d'intérêt, chaque miARN, mir-449a, mir-449b, mir-34a, mir-34b* et mir-34c-5p, est transfecté dans des cultures primaires de cellules épithéliales respiratoires non-différenciées (HAECs) et étudié par puces transcriptome (Affymetrix®), 48h après la transfection, les gènes différentiellement modulés.

Etant donné que mir-449a, mir-449b, mir-34a, mir-34b*, mir-34c-5p partagent le même « seed » (séquence 2-7), ils sont susceptibles d'interagir avec des cibles communes. De ce fait, 95 gènes communs ont été obtenus comme significativement modulés par ces miARN.

Classiquement il est admis qu'un miARN va directement agir sur ses ARNm cibles en vue de réprimer leur expression. La **Figure 12** montre l'enrichissement de gènes cibles contenant de la région complémentaire de la séquence de reconnaissance de ces miARN d'intérêt en réponse à 48h de transfection de chacun de ces miARN. Nous avons observé sur les 95 gènes modulés que 41 gènes sont significativement réprimés (voir le **Tableau X**).

Sur ces 41 gènes réprimés, 18 gènes sont des cibles prédites (par analyse *in silico*) communes aux 5 miARN sélctionnés (voir le **Tableau XI)** et dont la régulation par lesdits miARN pourraient jouer un rôle clé dans la différenciation de l'épithélium respiratoire et dans la mise en place d'une stratégie thérapeutique de maladies respiratoires (mucoviscidose, asthme, bronchopneumopathies obstructives chronique, dyskinésie ciliaire primaire...).

**Tableau IX : 41 gènes réprimés communs aux miARN mir-34a, mir-34b*, mir-34c-5p, mir-449a et mir-449b après transfection dans des HAECs de chacun des miARN d'intérêt.**

| | **Gene Description** | **Niveau d'intensité d'expression en log2** | **Modulation en présence de mir34a** | **Modulation en présence de mir34b-5p** | **Modulation en présence de mir34c-5p** | **Modulation en présence de mir449a** | **Modulation en présence de mir449b** |
|---|---|---|---|---|---|---|---|
| HIST1H3B | histone cluster 1, H3b | 7,71 | -0,57 | -0,42 | -0,74 | -1,03 | -0,66 |
| CDC2 | cell division cycle 2, G1 to S and G2 to M | 5,59 | -0,57 | -0,29 | -0,64 | -1,00 | -0,67 |
| DTL | denticleless homolog (Drosophila) | 6,47 | -0,65 | -0,38 | -0,86 | -0,97 | -0,81 |
| KLRK1//KLRC4 | killer cell lectin-like receptor subfamily K, member 1 // killer cell lectin-like receptor subfamily C, member 4 | 4,16 | -0,78 | -0,90 | -0,85 | -0,97 | -0,62 |
| KIAA0101 | KIAA0101 | 6,71 | -0,88 | -0,65 | -0,84 | -0,92 | -0,72 |
| NUF2 | NUF2, NDC80 kinetochore complex component, homolog (S. cerevisiae) | 6,20 | -0,91 | -0,55 | -0,68 | -0,92 | -0,75 |
| HIST1H2BM | histone cluster 1, H2bm | 8,72 | -0,44 | -0,59 | -0,69 | -0,92 | -0,66 |
| RAD51AP1 | RAD51 associated protein 1 | 5,42 | -1,03 | -0,60 | -0,92 | -0,87 | -0,65 |
| CENPK | centromere protein K | 6,38 | -0,89 | -0,60 | -0,74 | -0,83 | -0,65 |
| CCNB2 | cyclin B2 | 6,51 | -0,65 | -0,55 | -0,59 | -0,83 | -0,77 |
| KIF11 | kinesin family member 11 | 5,98 | -0,43 | -0,20 | -0,58 | -0,82 | -0,54 |
| NUSAP1 | nucleolar and spindle associated protein 1 | 6,73 | -0,49 | -0,30 | -0,45 | -0,82 | -0,51 |
| TOP2A | topoisomerase (DNA) II alpha 170kDa | 7,06 | -0,70 | -0,21 | -0,63 | -0,80 | -0,52 |
| DEPDC1 | DEP domain containing 1 | 5,22 | -0,80 | -0,39 | -0,54 | -0,80 | -0,71 |
| ASPM | asp (abnormal spindle) homolog, microcephaly associated (Drosophila) | 5,73 | -0,59 | -0,32 | -0,62 | -0,77 | -0,67 |
| ARHGAP11B | Rho GTPase activating protein 11B | 4,69 | -0,82 | -0,75 | -0,71 | -0,76 | -0,84 |
| HELLS | helicase, lymphoid-specific | 5,95 | -0,78 | -0,55 | -0,69 | -0,74 | -0,79 |
| CENPI | centromere protein I | 6,39 | -0,54 | -0,67 | -0,69 | -0,73 | -0,91 |
| CENPF | centromere protein F, 350/400ka (mitosin) | 6,61 | -0,64 | -0,34 | -0,57 | -0,72 | -0,51 |
| BUB1 | BUB1 budding uninhibited by benzimidazoles 1 homolog (yeast) | 6,49 | -0,54 | -0,25 | -0,54 | -0,72 | -0,60 |
| RRM2 | ribonucleotide reductase M2 polypeptide | 7,72 | -0,57 | -0,29 | -0,76 | -0,71 | -0,60 |
| TTK | TTK protein kinase | 5,11 | -0,50 | -0,39 | -0,81 | -0,69 | -0,67 |
| ARHGDIB | Rho GDP dissociation inhibitor (GDI) beta | 8,43 | -0,54 | -0,48 | -0,70 | -0,69 | -0,59 |
| CEP55 | centrosomal protein 55kDa | 6,68 | -0,52 | -0,37 | -0,62 | -0,68 | -0,64 |
| TPX2 | TPX2, microtubule-associated, homolog (Xenopus laevis) | 6,89 | -0,69 | -0,42 | -0,61 | -0,68 | -0,60 |
| FOXM1 | forkhead box M1 | 6,22 | -0,64 | -0,37 | -0,38 | -0,66 | -0,56 |
| AURKA | aurora kinase A | 7,42 | -0,51 | -0,38 | -0,51 | -0,65 | -0,66 |
| CASC5 | cancer susceptibility candidate 5 | 6,03 | -0,47 | -0,14 | -0,45 | -0,63 | -0,59 |
| SPC25 | SPC25, NDC80 kinetochore complex component, homolog (S. cerevisiae) | 4,93 | -0,46 | -0,32 | -0,49 | -0,61 | -0,52 |
| PRC1 | protein regulator of cytokinesis 1 | 7,81 | -0,57 | -0,48 | -0,57 | -0,60 | -0,65 |
| ARHGAP11A | Rho GTPase activating protein 11A | 6,53 | -0,40 | -0,24 | -0,46 | -0,60 | -0,53 |
| CDC25A | cell division cycle 25 homolog A (S. pombe) | 6,33 | -0,60 | -0,46 | -0,54 | -0,59 | -0,55 |
| PRR11 | proline rich 11 | 6,92 | -0,59 | -0,40 | -0,69 | -0,58 | -0,67 |
| CDC6 | cell division cycle 6 homolog (S. cerevisiae) | 7,64 | -0,38 | -0,22 | -0,57 | -0,57 | -0,52 |
| FLJ27243 | FLJ27243 protein | 6,60 | -0,48 | -0,54 | -0,47 | -0,55 | -0,65 |
| LOC100130131 | similar to melanoma antigen | 6,14 | -0,32 | -0,44 | -0,34 | -0,54 | -0,51 |
| LOC100130131 | similar to melanoma antigen | 6,14 | -0,32 | -0,44 | -0,34 | -0,54 | -0,51 |
| DLGAP5 | discs, large (Drosophila) homolog-associated protein 5 | 6,64 | -0,68 | -0,09 | -0,48 | -0,52 | -0,50 |
| TRIP13 | thyroid hormone receptor interactor 13 | 7,35 | -0,54 | -0,57 | -0,58 | -0,51 | -0,55 |
| LOC253724 | hypothetical LOC253724 | 3,82 | -0,59 | -0,70 | -0,95 | -0,51 | -0,79 |
| PLK4 | polo-like kinase 4 (Drosophila) | 6,23 | -0,43 | -0,18 | -0,58 | -0,50 | -0,55 |

**Tableau XI. Cibles prédites réprimées et communes à mir-449a, mir-449b, mir-34a, mir-34b* et mir-34c-5p et réprimées après transfection dans des HAECs de chacun des miARN sélectionné.**

| **Gene_Symbol** | **Gene Description** | **Niveau d'intensité d'expression en log2** | **Modulation en présence de mir34a** | **Modulation en présence de mir34b*** | **Modulation en présence de mir34c-5p** | **Modulation en présence de mir449a** | **Modulation en présence de mir449b** |
|---|---|---|---|---|---|---|---|
| DTL | denticleless homoloq (Drosophila) | 6,47 | -0,65 | -0,38 | -0,86 | -0,97 | -0,81 |
| KLRK1//KLRC4 | killer cell lectin-like receptor subfamily K, member 1 | 4,16 | -0,78 | -0,90 | -0,85 | -0,97 | -0,62 |
| KIAA0101 | KIAA0101 | 6,71 | -0,88 | -0,65 | -0,84 | -0,92 | -0,72 |
| CENPK | centromere protein K | 6,38 | -0,89 | -0,60 | -0,74 | -0,83 | -0,65 |
| CCNB2 | cyclin B2 | 6,51 | -0,65 | -0,55 | -0,59 | -0,83 | -0,77 |
| KIF11 | kinesin family member 11 | 5,98 | -0,43 | -0,20 | -0,58 | -0,82 | -0,54 |
| NUSAP1 | nucleolar and spindle associated protein 1 | 6,73 | -0,49 | -0,30 | -0,45 | -0,82 | -0,51 |
| TOP2A | topoisomerase (DNA) II alpha 170kDa | 7,06 | -0,70 | -0,21 | -0,63 | -0,80 | -0,52 |
| DEPDC1 | DEP domain containing 1 | 5,22 | -0,80 | -0,39 | -0,54 | -0,80 | -0,71 |
| RRM2 | ribonucleotide reductase M2 polypeptide | 7,72 | -0,57 | -0,29 | -0,76 | -0,71 | -0,60 |
| ARHGDIB | Rho GDP dissociation inhibitor (GDI) beta | 8,43 | -0,54 | -0,48 | -0,70 | -0,69 | -0,59 |
| CEP55 | centrosomal protein 55kDa | 6,68 | -0,52 | -0,37 | -0,62 | -0,68 | -0,64 |
| TPX2 | TPX2, microtubule-associated, homolog (Xenopus laevis) | 6,89 | -0,69 | -0,42 | -0,61 | -0,68 | -0,60 |
| FOXM1 | forkhead box M1 | 6,22 | -0,64 | -0,37 | -0,38 | -0,66 | -0,56 |
| AURKA | aurora kinase A | 7,42 | -0,51 | -0,38 | -0,51 | -0,65 | -0,66 |
| PRC1 | protein regulator of cytokinesis 1 | 7,81 | -0,57 | -0,48 | -0,57 | -0,60 | -0,65 |
| CDC25A | cell division cycle 25 homoloq A (S. pombe) | 6,33 | -0,60 | -0,46 | -0,54 | -0,59 | -0,55 |
| CDC6 | cell division cycle 6 homolog (S. cerevisiae) | 7,64 | -0,38 | -0,22 | -0,57 | -0,57 | -0,52 |

Une analyse bioinformatique à l'aide du software Ingenuity Pathway Analysis software (IPA) (Ingenuity Systems, Mountain View, USA) a permis d'identifier des réseaux d'interaction entre ces gènes et d'associer des fonctions biologiques et des maladies aux résultats expérimentaux (voir la **Figure 13**).

Les voies régulées par l'expression des miARN sélectionnés sont des voies majeures de régulation du cycle cellulaire.

### VI- Etude de l'impact fonctionel de la suppression de l'expression des miR-449 sur la ciliogénèse

L'impact de l'extinction de l'expression des miR-449 sur la ciliogénèse a ensuite été étudié.

### VI-A. Matériels et méthodes

### Cellules HAECs

Six cultures indépendantes de cellules HAEC ont été transfectées avec un oligonucléotide dirigé contre les miR-449 et conjugué à une molécule de cholestérol ; la ciliogenèse a été évaluée au cours du temps de régénération.

Ces essais utilisent un oligonucléotide antisens du miR-449a (antagomir) lié en 3' par une liaison 2'-O-méthyl à une molécule de cholestérol et a pour séquence : 5'-aₛcₛcₛagcuaacaauacacugcₛcₛa-Chol-3' (SEQ. ID. N°207) (les liaisons phosphorothioate sont indiquées par l'indice ₛ) (obtenu après de Eurogentec (Seraing, Belgique).

Cet antagomir cible le miR-449a d'*Homo sapiens* (correspondance complète) et le miR-449b avec un mésappariement.

Le contrôle négatif utilisé est le Clear-miR(tm) de séquence : 5'-cₛaₛuscgucgaucguagcgₛcₛa-Chol-3' (SEQ. ID. N°208) de Eurogentec.

L'antagomir (100 µM) a été pré-incubé dans du sérum de veau foetal (SVF) pendant 30 min RT. Ensuite, le mélange antagomir-SVF dans un milieu de différenciation (20 µM d'antagomir) est ajouté sur la face apicale des cellules HAECs primaires. Après 2h à 37°C, le mélange est retiré pour restaurer l'interface air-liquide.

La transfection est renouvelée tous les 5 jours avec un antagomir fraichement préparé jusqu'à ce que les cellules de controles aient atteint une différenciation complète (généralement après 21 jours).

### Xenopus laevis.

Les oligonucléotides morpholino (MO) dirigés contre les miR-449 (GeneTools, LLC, Philomath, Oregon, USA) ont pour séquence :
- MO-449a : 5'-ACCAGCTAACATTACACTGCCT-3' (SEQ. ID. N°209)
- MO-449b : 5'-GCCAGCTAAAACTACACTGCCT- 3' (SEQ. ID. N°210)
- MO-449c: 5'-ACAGCCAGCTAGCAAGTGCACTGCC-3' (SEQ. ID. N°211)
- et le MO de contrôle : 5'-TGCACGTTTCAATACAGACCGT-3' (SEQ. ID. N°212)

Le MO anti-DLL1 utilisé est celui décrit par Morichika. et al. (Dev Growth Differ 52 (2), 235 (2010).)

### VI-B. Résultats et discussions

Le miR-449 invalidé par son antagomir conduit à une réduction significative du nombre de cellules HAECs ciliées au stade ALI-D21 (moyenne de l'inhibition de la ciliogenèse: 2,3 ± 0,3, n = 6, P <0,001) (voir la **Figure 14****),** parallèlement à une réduction du même ordre de l'expression de miR-449 **(****Figure 14****,** cliché b).

MiR-449 a également été invalidé dans les cellules d'embryons de Xenope par injection épidermique d'un mélange d'oligonucléotides morpholino ciblant le miR-449 mature. L'invalidation de miR-449 empêche la multiciliogénèse, tel que révélé par coloration à la tubuline acétylée au stade de développement embryonnaire du bourgeon caudal et chez les têtards (n = 112) (voir la **Figure 14****,** clichés c à i).

La multiciliogénèse exige: (i) une sortie définitive du cycle cellulaire, suivie de (ii) d'une centriologénèse caractérisée par la multiplication de centaines de corps basaux, dérivés de centrioles nouvellement synthétisés ; (iii) la migration des corps basaux jusqu'à la membrane apicale où ils agissent en tant que centres d'organisation des microtubules et permettent l'assemblage des axonèmes mobiles.

Dans les deux modèles étudiés, le ratio de cellules tubuline-positives (coloration des cils) et de cellules centrine-2-positives (coloration des corps basaux) n'est pas affecté par la suppression de l'expression de miR-449, ce qui suggère que miR-449 agit avant la formation des centrioles.

Il est intéressant de noter que, alors que l'invalidation de miR-449 dans l'épiderme de Xenope supprime la multiciliogénèse, elle n'a pas supprimé l'expression des ARNm des marqueurs des cellules ciliées incluant l'α-tubuline, Tex15 et le facteur de transcription Foxj1 (voir la **Figure 15****).** Ces données indiquent que miR-449 est nécessaire à la différenciation terminale, mais pas à la spécification des cellules multiciliées, confirmant ainsi le rôle de miR-449 comme régulateur principal de la ciliogénèse chez les vertébrés.

### VII- Effet d'une transfection de miR-449 sur les cibles de miR-449

Afin d'évaluer l'impact de miR-449 sur la ciliogénèse, il est apparu souhaitable de vérifier que les cibles de miR-449 étaient inhibées au cours de la différenciation terminale et après transfection de miR-449.

### VII-A. Matériels et méthodes

### Analyse du cycle cellulaire par cytométrie de flux

Les cycles cellulaires de cellules A549 d'adénocarcinome du poumon sont synchronisées par une culture d'une nuit par carence en sérum puis transfectées avec les microARN ; les cellules sont ensuite cultivées en DMEM supplémenté avec L-glutamine et FCS à 10% jusqu'à 30% de confluence. Les cellules sont collectées 48h plus tard, fixées avec de l'éthanol 80% et colorées avec 0,1 ml d'une solution d'iodure de propidium (37°C, 30 minutes) contenant une RNAse A (50 µg/ml).

Les données sont lues sur un cytomètre en flux FACScalibur (Becton-Dickinson). Les pourcentages de cellule en phase G1, S et G2+M ont été calculés avec le logiciel Pro Cellquest.

### Construction des plasmides et mesures de l'activité luciférase

Des séquences complètes ou partielles de la région 3' non traduite de Areg, Ccnb1, Ccne2, Cdc25a, Dil1 et Notch1 ont été amplifiées et clonées dans le vecteur psiCheck2 (Promega).

Les constructions de vecteurs psiCheck ainsi obtenues ont été cotransfectées avec des microARN synthétiques ou un contrôle négatif (Ambion, Applied System) dans des cellules HEK293T. L'activité luciférase est mesurée tel que décrit par Pottier et al. (PLoS One 4 (8), e6718 (2009)).

### VII-B. Résultats

Les cibles de miR-449 ont été définies par l'analyse des profils d'expression suivants : (i) à quatre stades de régénération de cellules HAECs (ALI-D0, ALI-D7, ALI-D14, ALI-D21), (ii) avec cellules HAECs en multiplication transfectées avec miR-449. L'annotation fonctionnelle des ARNm différentiellement exprimés par Gene Set Enrichment Analysis (GSEA) (Edgar et al. Nucleic Acids Res 30 (1), 207 (2002)) révéle une augmentation significative des gènes associés au stade G2/M et à la ciliogénèse (voir la **Figure 16****).**

Les transcrits modulés par miR-449 sont analysés avec des outils de prédictions de cibles des microARN (http://www.microarray.fr: 8080/merge/index) conduisant à l'identification de plusieurs cibles potentielles de ce microARN, qui ont été validés avec un test luciférase **(****Figure 17****,** clichés a, c).

Un premier groupe de cibles validées de miR-449a/b comprend l'amphiregulin (Areg), la cycline B1 (Ccnb1), la cycline E2 (Ccne2), et la cell division cycle 25 homolog A (Cdc25A), qui codent pour des protéines impliquées dans régulation du cycle cellulaire. En effet, la transfection de miR-449 conduit à l'arrêt du cycle cellulaire en phase G1 **(****Figure 17b****),** tel que rapporté par Feng, M. et al. (Cell Cycle 9 (2), 213 (2010)), Lize et al. (Cell Death Differ (2009), Noonan et al. (Oncogene 28 (14), 1714 (2009)) et Yang, X. et al. (Genes Dev 23 (20), 2388 (2009)), et est une étape cruciale avant le démarrage de la centriogénèse.

Un deuxième groupe de cibles validées de miR-449 se compose de Notch1 et du ligand de Notch, DLL1 **(****Figure 17c****).** De façon intéressante, le blocage de la voie de signalisation Notch avec un antagoniste sécrétase gamma (DCT, 10 uM) pendant la régénération des cellules HAEC potentialise significativement la ciliogénèse **(****Figure 17d****)** ce qui est en accord avec la revue de Tsao et al. (Development 136 (13), 2297 (2009)).

Ces résultats sont cohérents avec l'observation que les précurseurs des cellules ciliées de l'épiderme de Xénope expriment transitoirement le ligand DII1 de Notch au moment de leur spécialisation et que l'expression de DII1 diminue rapidement avec le temps parallèlement avec l'accumulation miR-449 dans ces progéniteurs **(****Figure 17e****).**

Comme cela pouvait être prévu à partir de cette observation, l'expression endogène de DII1 est restée élevée dans les précurseurs des cellules ciliées des embryons modifiés avec le MO miR-449 (Figure 17 e et f) suggérant que miR-449 réprime l'expression de DII1.

### VIII- Effet de l'activité soutenue de DII1 sur la ciliogénèse.

Les conséquences d'une activité soutenue de DII1 sur la ciliogenèse a ensuite été évaluée.

L'injection d'un ARN synthétique de DII1 dépourvue de site de liaison à miR-449 a pour conséquence (1) une spécialisation excessive des cellules ciliées, (2) une ciliogénèse déficiente dans la grande majorité de ces cellules, phénotype identique à celui observé chez les embryons déplétés en miR-449 **(****Figure 17g****).**

L'inhibition latérale par la signalisation Notch est connue pour supprimer l'identité de la cellule multiciliée. L'augmentation de la spécification des cellules ciliées observée après une surexpression de DII1 est probablement causée par l'inhibition de l'activité de Notch, tel que rapporté par Deblandre et al. (Development 126 (21), 4715 (1999)).

Paralèllement, la suppression de l'expression de DII1 endogène par des oligonucléotides morpholinos a conduit à une spécification excessive des cellules ciliées **(****Figure 17g****).** Ainsi, miR-449 peut déclencher la multiciliogénèse en réprimant l'expression de DII1. À l'appui de ce modèle, une multiciliogénèse déficiente causée par l'extinction de l'expression de miR-449 est efficacement restaurée l'inhibition de la cible de miR-449, DII1 **(****Figure 17g****).**

Ainsi ces résultats montrent que la répression de l'expresion de DII1 est au centre du mécanisme d'action de miR-449.

## Revendications

1. miARN sélectionné dans le groupe constitué par hsa-miR-92b (SEQ ID N°49), hsa-miR-1975 (SEQ ID N°15), hsa-miR-99a (SEQ ID N°52), hsa-miR-191 (SEQ ID N°13), hsa-miR-378 (SEQ ID N°43), hsa-miR-125a-5p (SEQ ID N°3), hsa-miR-203 (SEQ ID N°19), et leur précurseurs de séquence SEQ ID N°107, 117, 119, 123, 147, 153 et 156 et/ou au moins un brin complémentaire des miARN éventuellement modifié chimiquement choisis parmi hsa-miR-205, hsa-miR-17, hsa-miR-29a, hsa-miR-193b, hsa-miR-210 hsa-miR-130a : hsa-miR-205* (SEQ ID N°72), hsa-miR-31 et hsa-miR-31* (SEQ ID N°37 et 89), hsa-miR-17* (SEQ ID N°63), hsa-miR-29a* (SEQ ID N°83), hsa-miR-193b* (SEQ ID N°66), hsa-miR-210* (SEQ ID N°74), hsa-miR-130a* (SEQ ID N°56) et leur précurseurs de séquence SEQ ID N°108, 115, 118, 124 à 126, 135 et 141 pour son utilisation pour la prévention et/ou le traitement de désordres liés à un dysfonctionnement des cils de tissu épithélial cilié.

2. miARN selon la revendication 1 pour son utilisation selon la revendication 1, **caractérisé en ce que** les désordres liés à un dysfonctionnement des cils de tissu épithélial cilié sont des ciliopathies primaires choisies parmi la dyskinésie ciliaire primaire ou syndrome de Kartagener ; *situs invertus;* l'infertilité masculine et féminine ; le syndrome d'Alstrom ; le syndrome de Bardet-Biedl ; le syndrome de Meckel-Gruber ; la polykystique des reins ; la dégénération rétinienne ; le syndrome de Senior-loken ou des ciliopathologies secondaires choisies parmi la mucoviscidose ; les broncho-pneumopathies obstructives chroniques (BCPO) ; l'asthme ; la bronchiolite et les infections respiratoires d'origine virale.

3. miARN **caractérisé en ce qu'**il comporte, du 2^{ème} au 8^{ème} nucléotide ou du 3^{ème} au 9^{ème} nucléotide, une séquence de reconnaissance sélectionnée dans le groupe constitué par GGCAGUG (SEQ ID N°175), AAUCACU (SEQ ID N°176) et AUCACUA (SEQ ID N°177) pour son utilisation pour la prévention et/ou le traitement des ciliopathies primaires choisies parmi la dyskinésie ciliaire primaire ou syndrome de Kartagener ; *situs invertus* ; l'infertilité masculine et féminine ; le syndrome d'Alstrom ; le syndrome de Bardet-Biedl ; le syndrome de Meckel-Gruber ; la dégénération rétinienne ; le syndrome de Senior-loken ou des ciliopathologies secondaires choisies parmi la mucoviscidose ; les broncho-pneumopathies obstructives chroniques (BCPO) ; l'asthme; la bronchiolite et les infections respiratoires d'origine virale.

4. miARN selon la revendication 3 pour son utilisation selon la revendication 3, **caractérisé en ce que** ledit miARN est sélectionné dans le groupe constitué par hsa-miR-34a (SEQ ID N°38), hsa-miR-34b (SEQ ID N°39), hsa-miR-34c-5p (SEQ ID N°40), hsa-miR-449a (SEQ ID N°46), hsa-miR-449b (SEQ ID N°47) et hsa-miR-449c (SEQ ID N°201).

5. miARN selon la revendication 1 pour son utilisation selon la revendication 1, **caractérisé en ce que** les désordres liés à un dysfonctionnement des cils de tissu épithélial cilié sont des pathologies impliquant un désordre de la régénération et/ou de la différenciation de l'épithélium respiratoire.

6. miARN selon la revendication 5 pour son utilisation selon la revendication 5, **caractérisé en ce que** les pathologies impliquant un désordre de la régénération et/ou de la différenciation de l'épithélium respiratoire sont choisies dans le groupe constitué par des maladies respiratoires chroniques et/ou héréditaires choisies parmi les broncho-pneumopathies obstructives chroniques (BCPO), la mucoviscidose, l'asthme, la dyskinésie ciliaire primaire, les inflammations et infections chroniques des voies respiratoires et les insuffisances respiratoires.

## Patentansprüche

1. Micro-RNA, ausgewählt aus der Gruppe, die gebildet ist von hsa-miR-92b (SEQ ID NR. 49), hsa-miR-1975 (SEQ ID NR. 15), hsa-miR-99a (SEQ ID NR. 52), hsa-miR-191 (SEQ ID NR. 13), hsa-miR-378 (SEQ ID NR. 43), hsa-miR-125a-5p (SEQ ID NR. 3), hsa-miR-203 (SEQ ID NR. 19) und ihren Vorläufern mit der Sequenz SEQ ID NR. 107, 117, 119, 123, 147, 153 und 156 und/oder mindestens einem komplementären Strang der eventuell chemisch modifizierten Micro-RNA, ausgewählt aus hsa-miR-205, hsa-miR-17, hsa-miR-29a, hsa-miR-193b, hsa-miR-210 hsa-miR-130a: hsa-miR-205* (SEQ ID NR. 72), hsa-miR-31 und hsa-miR-31* (SEQ ID NR. 37 und 89), hsa-miR-17* (SEQ ID NR. 63), hsa-miR-29a* (SEQ ID NR. 83), hsa-miR-193b* (SEQ ID NR. 66), hsa-miR-210* (SEQ ID NR. 74), hsa-miR-130a* (SEQ ID NR. 56) und ihren Vorläufern mit der Sequenz SEQ ID NR. 108, 115, 118, 124 bis 126, 135 und 141 für ihre Verwendung zur Vorbeugung und/oder Behandlung von Störungen, die mit einer Fehlfunktion der Zilien des ziliierten Epithelgewebes verbunden sind.

2. Micro-RNA nach Anspruch 1 für ihre Verwendung nach Anspruch 1, **dadurch gekennzeichnet, dass** die Störungen, die mit einer Fehlfunktion der Zilien des ziliierten Epithelgewebes verbunden sind, primäre Ziliopathien, die ausgewählt sind aus der primären Ziliendyskinesie oder dem Kartagener-Syndrom; *situs invertus;* der Unfruchtbarkeit beim Mann und bei der Frau; dem Alstrom-Syndrom; dem Bardet-Biedl-Syndrom; dem Meckel-Gruber-Syndrom; der multizystischen Niere; der Netzhautdegeneration; dem Senior-loken-Syndrom oder sekundäre Ziliopathien, ausgewählt aus der Mukoviszidose; den chronischen obstruktiven Broncho-Pneumopathien (COPD); dem Asthma; der Bronchiolitis und den viralen Atemwegserkrankungen, sind.

3. Micro-RNA, **dadurch gekennzeichnet**, das sie vom 2. bis 8. Nukleotid oder vom 3. bis 9. Nukleotid eine Erkennungssequenz aufweist, ausgewählt aus der Gruppe, die gebildet ist von GGCAGUGA (SEQ ID NR. 175), AAUCACU (SEQ ID NR. 176) und AUCACUA (SEQ ID NR. 177), für ihre Verwendung zur Vorbeugung und/oder Behandlung von primären Ziliopathien, ausgewählt aus der primären Ziliendyskinesie oder dem Kartagener-Syndrom; situs *invertus;* der Unfruchtbarkeit beim Mann und bei der Frau; dem Alstrom-Syndrom; dem Bardet-Biedl-Syndrom; dem Meckel-Gruber-Syndrom; der Netzhautdegeneration; dem Senior-loken-Syndrom oder von sekundären Ziliopathien, ausgewählt aus der Mukoviszidose; den chronischen obstruktiven Broncho-Pneumopathien (COPD); dem Asthma; der Bronchiolitis und den viralen Atemwegserkrankungen.

4. Micro-RNA nach Anspruch 3 für ihre Verwendung nach Anspruch 3, **dadurch gekennzeichnet, dass** die Micro-RNA aus der Gruppe ausgewählt ist, die von hsa-miR-34a (SEQ ID NR. 38), hsa-miR-34b (SEQ ID NR. 39), hsa-miR-34c-5p (SEQ ID NR. 40), hsa- miR-449a (SEQ ID NR. 46), hsa-miR-449b (SEQ ID NR. 47) und hsa-miR-449c (SEQ ID NR. 201) gebildet ist.

5. Micro-RNA nach Anspruch 1 für ihre Verwendung nach Anspruch 1, **dadurch gekennzeichnet, dass** die mit einer Fehlfunktion der Zilien des ziliierten Epithelgewebes verbundenen Störungen Erkrankungen sind, die eine Fehlfunktion der Regeneration und/oder der Differenzierung des respiratorischen Epitheliums implizieren.

6. Micro-RNA nach Anspruch 5 für ihre Verwendung nach Anspruch 5, **dadurch gekennzeichnet, dass** die Erkrankungen, die eine Störung der Regeneration und/oder der Differenzierung des respiratorischen Epitheliums implizieren, aus der Gruppe ausgewählt sind, die von den chronischen und/oder erblichen Atemwegserkrankungen gebildet ist, die aus den chronischen obstruktiven Broncho-Pneumopathien (COPD), der Mukoviszidose, dem Asthma, der primären Ziliendyskinesie, den chronischen Entzündungen und Infektionen der Atemwege und der respiratorischen Insuffizienz ausgewählt sind.

## Claims

1. A miRNA selected from the group consisting of hsa-miR-92b (SEQ ID NO.49), hsa-miR-1975 (SEQ ID NO.15), hsa-miR-99a (SEQ ID NO.52), hsa-miR-191 (SEQ ID NO.13), hsa-miR-378 (SEQ ID NO.43), hsa-miR-125a-5p(SEQ ID NO.3), hsa-miR-203(SEQ ID NO.19), and their sequence precursors SEQ ID NOS.107,117,119,123,147,153 and 156 and/or at least one complementary strand of miRNAs, optionally chemically modified selected from among hsa-miR-205, hsa-miR-17, hsa-miR-29a, hsa-miR-193b, hsa-miR-210, hsa-miR-130a: hsa-miR-205* (SEQ ID NO.72), hsa-miR-31 and hsa-miR-31* (SEQ ID NOS.37 and 89), hsa-miR-17* (SEQ ID NO.63), hsa-miR-29a* (SEQ ID NO.83), hsa-miR-193b (SEQ ID NO.66), hsa-miR-210* (SEQ ID NO.74), hsa-miR-130a* (SEQ ID NO.56), and their sequence precursors SEQ ID NOS.108, 115, 118, 124 to 126, 135 and 141 for its use for preventing and/or treating disorders related to dysfunction of the lashes of ciliated epithelial tissue.

2. The miRNA according to claim 1, for its use according to claim 1, **characterized in that** the disorders related to dysfunction of the lashes of the ciliated epithelial tissue are primary ciliopathies selected from among primary ciliary dyskinesia or Kartagener's syndrome; *situs invertus;* male and female infertility, Alstrom's syndrome; Bardet-Biedl syndrome; Meckel-Gruber syndrome; polycystic kidney disease; retinal degeneration; Senior-Loken syndrome or secondary ciliopathies selected from among cystic fibrosis; chronic obstructive pulmonary disease (COPD); asthma; bronchiolitis and respiratory infections of viral origin.

3. An miRNA, **characterized in that** it includes from the 2^{nd} to the 8^{th} nucléotide or from the 3^{rd} to the 9^{th} nucleotide, a recognition sequence), selected from the group formed with GGCAGUG (SEQ ID NO.175), AAUCACU (SEQ ID NO.176) and AUCACUA (SEQ ID NO.177) for its use for preventing and/or treating primary ciliopathies selected from primary ciliary dyskinesia or Kartagener's syndrome; *situs invertus;* male and female infertility, Alstrom's syndrome; Bardet-Biedl syndrome; Meckel-Gruber syndrome; retinal degeneration; Senior-Loken syndrome or secondary ciliopathies selected from among cystic fibrosis; chronic obstructive pulmonary disease (COPD); asthma; bronchiolitis and respiratory infections of viral origin.

4. The miRNA according to claim 3 for its use according to claim 3, **characterized in that** said miRNA is selected from the group formed with hsa-miR-34a (SEQ ID NO.38), hsa-miR-34b (SEQ ID NO.39), hsa-miR-34c-5p (SEQ ID NO.40), hsa-miR-449a (SEQ ID NO.46), hsa-miR-449b (SEQ ID NO.47) and hsa-miR-449c (SEQ ID NO.201).

5. The miRNA according to claim 1, for its use according to claim 1, **characterized in that** the disorders related to dysfunction of the lashes of the ciliated epithelial tissue are pathologies involving a disorder of the regeneration and/or the differentiation of the respiratory epithelium.

6. The miRNA according to claim 5, for its use according to claim 5, **characterized in that** the pathologies involving a disorder of the regeneration and/or the differentiation of the respiratory epithelium are selected from among the group formed with chronic and/or hereditary respiratory diseases selected from among chronic obstructive pulmonary diseases (COPD), cystic fibrosis, asthma, primary ciliar dyskinesia, chronic inflammations and infections of respiratory airways and respiratory disorders.


### SEQUENCE LISTING

<110> CENTRE NATIONAL DE LA RECHERCHE SCIENTIFIQUE
<120> utilisation de microARN pour le traitement de pathologies liées à un dysfonctionnement des cils des cellules épithéliales multiciliées
<130> F644 Cas251WO
<160> 212
<170> PatentIn version 3.4
<210> 1
   <211> 22
   <212> RNA
   <213> Homo sapiens
<400> 1
   aacccguaga uccgaacuug ug 22
<210> 2
   <211> 21
   <212> RNA
   <213> Homo sapiens
<400> 2
   uaaagugcug acagugcaga u 21
<210> 3
   <211> 24
   <212> RNA
   <213> Homo sapiens
<400> 3
   ucccugagac ccuuuaaccu guga 24
<210> 4
   <211> 22
   <212> RNA
   <213> Homo sapiens
<400> 4
   cagugcaaug uuaaaagggc au 22
<210> 5
   <211> 21
   <212> RNA
   <213> Homo sapiens
<400> 5
   uaccacaggg uagaaccacg g 21
<210> 6
   <211> 22
   <212> RNA
   <213> Homo sapiens
<400> 6
   uaacacuguc ugguaaagau gg 22
<210> 7
   <211> 22
   <212> RNA
   <213> Homo sapiens
<400> 7
   ucagugcacu acagaacuuu gu 22
<210> 8
   <211> 21
   <212> RNA
   <213> Homo sapiens
<400> 8
   ucgaggagcu cacagucuag u 21
<210> 9
   <211> 22
   <212> RNA
   <213> Homo sapiens
<400> 9
   uagcagcaca uaaugguuug ug 22
<210> 10
   <211> 22
   <212> RNA
   <213> Homo sapiens
<400> 10
   uagcagcacg uaaauauugg cg 22
<210> 11
   <211> 23
   <212> RNA
   <213> Homo sapiens
<400> 11
   caaagugcuu acagugcagg uag 23
<210> 12
   <211> 23
   <212> RNA
   <213> Homo sapiens
<400> 12
   aacauucaac gcugucggug agu 23
<210> 13
   <211> 23
   <212> RNA
   <213> Homo sapiens
<400> 13
   caacggaauc ccaaaagcag cug 23
<210> 14
   <211> 22
   <212> RNA
   <213> Homo sapiens
<400> 14
   aacuggcccu caaagucccg cu 22
<210> 15
   <211> 25
   <212> RNA
   <213> Homo sapiens
<400> 15
   cccccacaac cgcgcuugac uagcu 25
<210> 16
   <211> 22
   <212> RNA
   <213> Homo sapiens
<400> 16
   uaacacuguc ugguaacgau gu 22
<210> 17
   <211> 22
   <212> RNA
   <213> Homo sapiens
<400> 17
   uaauacugcc ugguaaugau ga 22
<210> 18
   <211> 23
   <212> RNA
   <213> Homo sapiens
<400> 18
   uaauacugcc ggguaaugau gga 23
<210> 19
   <211> 22
   <212> RNA
   <213> Homo sapiens
<400> 19
   gugaaauguu uaggaccacu ag 22
<210> 20
   <211> 22
   <212> RNA
   <213> Homo sapiens
<400> 20
   uccuucauuc caccggaguc ug 22
<210> 21
   <211> 22
   <212> RNA
   <213> Homo sapiens
<400> 21
   uagcuuauca gacugauguu ga 22
<210> 22
   <211> 22
   <212> RNA
   <213> Homo sapiens
<400> 22
   cugugcgugu gacagcggcu ga 22
<210> 23
   <211> 22
   <212> RNA
   <213> Homo sapiens
<400> 23
   aagcugccag uugaagaacu gu 22
<210> 24
   <211> 21
   <212> RNA
   <213> Homo sapiens
<400> 24
   caagucacua gugguuccgu u 21
<210> 25
   <211> 21
   <212> RNA
   <213> Homo sapiens
<400> 25
   aucacauugc cagggauuuc c 21
<210> 26
   <211> 21
   <212> RNA
   <213> Homo sapiens
<400> 26
   aucacauugc cagggauuac c 21
<210> 27
   <211> 22
   <212> RNA
   <213> Homo sapiens
<400> 27
   cauugcacuu gucucggucu ga 22
<210> 28
   <211> 22
   <212> RNA
   <213> Homo sapiens
<400> 28
   uucaaguaau ccaggauagg cu 22
<210> 29
   <211> 21
   <212> RNA
   <213> Homo sapiens
<400> 29
   uucaaguaau ucaggauagg u 21
<210> 30
   <211> 21
   <212> RNA
   <213> Homo sapiens
<400> 30
   uucacagugg cuaaguucug c 21
<210> 31
   <211> 22
   <212> RNA
   <213> Homo sapiens
<400> 31
   uagcaccauc ugaaaucggu ua 22
<210> 32
   <211> 22
   <212> RNA
   <213> Homo sapiens
<400> 32
   uagcaccauu ugaaaucggu ua 22
<210> 33
   <211> 22
   <212> RNA
   <213> Homo sapiens
<400> 33
   uguaaacauc cuacacucag cu 22
<210> 34
   <211> 23
   <212> RNA
   <213> Homo sapiens
<400> 34
   uguaaacauc cuacacucuc agc 23
<210> 35
   <211> 22
   <212> RNA
   <213> Homo sapiens
<400> 35
   uguaaacauc cccgacugga ag 22
<210> 36
   <211> 22
   <212> RNA
   <213> Homo sapiens
<400> 36
   uguaaacauc cuugacugga ag 22
<210> 37
   <211> 21
   <212> RNA
   <213> Homo sapiens
<400> 37
   aggcaagaug cuggcauagc u 21
<210> 38
   <211> 22
   <212> RNA
   <213> Homo sapiens
<400> 38
   uggcaguguc uuagcugguu gu 22
<210> 39
   <211> 22
   <212> RNA
   <213> Homo sapiens
<400> 39
   caaucacuaa cuccacugcc au 22
<210> 40
   <211> 23
   <212> RNA
   <213> Homo sapiens
<400> 40
   aggcagugua guuagcugau ugc 23
<210> 41
   <211> 22
   <212> RNA
   <213> Homo sapiens
<400> 41
   uaaugccccu aaaaauccuu au 22
<210> 42
   <211> 22
   <212> RNA
   <213> Homo sapiens
<400> 42
   uuauaauaca accugauaag ug 22
<210> 43
   <211> 21
   <212> RNA
   <213> Homo sapiens
<400> 43
   acuggacuug gagucagaag g 21
<210> 44
   <211> 23
   <212> RNA
   <213> Homo sapiens
<400> 44
   aaugacacga ucacucccgu uga 23
<210> 45
   <211> 22
   <212> RNA
   <213> Homo sapiens
<400> 45
   uaauacuguc ugguaaaacc gu 22
<210> 46
   <211> 22
   <212> RNA
   <213> Homo sapiens
<400> 46
   uggcagugua uuguuagcug gu 22
<210> 47
   <211> 22
   <212> RNA
   <213> Homo sapiens
<400> 47
   aggcagugua uuguuagcug gc 22
<210> 48
   <211> 22
   <212> RNA
   <213> Homo sapiens
<400> 48
   cacgcucaug cacacaccca ca 22
<210> 49
   <211> 22
   <212> RNA
   <213> Homo sapiens
<400> 49
   uauugcacuc gucccggccu cc 22
<210> 50
   <211> 24
   <212> RNA
   <213> Homo sapiens
<400> 50
   uggggagcug aggcucuggg ggug 24
<210> 51
   <211> 23
   <212> RNA
   <213> Homo sapiens
<400> 51
   uuuggcacua gcacauuuuu gcu 23
<210> 52
   <211> 22
   <212> RNA
   <213> Homo sapiens
<400> 52
   aacccguaga uccgaucuug ug 22
<210> 53
   <211> 22
   <212> RNA
   <213> Homo sapiens
<400> 53
   caagcuugua ucuauaggua ug 22
<210> 54
   <211> 22
   <212> RNA
   <213> Homo sapiens
<400> 54
   ccgcacugug gguacuugcu gc 22
<210> 55
   <211> 22
   <212> RNA
   <213> Homo sapiens
<400> 55
   acaggugagg uucuugggag cc 22
<210> 56
   <211> 22
   <212> RNA
   <213> Homo sapiens
<400> 56
   uucacauugu gcuacugucu gc 22
<210> 57
   <211> 22
   <212> RNA
   <213> Homo sapiens
<400> 57
   cagugguuuu acccuauggu ag 22
<210> 58
   <211> 22
   <212> RNA
   <213> Homo sapiens
<400> 58
   caucuuccag uacaguguug ga 22
<210> 59
   <211> 22
   <212> RNA
   <213> Homo sapiens
<400> 59
   aaaguucuga gacacuccga cu 22
<210> 60
   <211> 21
   <212> RNA
   <213> Homo sapiens
<400> 60
   cuagacugaa gcuccuugag g 21
<210> 61
   <211> 22
   <212> RNA
   <213> Homo sapiens
<400> 61
   caggccauau ugugcugccu ca 22
<210> 62
   <211> 22
   <212> RNA
   <213> Homo sapiens
<400> 62
   ccaguauuaa cugugcugcu ga 22
<210> 63
   <211> 22
   <212> RNA
   <213> Homo sapiens
<400> 63
   acugcaguga aggcacuugu ag 22
<210> 64
   <211> 22
   <212> RNA
   <213> Homo sapiens
<400> 64
   accaucgacc guugauugua cc 22
<210> 65
   <211> 22
   <212> RNA
   <213> Homo sapiens
<400> 65
   gcugcgcuug gauuucgucc cc 22
<210> 66
   <211> 22
   <212> RNA
   <213> Homo sapiens
<400> 66
   cgggguuuug agggcgagau ga 22
<210> 67
   <211> 25
   <212> RNA
   <213> Homo sapiens
<400> 67
   ggggguguug gcgcgaacug aucga 25
<210> 68
   <211> 22
   <212> RNA
   <213> Homo sapiens
<400> 68
   caucuuaccg gacagugcug ga 22
<210> 69
   <211> 22
   <212> RNA
   <213> Homo sapiens
<400> 69
   caucuuacug ggcagcauug ga 22
<210> 70
   <211> 22
   <212> RNA
   <213> Homo sapiens
<400> 70
   cgucuuaccc agcaguguuu gg 22
<210> 71
   <211> 22
   <212> RNA
   <213> Homo sapiens
<400> 71
   cacuuuacaa auccugguga uc 22
<210> 72
   <211> 21
   <212> RNA
   <213> Homo sapiens
<400> 72
   gauuucagug gagugaaguu c 21
<210> 73
   <211> 21
   <212> RNA
   <213> Homo sapiens
<400> 73
   caacaccagu cgaugggcug u 21
<210> 74
   <211> 22
   <212> RNA
   <213> Homo sapiens
<400> 74
   gacacgcaca cugucgccga cu 22
<210> 75
   <211> 22
   <212> RNA
   <213> Homo sapiens
<400> 75
   aguucuucag uggcaagcuu ua 22
<210> 76
   <211> 23
   <212> RNA
   <213> Homo sapiens
<400> 76
   aaaauggugc ccuagugacu aca 23
<210> 77
   <211> 22
   <212> RNA
   <213> Homo sapiens
<400> 77
   gggguuccug gggaugggau uu 22
<210> 78
   <211> 22
   <212> RNA
   <213> Homo sapiens
<400> 78
   uggguuccug gcaugcugau uu 22
<210> 79
   <211> 21
   <212> RNA
   <213> Homo sapiens
<400> 79
   aggcggagac uugggcaauu g 21
<210> 80
   <211> 22
   <212> RNA
   <213> Homo sapiens
<400> 80
   ccuauucuug guuacuugca cg 22
<210> 81
   <211> 22
   <212> RNA
   <213> Homo sapiens
<400> 81
   ccuguucucc auuacuuggc uc 22
<210> 82
   <211> 22
   <212> RNA
   <213> Homo sapiens
<400> 82
   agagcuuagc ugauugguga ac 22
<210> 83
   <211> 22
   <212> RNA
   <213> Homo sapiens
<400> 83
   acugauuucu uuugguguuc ag 22
<210> 84
   <211> 22
   <212> RNA
   <213> Homo sapiens
<400> 84
   ugaccgauuu cuccuggugu uc 22
<210> 85
   <211> 22
   <212> RNA
   <213> Homo sapiens
<400> 85
   cugggaggug gauguuuacu uc 22
<210> 86
   <211> 22
   <212> RNA
   <213> Homo sapiens
<400> 86
   cugggagaag gcuguuuacu cu 22
<210> 87
   <211> 22
   <212> RNA
   <213> Homo sapiens
<400> 87
   cuuucaguca gauguuugcu gc 22
<210> 88
   <211> 22
   <212> RNA
   <213> Homo sapiens
<400> 88
   cuuucagucg gauguuuaca gc 22
<210> 89
   <211> 22
   <212> RNA
   <213> Homo sapiens
<400> 89
   ugcuaugcca acauauugcc au 22
<210> 90
   <211> 22
   <212> RNA
   <213> Homo sapiens
<400> 90
   caaucagcaa guauacugcc cu 22
<210> 91
   <211> 23
   <212> RNA
   <213> Homo sapiens
<400> 91
   uaggcagugu cauuagcuga uug 23
<210> 92
   <211> 22
   <212> RNA
   <213> Homo sapiens
<400> 92
   aaucacuaac cacacggcca gg 22
<210> 93
   <211> 22
   <212> RNA
   <213> Homo sapiens
<400> 93
   agggacuuuc aggggcagcu gu 22
<210> 94
   <211> 22
   <212> RNA
   <213> Homo sapiens
<400> 94
   cuuaucagau uguauuguaa uu 22
<210> 95
   <211> 22
   <212> RNA
   <213> Homo sapiens
<400> 95
   cuccugacuc cagguccugu gu 22
<210> 96
   <211> 22
   <212> RNA
   <213> Homo sapiens
<400> 96
   aucgggaaug ucguguccgc cc 22
<210> 97
   <211> 22
   <212> RNA
   <213> Homo sapiens
<400> 97
   auuaugacag accauuuugg ca 22
<210> 98
   <211> 22
   <212> RNA
   <213> Homo sapiens
<400> 98
   accgucacau aacaaucgac ca 22
<210> 99
   <211> 22
   <212> RNA
   <213> Homo sapiens
<400> 99
   cagccacaac uacccugcca cu 22
<210> 100
   <211> 23
   <212> RNA
   <213> Homo sapiens
<400> 100
   ugagugugug ugugugagug ugu 23
<210> 101
   <211> 22
   <212> RNA
   <213> Homo sapiens
<400> 101
   auaacgugag cagggccgga gg 22
<210> 102
   <211> 24
   <212> RNA
   <213> Homo sapiens
<400> 102
   accccucgac uccgagaccc ccac 24
<210> 103
   <211> 22
   <212> RNA
   <213> Homo sapiens
<400> 103
   aaucaugugc agugccaaua ug 22
<210> 104
   <211> 22
   <212> RNA
   <213> Homo sapiens
<400> 104
   caagcucgcu ucuauggguc ug 22
<210> 105
   <211> 80
   <212> DNA
   <213> Homo sapiens
<400> 105
   ccuguugcca caaacccgua gauccgaacu ugugguauua guccgcacaa gcuuguaucu 60
   auagguaugu gucuguuagg 80
<210> 106
   <211> 82
   <212> DNA
   <213> Homo sapiens
<400> 106
   ccugccgggg cuaaagugcu gacagugcag auaguggucc ucuccgugcu accgcacugu 60
   ggguacuugc ugcuccagca gg 82
<210> 107
   <211> 86
   <212> DNA
   <213> Homo sapiens
<400> 107
   ugccagucuc uaggucccug agacccuuua accugugagg acauccaggg ucacagguga 60
   gguucuuggg agccuggcgu cuggcc 86
<210> 108
   <211> 89
   <212> DNA
   <213> Homo sapiens
<400> 108
   ugcugcuggc cagagcucuu uucacauugu gcuacugucu gcaccuguca cuagcagugc 60
   aauguuaaaa gggcauuggc cguguagug 89
<210> 109
   <211> 100
   <212> DNA
   <213> Homo sapiens
<400> 109
   ugugucucuc ucuguguccu gccagugguu uuacccuaug guagguuacg ucaugcuguu 60
   cuaccacagg guagaaccac ggacaggaua ccggggcacc 100
<210> 110
   <211> 95
   <212> DNA
   <213> Homo sapiens
<400> 110
   cggccggccc uggguccauc uuccaguaca guguuggaug gucuaauugu gaagcuccua 60
   acacugucug guaaagaugg cucccgggug gguuc 95
<210> 111
   <211> 68
   <212> DNA
   <213> Homo sapiens
<400> 111
   gaggcaaagu ucugagacac uccgacucug aguaugauag aagucagugc acuacagaac 60
   uuugucuc 68
<210> 112
   <211> 90
   <212> DNA
   <213> Homo sapiens
<400> 112
   uuuccugccc ucgaggagcu cacagucuag uaugucucau ccccuacuag acugaagcuc 60
   cuugaggaca gggaugguca uacucaccuc 90
<210> 113
   <211> 83
   <212> DNA
   <213> Homo sapiens
<400> 113
   ccuuggagua aaguagcagc acauaauggu uuguggauuu ugaaaaggug caggccauau 60
   ugugcugccu caaaaauaca agg 83
<210> 114
   <211> 89
   <212> DNA
   <213> Homo sapiens
<400> 114
   gucagcagug ccuuagcagc acguaaauau uggcguuaag auucuaaaau uaucuccagu 60
   auuaacugug cugcugaagu aagguugac 89
<210> 115
   <211> 84
   <212> DNA
   <213> Homo sapiens
<400> 115
   gucagaauaa ugucaaagug cuuacagugc agguagugau augugcaucu acugcaguga 60
   aggcacuugu agcauuaugg ugac 84
<210> 116
   <211> 110
   <212> DNA
   <213> Homo sapiens
<400> 116
   ugaguuuuga gguugcuuca gugaacauuc aacgcugucg gugaguuugg aauuaaaauc 60
   aaaaccaucg accguugauu guacccuaug gcuaaccauc aucuacucca 110
<210> 117
   <211> 92
   <212> DNA
   <213> Homo sapiens
<400> 117
   cggcuggaca gcgggcaacg gaaucccaaa agcagcuguu gucuccagag cauuccagcu 60
   gcgcuuggau uucguccccu gcucuccugc cu 92
<210> 118
   <211> 83
   <212> DNA
   <213> Homo sapiens
<400> 118
   guggucucag aaucgggguu uugagggcga gaugaguuua uguuuuaucc aacuggcccu 60
   caaagucccg cuuuuggggu cau 83
<210> 119
   <211> 75
   <212> DNA
   <213> Homo sapiens
<400> 119
   aguugguccg aguguugugg guuauuguua aguugauuua acauugucuc cccccacaac 60
   cgcgcuugac uagcu 75
<210> 120
   <211> 90
   <212> DNA
   <213> Homo sapiens
<400> 120
   ccgggccccu gugagcaucu uaccggacag ugcuggauuu cccagcuuga cucuaacacu 60
   gucugguaac gauguucaaa ggugacccgc 90
<210> 121
   <211> 95
   <212> DNA
   <213> Homo sapiens
<400> 121
   ccagcucggg cagccguggc caucuuacug ggcagcauug gauggaguca ggucucuaau 60
   acugccuggu aaugaugacg gcggagcccu gcacg 95
<210> 122
   <211> 68
   <212> DNA
   <213> Homo sapiens
<400> 122
   cccucgucuu acccagcagu guuugggugc gguugggagu cucuaauacu gccggguaau 60
   gauggagg 68
<210> 123
   <211> 110
   <212> DNA
   <213> Homo sapiens
<400> 123
   guguugggga cucgcgcgcu ggguccagug guucuuaaca guucaacagu ucuguagcgc 60
   aauugugaaa uguuuaggac cacuagaccc ggcgggcgcg gcgacagcga 110
<210> 124
   <211> 110
   <212> DNA
   <213> Homo sapiens
<400> 124
   aaagauccuc agacaaucca ugugcuucuc uuguccuuca uuccaccgga gucugucuca 60
   uacccaacca gauuucagug gagugaaguu caggaggcau ggagcugaca 110
<210> 125
   <211> 72
   <212> DNA
   <213> Homo sapiens
<400> 125
   ugucggguag cuuaucagac ugauguugac uguugaaucu cauggcaaca ccagucgaug 60
   ggcugucuga ca 72
<210> 126
   <211> 110
   <212> DNA
   <213> Homo sapiens
<400> 126
   acccggcagu gccuccaggc gcagggcagc cccugcccac cgcacacugc gcugccccag 60
   acccacugug cgugugacag cggcugaucu gugccugggc agcgcgaccc 110
<210> 127
   <211> 85
   <212> DNA
   <213> Homo sapiens
<400> 127
   ggcugagccg caguaguucu ucaguggcaa gcuuuauguc cugacccagc uaaagcugcc 60
   aguugaagaa cuguugcccu cugcc 85
<210> 128
   <211> 81
   <212> DNA
   <213> Homo sapiens
<400> 128
   gggcuuucaa gucacuagug guuccguuua guagaugauu gugcauuguu ucaaaauggu 60
   gcccuaguga cuacaaagcc c 81
<210> 129
   <211> 73
   <212> DNA
   <213> Homo sapiens
<400> 129
   ggccggcugg gguuccuggg gaugggauuu gcuuccuguc acaaaucaca uugccaggga 60
   uuuccaaccg acc 73
<210> 130
   <211> 97
   <212> DNA
   <213> Homo sapiens
<400> 130
   cucaggugcu cuggcugcuu ggguuccugg caugcugauu ugugacuuaa gauuaaaauc 60
   acauugccag ggauuaccac gcaaccacga ccuuggc 97
<210> 131
   <211> 84
   <212> DNA
   <213> Homo sapiens
<400> 131
   ggccaguguu gagaggcgga gacuugggca auugcuggac gcugcccugg gcauugcacu 60
   ugucucgguc ugacagugcc ggcc 84
<210> 132
   <211> 77
   <212> DNA
   <213> Homo sapiens
<400> 132
   guggccucgu ucaaguaauc caggauaggc ugugcagguc ccaaugggcc uauucuuggu 60
   uacuugcacg gggacgc 77
<210> 133
   <211> 77
   <212> DNA
   <213> Homo sapiens
<400> 133
   ccgggaccca guucaaguaa uucaggauag guugugugcu guccagccug uucuccauua 60
   cuuggcucgg ggaccgg 77
<210> 134
   <211> 97
   <212> DNA
   <213> Homo sapiens
<400> 134
   accucucuaa caaggugcag agcuuagcug auuggugaac agugauuggu uuccgcuuug 60
   uucacagugg cuaaguucug caccugaaga gaaggug 97
<210> 135
   <211> 64
   <212> DNA
   <213> Homo sapiens
<400> 135
   augacugauu ucuuuuggug uucagaguca auauaauuuu cuagcaccau cugaaaucgg 60
   uuau 64
<210> 136
   <211> 88
   <212> DNA
   <213> Homo sapiens
<400> 136
   aucucuuaca caggcugacc gauuucuccu gguguucaga gucuguuuuu gucuagcacc 60
   auuugaaauc gguuaugaug uaggggga 88
<210> 137
   <211> 88
   <212> DNA
   <213> Homo sapiens
<400> 137
   accaaguuuc aguucaugua aacauccuac acucagcugu aauacaugga uuggcuggga 60
   gguggauguu uacuucagcu gacuugga 88
<210> 138
   <211> 89
   <212> DNA
   <213> Homo sapiens
<400> 138
   accaugcugu agugugugua aacauccuac acucucagcu gugagcucaa gguggcuggg 60
   agaggguugu uuacuccuuc ugccaugga 89
<210> 139
   <211> 70
   <212> DNA
   <213> Homo sapiens
<400> 139
   guuguuguaa acauccccga cuggaagcug uaagacacag cuaagcuuuc agucagaugu 60
   uugcugcuac 70
<210> 140
   <211> 92
   <212> DNA
   <213> Homo sapiens
<400> 140
   gggcagucuu ugcuacugua aacauccuug acuggaagcu guaagguguu cagaggagcu 60
   uucagucgga uguuuacagc ggcaggcugc ca 92
<210> 141
   <211> 71
   <212> DNA
   <213> Homo sapiens
<400> 141
   ggagaggagg caagaugcug gcauagcugu ugaacuggga accugcuaug ccaacauauu 60
   gccaucuuuc c 71
<210> 142
   <211> 110
   <212> DNA
   <213> Homo sapiens
<400> 142
   ggccagcugu gaguguuucu uuggcagugu cuuagcuggu uguugugagc aauaguaagg 60
   aagcaaucag caaguauacu gcccuagaag ugcugcacgu uguggggccc 110
<210> 143
   <211> 84
   <212> DNA
   <213> Homo sapiens
<400> 143
   gugcucgguu uguaggcagu gucauuagcu gauuguacug uggugguuac aaucacuaac 60
   uccacugcca ucaaaacaag gcac 84
<210> 144
   <211> 77
   <212> DNA
   <213> Homo sapiens
<400> 144
   agucuaguua cuaggcagug uaguuagcug auugcuaaua guaccaauca cuaaccacac 60
   ggccagguaa aaagauu 77
<210> 145
   <211> 87
   <212> DNA
   <213> Homo sapiens
<400> 145
   accgcaggga aaaugaggga cuuuuggggg cagauguguu uccauuccac uaucauaaug 60
   ccccuaaaaa uccuuauugc ucuugca 87
<210> 146
   <211> 72
   <212> DNA
   <213> Homo sapiens
<400> 146
   uacaucggcc auuauaauac aaccugauaa guguuauagc acuuaucaga uuguauugua 60
   auugucugug ua 72
<210> 147
   <211> 66
   <212> DNA
   <213> Homo sapiens
<400> 147
   agggcuccug acuccagguc cuguguguua ccuagaaaua gcacuggacu uggagucaga 60
   aggccu 66
<210> 148
   <211> 87
   <212> DNA
   <213> Homo sapiens
<400> 148
   gaaagcgcuu uggaaugaca cgaucacucc cguugagugg gcacccgaga agccaucggg 60
   aaugucgugu ccgcccagug cucuuuc 87
<210> 149
   <211> 83
   <212> DNA
   <213> Homo sapiens
<400> 149
   cgccggccga ugggcgucuu accagacaug guuagaccug gcccucuguc uaauacuguc 60
   ugguaaaacc guccauccgc ugc 83
<210> 150
   <211> 91
   <212> DNA
   <213> Homo sapiens
<400> 150
   cuguguguga ugagcuggca guguauuguu agcugguuga auaugugaau ggcaucggcu 60
   aacaugcaac ugcugucuua uugcauauac a 91
<210> 151
   <211> 97
   <212> DNA
   <213> Homo sapiens
<400> 151
   ugaccugaau cagguaggca guguauuguu agcuggcugc uugggucaag ucagcagcca 60
   caacuacccu gccacuugcu ucuggauaaa uucuucu 97
<210> 152
   <211> 96
   <212> DNA
   <213> Homo sapiens
<400> 152
   gggaccugcg ugggugcggg cgugugagug ugugugugug aguguguguc gcuccggguc 60
   cacgcucaug cacacaccca cacgcccaca cucagg 96
<210> 153
   <211> 96
   <212> DNA
   <213> Homo sapiens
<400> 153
   cgggccccgg gcgggcggga gggacgggac gcggugcagu guuguuuuuu cccccgccaa 60
   uauugcacuc gucccggccu ccggcccccc cggccc 96
<210> 154
   <211> 82
   <212> DNA
   <213> Homo sapiens
<400> 154
   ugugggcagg gcccugggga gcugaggcuc uggggguggc cggggcugac ccugggccuc    60
   ugcuccccag ugucugaccg cg 82
<210> 155
   <211> 78
   <212> DNA
   <213> Homo sapiens
<400> 155
   uggccgauuu uggcacuagc acauuuuugc uugugucucu ccgcucugag caaucaugug 60
   cagugccaau augggaaa 78
<210> 156
   <211> 81
   <212> DNA
   <213> Homo sapiens
<400> 156
   cccauuggca uaaacccgua gauccgaucu uguggugaag uggaccgcac aagcucgcuu 60
   cuaugggucu gugucagugu g 81
<210> 157
   <211> 22
   <212> RNA
   <213> Homo sapiens
<400> 157
   ugagguagua gguuguauag uu 22
<210> 158
   <211> 22
   <212> RNA
   <213> Homo sapiens
<400> 158
   ugagguagua gguugugugg uu 22
<210> 159
   <211> 22
   <212> RNA
   <213> Homo sapiens
<400> 159
   ugagguagua gguuguaugg uu 22
<210> 160
   <211> 22
   <212> RNA
   <213> Homo sapiens
<400> 160
   ugagguagga gguuguauag uu 22
<210> 161
   <211> 22
   <212> RNA
   <213> Homo sapiens
<400> 161
   ugagguagua gauuguauag uu 22
<210> 162
   <211> 22
   <212> RNA
   <213> Homo sapiens
<400> 162
   ugagguagua guuuguacag uu 22
<210> 163
   <211> 21
   <212> RNA
   <213> Homo sapiens
<400> 163
   cuauacaauc uacugucuuu c 21
<210> 164
   <211> 22
   <212> RNA
   <213> Homo sapiens
<400> 164
   cuauacaacc uacugccuuc cc 22
<210> 165
   <211> 22
   <212> RNA
   <213> Homo sapiens
<400> 165
   uagaguuaca cccugggagu ua 22
<210> 166
   <211> 22
   <212> RNA
   <213> Homo sapiens
<400> 166
   cuauacggcc uccuagcuuu cc 22
<210> 167
   <211> 22
   <212> RNA
   <213> Homo sapiens
<400> 167
   cuauacaauc uauugccuuc cc 22
<210> 168
   <211> 21
   <212> RNA
   <213> Homo sapiens
<400> 168
   cuguacaggc cacugccuug c 21
<210> 169
   <211> 80
   <212> DNA
   <213> Homo sapiens
<400> 169
   ugggaugagg uaguagguug uauaguuuua gggucacacc caccacuggg agauaacuau 60
   acaaucuacu gucuuuccua 80
<210> 170
   <211> 83
   <212> DNA
   <213> Homo sapiens
<400> 170
   cggggugagg uaguagguug ugugguuuca gggcagugau guugccccuc ggaagauaac 60
   uauacaaccu acugccuucc cug 83
<210> 171
   <211> 84
   <212> DNA
   <213> Homo sapiens
<400> 171
   gcauccgggu ugagguagua gguuguaugg uuuagaguua cacccuggga guuaacugua 60
   caaccuucua gcuuuccuug gagc 84
<210> 172
   <211> 79
   <212> DNA
   <213> Homo sapiens
<400> 172
   cccgggcuga gguaggaggu uguauaguug aggaggacac ccaaggagau cacuauacgg 60
   ccuccuagcu uuccccagg 79
<210> 173
   <211> 87
   <212> DNA
   <213> Homo sapiens
<400> 173
   ucagagugag guaguagauu guauaguugu gggguaguga uuuuacccug uucaggagau 60
   aacuauacaa ucuauugccu ucccuga 87
<210> 174
   <211> 84
   <212> DNA
   <213> Homo sapiens
<400> 174
   aggcugaggu aguaguuugu acaguuugag ggucuaugau accacccggu acaggagaua 60
   acuguacagg ccacugccuu gcca 84
<210> 175
   <211> 7
   <212> DNA
   <213> Homo sapiens
<400> 175
   ggcagug 7
<210> 176
   <211> 7
   <212> RNA
   <213> Homo sapiens
<400> 176
   aaucacu 7
<210> 177
   <211> 7
   <212> RNA
   <213> Homo sapiens
<400> 177
   aucacua 7
<210> 178
   <211> 1889
   <212> DNA
   <213> Homo sapiens
<400> 178
   atgacatttc aaggatagaa gtatacctga ttttttttcc ttttaatttt cctggtgcca   60
   atttcaagtt ccaagttgct aatacagcaa caatttatga attgaattat cttggttgaa  120
   aataaaaaga tcactttctc agttttcata agtattatgt ctcttctgag ctatttcatc  180
   tatttttggc agtctgaatt tttaaaaccc atttaaattt ttttccttac ctttttattt  240
   gcatgtggat caaccatcgc tttattggct gagatatgaa catattgttg aaaggtaatt  300
   tgagagaaat atgaagaact gaggaggaaa aaaaaaaaaa agaaaagaac caacaacctc  360
   aactgcctac tccaaaatgt tggtcatttt atgttaaggg aagaattcca gggtatggcc  420
   atggagtgta caagtatgtg ggcagatttt cagcaaactc ttttcccact gtttaaggag  480
   ttagtggatt actgccattc acttcataat ccagtaggat ccagtgatcc ttacaagtta  540
   gaaaacataa tcttctgcct tctcatgatc caactaatgc cttactcttc ttgaaatttt  600
   aacctatgat attttctgtg cctgaatatt tgttatgtag ataacaagac ctcagtgcct  660
   tcctgttttt cacattttcc ttttcaaata gggtctaact cagcaactcg ctttaggtca  720
   gcagcctccc tgaagaccaa aattagaata tccatgacct agttttccat gcgtgtttct  780
   gactctgagc tacagagtct ggtgaagctc acttctgggc ttcatctggc aacatcttta  840
   tccgtagtgg gtatggttga cactagccca atgaaatgaa ttaaagtgga ccaatagggc  900
   tgagctctct gtgggctggc agtcctggaa gccagctttc cctgcctctc atcaactgaa  960
   tgaggtcagc atgtctattc agcttcgttt attttcaaga ataatcacgc tttcctgaat 1020
   ccaaactaat ccatcaccgg ggtggtttag tggctcaaca ttgtgttccc atttcagctg 1080
   atcagtgggc ctccaaggag gggctgtaaa atggaggcca ttgtgtgagc ctatcagagt 1140
   tgctgcaaac ctgacccctg ctcagtaaag cacttgcaac cgtctgttat gctgtgacac 1200
   atggcccctc cccctgccag gagctttgga cctaatccaa gcatcccttt gcccagaaag 1260
   aagatggggg aggaggcagt aataaaaaga ttgaagtatt ttgctggaat aagttcaaat 1320
   tcttctgaac tcaaactgag gaatttcacc tgtaaacctg agtcgtacag aaagctgcct 1380
   ggtatatcca aaagcttttt attcctcctg ctcatattgt gattctgcct ttggggactt 1440
   ttcttaaacc ttcagttatg attttttttt catacactta ttggaactct gcttgatttt 1500
   tgcctcttcc agtcttcctg acactttaat taccaacctg ttacctactt tgactttttg 1560
   catttaaaac agacactggc atggatatag ttttactttt aaactgtgta cataactgaa 1620
   aatgtgctat actgcatact ttttaaatgt aaagatattt ttatctttat atgaagaaaa 1680
   tcacttagga aatggctttg tgattcaatc tgtaaactgt gtattccaag acatgtctgt 1740
   tctacataga tgcttagtcc ctcatgcaaa tcaattactg gtccaaaaga ttgctgaaat 1800
   tttatatgct tactgatata ttttacaatt ttttatcatg catgtcctgt aaaggttaca 1860
   agcctgcaca ataaaaatgt ttaacggtt                                   1889
<210> 179
   <211> 301
   <212> DNA
   <213> Homo sapiens
<400> 179
   ctgaagataa aattacagga tatcacattg gagtcactgc caagtcatag ccataaatga 60
   tgagtcggtc ctctttccag tggatcataa gacaatggac cctttttgtt atgatggttt 120
   taaactttca attgtcactt tttatgctat ttctgtatat aaaggtgcac gaaggtaaaa 180
   agtatttttt caagttgtaa ataatttatt taatatttaa tggaagtgta tttattttac 240
   agctcattaa acttttttaa ccaaacagaa aaaaaaaaaa aaaaaaaaaa aaaaaaaaaa 300
   a 301
<210> 180
   <211> 776
   <212> DNA
   <213> Homo sapiens
<400> 180
   gaatcgtgca gggggagaaa tccttgagcc agggctgcca tataacctga caggaacatg 60
   ctactgaagt ttattttacc attgactgct gccctcaatc tagaacgcta cacaagaaat 120
   atttgtttta ctcagcaggt gtgccttaac ctccctattc agaaagctcc acatcaataa 180
   acatgacact ctgaagtgaa agtagccacg agaattgtgc tacttatact ggttcataat 240
   ctggaggcaa ggttcgactg cagccgcccc gtcagcctgt gctaggcatg gtgtcttcac 300
   aggaggcaaa tccagagcct ggctgtgggg aaagtgacca ctctgccctg accccgatca 360
   gttaaggagc tgtgcaataa ccttcctagt acctgagtga gtgtgtaact tattgggttg 420
   gcgaagcctg gtaaagctgt tggaatgagt atgtgattct ttttaagtat gaaaataaag 480
   atatatgtac agacttgtat tttttctctg gtggcattcc tttaggaatg ctgtgtgtct 540
   gtccggcacc ccggtaggcc tgattgggtt tctagtcctc cttaaccact tatctcccat 600
   atgagagtgt gaaaaatagg aacacgtgct ctacctccat ttagggattt gcttgggata 660
   cagaagaggc catgtgtctc agagctgtta agggcttatt tttttaaaac attggagtca 720
   tagcatgtgt gtaaacttta aatatgcaaa taaataagta tctatgtcta aaaaaa 776
<210> 181
   <211> 504
   <212> DNA
   <213> Homo sapiens
<400> 181
   agcaaagagg aaagcagacc catggctcag gacaagctcc cagtgatcac tcaagaatct 60
   ggctctcatt ctaagaggct gtgctgccca gtatggtggt tgtgataaat ctaaaccagc 120
   cctgcatgaa acagagtcca agctgtctcc caacagcctg ggttcggtcc ttggctggcc 180
   caggcccagt taagcctgtg gccaccaagc agctcatctg agcactttgg gatgtattca 240
   gcctacgttg ccctggaaaa ggaagcagga gatgtctccc tgtgggaaag gagaagagaa 300
   gttgtctctg agtcccctgt caccagttgg attcatttct tggaagagcc agaatgagcc 360
   actttgacca ccctcgggtg ctatgggtga cacaagagct gtccactggg tgtttgcaga 420
   ataattacac tatcttatgt ctggatcctg atgatttcac agctaaatgg caaaaataaa 480
   acatgtttcc cataaaaaaa aaaa 504
<210> 182
   <211> 622
   <212> DNA
   <213> Homo sapiens
<400> 182
   cttgtaaact tgagttggag tactatattt acaaataaaa ttggcaccat gtgccatctg 60
   tacatattac tgttgcattt acttttaata aagcttgtgg ccccttttac ttttttatag 120
   cttaactaat ttgaatgtgg ttacttccta ctgtagggta gcggaaaagt tgtcttaaaa 180
   ggtatggtgg ggatattttt aaaaactcct tttggtttac ctggggatcc aattgatgta 240
   tatgtttata tactgggttc ttgttttata tacctggctt ttactttatt aatatgagtt 300
   actgaaggtg atggaggtat ttgaaaattt tacttccata ggacatactg catgtaagcc 360
   aagtcatgga gaatctgctg catagctcta ttttaaagta aaagtctacc accgaatccc 420
   tagtccccct gttttctgtt tcttcttgtg attgctgcca taattctaag ttatttactt 480
   ttaccactat ttaagttatc aactttagct agtatcttca aactttcact ttgaaaaatg 540
   agaattttat attctaagcc agttttcatt ttggttttgt gttttggtta ataaaacaat 600
   actcaaatac aaaaaaaaaa aa 622
<210> 183
   <211> 1424
   <212> DNA
   <213> Homo sapiens
<400> 183
   agaagataac taagcaaaca agttggaatt caccaagatt gggtagaact ggtatcactg 60
   aactactaaa gttttacaga aagtagtgct gtgattgatt gccctagcca attcacaagt 120
   tacactgcca ttctgatttt aaaacttaca attggcacta aagaatacat ttaattattt 180
   cctatgttag ctgttaaaga aacagcagga cttgtttaca aagatgtctt cattcccaag 240
   gttactggat agaagccaac cacagtctat accatagcaa tgtttttcct ttaatccagt 300
   gttactgtgt ttatcttgat aaactaggaa ttttgtcact ggagttttgg actggataag 360
   tgctacctta aagggtatac taagtgatac agtactttga atctagttgt tagattctca 420
   aaattcctac actcttgact agtgcaattt ggttcttgaa aattaaattt aaacttgttt 480
   acaaaggttt agttttgtaa taaggtgact aatttatcta tagctgctat agcaagctat 540
   tataaaactt gaatttctac aaatggtgaa atttaatgtt ttttaaacta gtttatttgc 600
   cttgccataa cacatttttt aactaataag gcttagatga acatggtgtt caacctgtgc 660
   tctaaacagt gggagtacca aagaaattat aaacaagata aatgctgtgg ctccttccta 720
   actggggctt tcttgacatg taggttgctt ggtaataacc tttttgtata tcacaatttg 780
   ggtgaaaaac ttaagtaccc tttcaaacta tttatatgag gaagtcactt tactactcta 840
   agatatccct aaggaatttt tttttttaat ttagtgtgac taaggcttta tttatgtttg 900
   tgaaactgtt aaggtccttt ctaaattcct ccattgtgag ataaggacag tgtcaaagtg 960
   ataaagctta acacttgacc taaacttcta ttttcttaag gaagaagagt attaaatata 1020
   tactgactcc tagaaatcta tttattaaaa aaagacatga aaacttgctg tacataggct 1080
   agctatttct aaatatttta aattagcttt tctaaaaaaa aaatccagcc tcataaagta 1140
   gattagaaaa ctagattgct agtttatttt gttatcagat atgtgaatct cttctccctt 1200
   tgaagaaact atacatttat tgttacggta tgaagtcttc tgtatagttt gtttttaaac 1260
   taatatttgt ttcagtattt tgtctgaaaa gaaaacacca ctaattgtgt acatatgtat 1320
   tatataaact taacctttta atactgttta tttttagccc attgtttaaa aaataaaagt 1380
   taaaaaaatt taactgctta aaagtaaaaa aaaaaaaaaa aaaa 1424
<210> 184
   <211> 1160
   <212> DNA
   <213> Homo sapiens
<400> 184
   attcttctct tacaccccac ccgaaagtat tcagctggca tttagagagc tacagtcttc 60
   attttagtgc tttacacatt cgggcctgaa aacaaatatg acctttttta cttgaagcca 120
   atgaatttta atctatagat tctttaatat tagcacagaa taatatcttt gggtcttact 180
   atttttaccc ataaaagtga ccaggtagac cctttttaat tacattcact acttctacca 240
   cttgtgtatc tctagccaat gtgcttgcaa gtgtacagat ctgtgtagag gaatgtgtgt 300
   atatttacct cttcgtttgc tcaaacatga gtgggtattt ttttgtttgt tttttttgtt 360
   gttgttgttt ttgaggcgcg tctcaccctg ttgcccaggc tggagtgcaa tggcgcgttc 420
   tctgctcact acagcacccg cttcccaggt tgaagtgatt ctcttgcctc agcctcccga 480
   gtagctggga ttacaggtgc ccaccaccgc gcccagctaa ttttttaatt tttagtagag 540
   acagggtttt accatgttgg ccaggctggt cttgaactcc tgaccctcaa gtgatctgcc 600
   caccttggcc tccctaagtg ctgggattat aggcgtgagc caccatgctc agccattaag 660
   gtattttgtt aagaacttta agtttagggt aagaagaatg aaaatgatcc agaaaaatgc 720
   aagcaagtcc acatggagat ttggaggaca ctggttaaag aatttatttc tttgtatagt 780
   atactatgtt catggtgcag atactacaac attgtggcat tttagactcg ttgagtttct 840
   tgggcactcc caagggcgtt ggggtcataa ggagactata actctacaga ttgtgaatat 900
   atttattttc aagttgcatt ctttgtcttt ttaagcaatc agatttcaag agagctcaag 960
   ctttcagaag tcaatgtgaa aattccttcc taggctgtcc cacagtcttt gctgccctta 1020
   gatgaagcca cttgtttcaa gatgactact ttggggttgg gttttcatct aaacacattt 1080
   ttccagtctt attagataaa ttagtccata tggttggtta atcaagagcc ttctgggttt 1140
   ggtttggtgg cattaaatgg 1160
<210> 185
   <211> 1778
   <212> DNA
   <213> Homo sapiens
<400> 185
   gggcggcagg accagccagc agcagcccaa gcttccctcc atcccccttt accctctttg   60
   ctgcagagaa acttaagcaa aggggacagc tgtgtgacat ttggagaggg ggcctgggac   120
   ttccatgcct taaacctacc tcccacactc ccaaggttgg agcccagggc atcttgctgg   180
   ctacgcctct tctgtccctg ttagacgtcc tccgtccata tcagaactgt gccacaatgc   240
   agttctgagc accgtgtcaa gctgctctga gccacagtgg gatgaaccag ccggggcctt   300
   atcgggctcc agccatctca tgaggggaga ggagacggag gggagtagag aagttacaca   360
   gaaatgctgc tggccaaata gcaaagacaa cctgggaagg aaaggtcttt gtgggataat   420
   ccatatgttt aatttattca acttcatcaa tcactttatt ttattttttt ttctaactcc   480
   tggagactta ttttactgct tcattaggtt gaaatactgc cattctaggt agggttttat   540
   tatcccaggg actacctcgg cttttaattt aaaaaaaaaa aagaagtggg taagaaaatg   600
   caaacctgtt ataagttatc ggacagaaag ctaggtgctc tgtcaccccc aggaggcgct   660
   gtggtactgg ggctgctgct atttaagcca agaactgagg tcctggtgag agcgttggac   720
   ccaggcttgg ctgcctgaca taagctaaat ctcccagacc caccactggc taccgatatc   780
   tatttggtgg gaggtgtggc cctgttcttc ctcaccccag ttccatgaca ttggctggta   840
   taggagccac agtcaggaaa gcacttgagg cagcatctgt tgggccaccc ccggctcagt   900
   gctggaatgt tgcagtgtag gtttcccagg gaaggggggt gggggtaggt gggctccaca     960
   ggatggggga ggagcatgtc cactgagtat cttccttatg ttgctgtgat attgatagct   1020
   tttattttct aatttttaaa aaatggtcat attatgagtc aaagagtatc aaatcagtgt   1080
   tggatggacc acccaagggt gaggagaggg gctggaagcc ctgggcatta ggagaaggga   1140
   gtgggtgctg gcatggacat gactggatag aattttctca ggagggagct tggtggattt   1200
   tgaaggtaaa actttctggg tttatcatgt tttaatttta gagacaggga gtgatgaatc   1260
   atcaccggtt gtccccttat ctaactccat aaaagtggga atttcaaaag aacacctcat   1320
   ccaaggagct ggggcagact tcattgattc tagagagacc tgtttcagtg cctactcatc   1380
   cctgccctct ggtgccagcc tccttaccat cacggcttca ctgaggtgta ggtgggtttt   1440
   tcttaaacag gagacagtct ctcccctctt acctcaactt cttggggtgg gaatcagtga   1500
   tactggagat ggctagttgc tgtgttacgg gtttgagtta catttggcta taaaacaatc   1560
   ttgttgggaa aaatgtgggg gagaggactt cttcctacac gcgcattgag acagattcca   1620
   actggttaat gatattgttt gtaagaaaga gattctgttg gttgactgcc taaagagaaa   1680
   ggtgggatgg ccttcagatt ataccagctt agctagcatt actaaccaac tgttggaagc   1740
   tctgaaaata aaagatcttg aacccataaa aaaaaaaa                           1778
<210> 186
   <211> 759
   <212> DNA
   <213> Homo sapiens
<400> 186
   aaggatgttt tcttttttca cacagtaaaa attcttatca ttcaaggata ttggaaccac     60
   aggactattt ggataaaaaa cattatttgc aaattaatgc gcataggcca tcttactttt    120
   attgcaaaat ggcatgtgct gccatctatt attcattttt aaatggtcat ttcttattca    180
   gtgagtgctt tagtgtttta aactatatgg ataagaatgc aggtagataa tattctaggc    240
   ataaaacatt taatgtacct tacctcatgc aatattcttt ggattctttg ttgatttatg    300
   atattgctaa tataatattt tcttaaaata tataacaata tcttttatgc attttgagtt    360
   ccagctggtg cttctttata tttagaaatt ataatgggaa ggtcatttaa tttacagatg    420
   gttttaaaat tgaggtaata tctgaggtgg cataatttaa aaatatttag caaatttgtt    480
   tcatatatac tgtcttattt ctagatttgt ttaaaattgg aatatgaaaa actaatggat    540
   aaagctagca taaaattgat attttagttt gtattattaa tatatcatgt taccttatat    600
   attaatctac tcttgattct gctaattatt accaacaaaa ttgtattcat gacattttat    660
   taatcctctg tgaattttct gtaaataaaa ttatttctga aaatctctaa aaaaaaaaaa    720
   aaaaaaaaaa agaaaaaaaa aaaaaaaaaa aaaaaaaaa                           759
<210> 187
   <211> 956
   <212> DNA
   <213> Homo sapiens
<400> 187
   caaaataagt atttgttttg atattaaaag attcaatact gtattttctg ttagcttgtg 60
   ggcattttga attatatatt tcacattttg cataaaactg cctatctacc tttgacactc 120
   cagcatgcta gtgaatcatg tatcttttag gctgctgtgc atttctcttg gcagtgatac 180
   ctccctgaca tggttcatca tcaggctgca atgacagaat gtggtgagca gcgtctactg 240
   agactactaa cattttgcac tgtcaaaata cttggtgagg aaaagatagc tcaggttatt 300
   gctaatgggt taatgcacca gcaagcaaaa tattttatgt tttggggttt tgaaaaatca 360
   aagataatta accaaggatc ttaactgtgt tcgcattttt tatccaagca cttagaaaac 420
   ctacaatcct aattttgatg tccattgtta agaggtggtg atagatacta tttttttttt 480
   catattgtat agcggttatt agaaaagttg gggattttct tgatctttat tgctgcttac 540
   cattgaaact taacccagct gtgttcccca actctgttct gcgcacgaaa cagtatctgt 600
   ttgaggcata atcttaagtg gccacacaca atgttttctc ttatgttatc tggcagtaac 660
   tgtaacttga attacattag cacattctgc ttagctaaaa ttgttaaaat aaactttaat 720
   aaacccatgt agccctctca tttgattgac agtattttag ttatttttgg cattcttaaa 780
   gctgggcaat gtaatgatca gatctttgtt tgtctgaaca ggtattttta tacatgcttt 840
   ttgtaaacca aaaactttta aatttcttca ggttttctaa catgcttacc actgggctac 900
   tgtaaatgag aaaagaataa aattatttaa tgttttaaaa aaaaaaaaaa aaaaaa 956
<210> 188
   <211> 1223
   <212> DNA
   <213> Homo sapiens
<400> 188
   cacccagccc ctctaggttt cagtttcctt atttctgagt gaagatgatg atgttggcta 60
   tgtcttctcc atggggtttt tatgagtatc aaacaagttg aaatactctt cttgatgtga 120
   atgcctgttc tcctgttctt cttttgcaga ttgctttact tgctctgctt cccagagatc 180
   cgcttctttt cttctctgct ttcttctttc tgcccctctt cctccccctc ctcttcttcc 240
   tcccattctt acccctttct agagattcat tttcactact ttattcagaa taacatctat 300
   agcaaagatt tctacttctt tcccgtatga cttttctccc cagctccaaa cctcatattt 360
   ctgattgcct caggatagtt cccttttgag atactttcct gtctttagct caatctcaac 420
   acattgaaaa cacaactcat tcacttgaaa aacacttact aaaatctcac tatatgctag 480
   gcagtatgtt aagcatgcat attgctctct ctaaaaatca gttctgtagg aatccctttc 540
   ccctcctgat ggtctcatcc ttatcctggt ctcccactca gggaacctcc tgattattct 600
   gctctgtgct tcctcagttc accctttgct ctcttttctt ttgcttcacc tagtcacatc 660
   ctgtgtgcct gacagccagt ctgctgtaat tgtcttagaa ctgatcctct tgactccaca 720
   ccactgctag gttgaacatc taaaagtcct gtgctgatcc tctcagtccc ctgctcatca 780
   actctcaagg acaacctatt accaaacacc tttgacctca acctctggag tcctcagccc 840
   aggacttgaa gttaggagat ctttttttgc cttggctctg ccataaatac accctgtaac 900
   cttggatgtg ttgcaacatc tgcaaggata ggaatttgat cactgctaag gttccttcaa 960
   acttgatcag ttcgctgttc tgtcttttgg atgttaattc acattcatct cctatattaa 1020
   gactttggct aagtcttgtg atttcttgcc ttattggagc ttttcttcta atctgaattc 1080
   ttgcttctct ctccttttcc tttacacacc cacacaagtc ctaacctctc caggaagtgt 1140
   tttgaaatga tgttacctgg gtttgagttc ttggcattag cctgtatcat ctatttggta 1200
   ttattatact attatagctg ttt 1223
<210> 189
   <211> 2770
   <212> DNA
   <213> Homo sapiens
<400> 189
   cctgccgctt tgccaggtgg gggtgggatc aaacgccctg agagtcccgg atgtccgagg 60
   cgggatgcaa accatcccgt cctgagcacg ggtccttcct ctctctttca tccacacttc 120
   tgttaacttc ccaccaccat caatcatctg atttcctgaa agtaattaat tgtgcattta 180
   ataccagtta gagttccgac tctgcatggt gtcacagtga aagcgccgac tgacttatgg 240
   ttttgattca agaatcgtct tattctggaa gtagatctga ataggctacc ggagccttgt 300
   ttttctaaag gggggcgctg tctagcactt aactagggta agcattctta acatgtattt 360
   ccacttgccc tgagtaaatc tgtggtgaga gaagcttcct ttctgcagtt taaaaaagct 420
   actgcttcct taggcttcat caggaagcca ccttcagttg tgaatcctat ggtgttattt 480
   attttgttcc tgaaatggga tttagtgcaa aaagtttaca actacagtct ttaacacatt 540
   tttttcaggg tatgacgact tgaatgttta tacttttatt ctataatttg ccctgcactt 600
   attttacaac ctagtaataa tgtggataaa tgtatctaca tgacacatgt caagaccaaa 660
   ataactgtga atgacacacc ttgctgtaaa tgaactgtgc taaccctgac tgtgggcttg 720
   agaacaaaga tgaactctag aactctagca gcctaactgc tgcttctcaa ataactgtgt 780
   gaacagtgag atattactgt ttgtttctaa aaatcctact gtgcccagtt tccttcacta 840
   catgccctgc attttttatt taaatattta gctgtagcgc catcagatat ggatgccttc 900
   taacaattgc tgtttgtaaa ataaatcagg atggtagaaa gtgattatat ggaaaattgg 960
   aacctggatg agaccttttc gttgaattct gaagagtaat gatgtgaaaa ttgatacagg 1020
   gcaagagatg attcttttgt ttttcttcta cttcatgtcc agaagagtaa gagggaaaat 1080
   ggacatatgt ttcatatcca agggtattca aactgtagtt agttggtacc tctgaaaaat 1140
   gagaatggtg agcgcacggg ttggttgttc tagcatgaat acaattctgg aaactgttat 1200
   gcaatttccc ttttttaacc cacattactt taggggtgca ttaagtcgcc aaactatact 1260
   agttctttgt attcctagac ttgctgatat ttacctctct cttgtctctt cagagtaaat 1320
   ggttcccttc tttccttcct actttccttc attctctctt ccttccctcc ttcctacttc 1380
   ttttcttcct tcctcttcct ctcttaaaac tatcttagat gtagaatcct ggtgtagggt 1440
   tttattttat ttttattttt tgacccaata aaatgttata tgaaagaatg aaaatattaa 1500
   tttaagagac tctgggagtc tgaataaagt agctttatat taactacagg ataatattag 1560
   ccttattacc cccacaagat tttttaaaac ttgaggtagg tagctacatt aaataaattt 1620
   gctacttata taaaaatttt tatcaacact aaacttttaa agtttacaag tttttttttt 1680
   cttttttaca gtcttctata gagttaggtt aaaaatgtgg ttctaaccat caacaattgc 1740
   atggttaaat gaccctgaac taaaactgat gggttcccta tcaaaacaaa taaaaatata 1800
   cctttttcag gtttcaatct gtgcagggta tatgcatgtt aattctacca tgcttaagaa 1860
   cttccacaaa atatttcatg gagaggtctg catttagacg gaaacagaaa ttgcttttcc 1920
   cctcactgtt cctgaatgct ctatacttgt tttaacattt ttgctatctt tttttattat 1980
   tctgatcatg atatgaccat ttaacctcag aattcataat tcctgagggg tgttaagaag 2040
   cagtcccatt ggtgaggata ttatgacttg gtgaccattc ttaggagtag aaaaccaagg 2100
   acaattgctt ctgtattcag tatccacttc ttaatgtggc tttatatgta aaaataataa 2160
   tgcagtggtt gtttctgtca ggaaaataaa tcttacagaa caactggtgg aattgaagct 2220
   gctgcgctag acttggatat tttgggtagt gaagaagcaa tggcaatctt gagtctatta 2280
   ttgtataatt tagtaaaaga aaaaaataat cgttggtggt cctactaaga gaatgcagct 2340
   tttttgagtt gtcacagagg ctgtgtgtgc cctacactga ccagggtttg taaaaccctt 2400
   tcattctggt acaagagtcg ggggtataac ttttatactt gaatctacct accaagttta 2460
   catttctcaa ttcctttttg taaggtgcta tttctgtatt taaataactt tcttttaacg 2520
   taaagctgct ttctgcttat cttattgcac tgctagttgt atgtaggtat taattttatt 2580
   gctgcttact gcttttgttt tcttattatt tagctctgct ctttttccta atggctatat 2640
   tatctatagc tatttacttg taactgtact acatgtaaac tgattttttg ttctgatttt 2700
   ttttctaata tttttaggaa aatattaagc tttataaaat agcaataaaa aataattcat 2760
   ttaagaagaa 2770
<210> 190
   <211> 970
   <212> DNA
   <213> Homo sapiens
<400> 190
   agccctgccc ttgcccctgt gctcaagctg tccaccatcc cgggcactcc aaggctcagt 60
   gcaccccaag cctctgagtg aggacagcag gcagggactg ttctgctcct catagctccc 120
   tgctgcctga ttatgcaaaa gtagcagtca caccctagcc actgctggga ccttgtgttc 180
   cccaagagta tctgattcct ctgctgtccc tgccaggagc tgaagggtgg gaacaacaaa 240
   ggcaatggtg aaaagagatt aggaaccccc cagcctgttt ccattctctg cccagcagtc 300
   tcttaccttc cctgatcttt gcagggtggt ccgtgtaaat agtataaatt ctccaaatta 360
   tcctctaatt ataaatgtaa gcttatttcc ttagatcatt atccagagac tgccagaagg 420
   tgggtaggat gacctggggt ttcaattgac ttctgttcct tgcttttagt tttgatagaa 480
   gggaagacct gcagtgcacg gtttcttcca ggctgaggta cctggatctt gggttcttca 540
   ctgcagggac ccagacaagt ggatctgctt gccagagtcc tttttgcccc tccctgccac 600
   ctccccgtgt ttccaagtca gctttcctgc aagaagaaat cctggttaaa aaagtctttt 660
   gtattgggtc aggagttgaa tttggggtgg gaggatggat gcaactgaag cagagtgtgg 720
   gtgcccagat gtgcgctatt agatgtttct ctgataatgt ccccaatcat accagggaga 780
   ctggcattga cgagaactca ggtggaggct tgagaaggcc gaaagggccc ctgacctgcc 840
   tggcttcctt agcttgcccc tcagctttgc aaagagccac cctaggcccc agctgaccgc 900
   atgggtgtga gccagcttga gaacactaac tactcaataa aagcgaaggt ggacaaaaaa 960
   aaaaaaaaaa 970
<210> 191
   <211> 309
   <212> DNA
   <213> Homo sapiens
<400> 191
   tatcatcagt aactgcagga catgattatt gaggcttgat tggcaaatac gacttctaca 60
   tccatattct catctttcat accatatcac actactacca ctttttgaag aatcatcaaa 120
   gagcaatgca aatgaaaaac actataattt actgtatact ctttgtttca ggatacttgc 180
   cttttcaatt gtcacttgat gatataattg caatttaaac tgttaagctg tgttcagtac 240
   tgtttctgaa taatagaaat cacttctcta aaagcaataa atttcaagca catttttaca 300
   taaaaaaaa 309
<210> 192
   <211> 976
   <212> DNA
   <213> Homo sapiens
<400> 192
   aatgtgaggc gattatttta agtaattatc ttaccaagcc caagactggt tttaaagtta 60
   cctgaagctc ttaacttcct cccctctgaa tttagtttgg ggaaggtgtt tttagtacaa 120
   gacatcaaag tgaagtaaag cccaagtgtt ctttagcttt ttataatact gtctaaatag 180
   tgaccatctc atgggcattg ttttcttctc tgctttgtct gtgttttgag tctgctttct 240
   tttgtcttta aaacctgatt tttaagttct tctgaactgt agaaatagct atctgatcac 300
   ttcagcgtaa agcagtgtgt ttattaacca tccactaagc taaaactaga gcagtttgat 360
   ttaaaagtgt cactcttcct ccttttctac tttcagtaga tatgagatag agcataatta 420
   tctgttttat cttagtttta tacataattt accatcagat agaactttat ggttctagta 480
   cagatactct actacactca gcctcttatg tgccaagttt ttctttaagc aatgagaaat 540
   tgctcatgtt cttcatcttc tcaaatcatc agaggccaaa gaaaaacact ttggctgtgt 600
   ctataacttg acacagtcaa tagaatgaag aaaattagag tagttatgtg attatttcag 660
   ctcttgacct gtcccctctg gctgcctctg agtctgaatc tcccaaagag agaaaccaat 720
   ttctaagagg actggattgc agaagactcg gggacaacat ttgatccaag atcttaaatg 780
   ttatattgat aaccatgctc agcaatgagc tattagattc attttgggaa atctccataa 840
   tttcaatttg taaactttgt taagacctgt ctacattgtt atatgtgtgt gacttgagta 900
   atgttatcaa cgtttttgta aatatttact atgtttttct attagctaaa ttccaacaat 960
   tttgtacttt aataaa 976
<210> 193
   <211> 81
   <212> DNA
   <213> Homo sapiens
<400> 193
   guuccacucu agcagcacgu aaauauuggc guagugaaau auauauuaaa caccaauauu 60
   acugugcugc uuuaguguga c 81
<210> 194
   <211> 110
   <212> DNA
   <213> Homo sapiens
<400> 194
   agaagggcua ucaggccagc cuucagagga cuccaaggaa cauucaacgc ugucggugag 60
   uuugggauuu gaaaaaacca cugaccguug acuguaccuu gggguccuua 110
<210> 195
   <211> 84
   <212> DNA
   <213> Homo sapiens
<400> 195
   ggcuguggcu ggauucaagu aauccaggau aggcuguuuc caucugugag gccuauucuu 60
   gauuacuugu uucuggaggc agcu 84
<210> 196
   <211> 72
   <212> DNA
   <213> Homo sapiens
<400> 196
   agauacugua aacauccuac acucucagcu guggaaagua agaaagcugg gagaaggcug 60
   uuuacucuuu cu 72
<210> 197
   <211> 111
   <212> DNA
   <213> Homo sapiens
<400> 197
   agaguguuca aggacagcaa gaaaaaugag ggacuuucag gggcagcugu guuuucugac 60
   ucagucauaa ugccccuaaa aauccuuauu guucuugcag ugugcaucgg g 111
<210> 198
   <211> 72
   <212> DNA
   <213> Homo sapiens
<400> 198
   agguugaggu aguagguugu auaguuuaga auuacaucaa gggagauaac uguacagccu 60
   ccuagcuuuc cu 72
<210> 199
   <211> 74
   <212> DNA
   <213> Homo sapiens
<400> 199
   gggugaggua guagguugua uaguuugggg cucugcccug cuaugggaua acuauacaau 60
   cuacugucuu uccu 74
<210> 200
   <211> 83
   <212> DNA
   <213> Homo sapiens
<400> 200
   ugugggauga gguaguagau uguauaguuu uagggucaua ccccaucuug gagauaacua 60
   uacagucuac ugucuuuccc acg 83
<210> 201
   <211> 25
   <212> RNA
   <213> Homo sapiens
<400> 201
   uaggcagugu auugcuagcg gcugu 25
<210> 202
   <211> 23
   <212> RNA
   <213> homo sapiens
<400> 202
   uugcuaguug cacuccucuc ugu 23
<210> 203
   <211> 92
   <212> DNA
   <213> homo sapiens
<400> 203
   gcugggaugu gucagguagg caguguauug cuagcggcug uuaaugauuu uaacaguugc 60
   uaguugcacu ccucucuguu gcauucagaa gc 92
<210> 204
   <211> 20
   <212> DNA
   <213> Homo sapiens
<400> 204
   ccagctaaca atacactgcc 20
<210> 205
   <211> 21
   <212> DNA
   <213> homo sapiens
<400> 205
   agctatgcca gcatcttgcc t 21
<210> 206
   <211> 22
   <212> DNA
   <213> homo sapiens
<400> 206
   gtgtaacacg tctatacgcc ca 22
<210> 207
   <211> 22
   <212> DNA
   <213> Homo sapiens
<400> 207
   accagcuaac aauacacugc ca 22
<210> 208
   <211> 20
   <212> DNA
   <213> Homo sapiens
<400> 208
   causcgucga ucguagcgca 20
<210> 209
   <211> 22
   <212> DNA
   <213> xenopus laevis
<400> 209
   accagctaac attacactgc ct 22
<210> 210
   <211> 25
   <212> DNA
   <213> xenopus laevis
<400> 210
   acagccagct agcaagtgca ctgcc 25
<210> 211
   <211> 25
   <212> DNA
   <213> xenopus laevis
<400> 211
   acagccagct agcaagtgca ctgcc 25
<210> 212
   <211> 22
   <212> DNA
   <213> xenopus laevis
<400> 212
   tgcacgtttc aatacagacc gt 22